# EUROPEAN PATENT APPLICATION

(11) **EP 3 932 941 A1**
(43) Date of publication of application: **05.01.2022**
(21) Application number: 20305758.3
(22) Date of filing: 03.07.2020
(51) Int. Cl.: C07K 14/435, C12N 9/50, C12Q 1/37

(54) **ACTIVATED REPORTER PROTEIN FOR THE DETECTION OF INFECTION IN A BIOLOGICAL SAMPLE**

(71) Applicant: INSTITUT PASTEUR, 75724 Paris Cedex 15 (FR)
(72) Inventor: MEYER, Björn, 75724 PARIS CEDEX 15 (FR); VIGNUZZI, Marco, 75724 PARIS CEDEX 15 (FR)
(74) Representative: Ernest Gutmann - Yves Plasseraud S.A.S.

(57) **Abstract**

The invention relates to novel means and processes for the detection of a virus in a biological sample comprising cells infected by the virus. In particular, the invention relates to a fluorescent reporter protein designed as a recombinant inactive form of flipGFP suitable for specific activation by viral components in particular by viral proteins, such as viral protease, wherein the viral component recognizes a cleavage site inserted in the recombinant flipGFP. The fluorescent reporter protein is suitable for use in an *in vitro* method of detection of virus infection in a biological sample when the virus is related to the viral components activating the inactive form of flipGFP into an active fluorescent flipGFP in a biological sample, especially a sample comprising cells, in particular unaltered cells.

SEQUENCE LISTING

<110>  INSTITUT PASTEUR

<120>  Activated reporter protein for the detection of infection in a
       biological sample

<130>  B14288/AD/CG

<140>  EPxxxxxxx
<141>  2020-07-03

<160>  149

<170>  PatentIn version 3.5

<210>  1
<211>  43
<212>  DNA
<213>  Artificial Sequence

<220>
<223>  Synthetic sequence

<400>  1
gacgagctgt acaagtaata acacatcgac aatcaacctc tgg                         43


<210>  2
<211>  41
<212>  DNA
<213>  Artificial Sequence

<220>
<223>  Synthetic sequence

<400>  2
cgtcaggcag gtccatggat cccgtacgcc cgggcggtgt c                           41


<210>  3
<211>  24
<212>  DNA
<213>  Artificial Sequence

<220>
<223>  Synthetic sequence

<400>  3
atggacctgc ctgacgacca ctac                                              24


<210>  4
<211>  24
<212>  DNA
<213>  Artificial Sequence

<220>
<223>  Synthetic sequence

<400>  4
ttacttgtac agctcgtcca tgcc                                              24

<210>  5
<211>  44
<212>  DNA
<213>  Artificial Sequence

<220>
<223>  Synthetic sequence

<400>  5
gctgctgctt cagcaagacc atggacctgc ctgacgacca ctac                        44


<210>  6
<211>  42
<212>  DNA
<213>  Artificial Sequence

<220>
<223>  Synthetic sequence

<400>  6
cttgctgaag cagcagccca tggatcccgt acgcccgggc gg                          42


<210>  7
<211>  36
<212>  DNA
<213>  Artificial Sequence

<220>
<223>  Synthetic sequence

<400>  7
cacggcggcg gcaagatgga cctgcctgac gaccac                                 36


<210>  8
<211>  37
<212>  DNA
<213>  Artificial Sequence

<220>
<223>  Synthetic sequence

<400>  8
ccaccacggg gtccatggat cccgtacgcc cgggcgg                                37


<210>  9
<211>  84
<212>  DNA
<213>  Artificial Sequence

<220>
<223>  Synthetic sequence

<400>  9
atggaccccg tggtggtgct gggcctgtgc ctgagctgcc tgctgctgct gagcctgtgg      60

aagcagagcc acggcggcgg caag                                              84

<210>  10
<211>  84
<212>  DNA
<213>  Artificial Sequence

<220>
<223>  Synthetic sequence

<400>  10
cttgccgccg ccgtggctct gcttccacag gctcagcagc agcaggcagc tcaggcacag      60

gcccagcacc accacggggt ccat                                              84


<210>  11
<211>  50
<212>  DNA
<213>  Artificial Sequence

<220>
<223>  Synthetic sequence

<400>  11
cggcggcgcc tacaccaggt acgtgaaggt gtccgccctg aaggaaaaag                  50


<210>  12
<211>  51
<212>  DNA
<213>  Artificial Sequence

<220>
<223>  Synthetic sequence

<400>  12
ggtgtaggcg ccgccgttca gctcccttga tgcatcggta atgccagccg c                51


<210>  13
<211>  50
<212>  DNA
<213>  Artificial Sequence

<220>
<223>  Synthetic sequence

<400>  13
gggcggcgcc cccaccaagg tgaccaaggt gtccgccctg aaggaaaaag                  50


<210>  14
<211>  51
<212>  DNA
<213>  Artificial Sequence

<220>
<223>  Synthetic sequence

<400>  14
ggtgggggcg ccgcccttca gggtgaatga tgcatcggta atgccagccg c                51


<210>  15

<211>  53
<212>  DNA
<213>  Artificial Sequence

<220>
<223>  Synthetic sequence

<400>  15
gaagggcggc aagatcgtga acaactggaa ggtgtccgcc ctgaaggaaa aag          53


<210>  16
<211>  51
<212>  DNA
<213>  Artificial Sequence

<220>
<223>  Synthetic sequence

<400>  16
cacgatcttg ccgcccttca gggcgattga tgcatcggta atgccagccg c          51


<210>  17
<211>  52
<212>  DNA
<213>  Artificial Sequence

<220>
<223>  Synthetic sequence

<400>  17
gtgctgcaga gcggcttcag gaagatgaag gtgtccgccc tgaaggaaaa ag          52


<210>  18
<211>  53
<212>  DNA
<213>  Artificial Sequence

<220>
<223>  Synthetic sequence

<400>  18
ctgaagccgc tctgcagcac ggcgctggtt gatgcatcgg taatgccagc cgc          53


<210>  19
<211>  53
<212>  DNA
<213>  Artificial Sequence

<220>
<223>  Synthetic sequence

<400>  19
gaccttccag agcgccgtga agaggaccaa ggtgtccgcc ctgaaggaaa aag          53


<210>  20
<211>  51
<212>  DNA
<213>  Artificial Sequence

<220>
<223>  Synthetic sequence

<400>  20
cacggcgctc tggaaggtca cgccgcttga tgcatcggta atgccagccg c                51


<210>  21
<211>  53
<212>  DNA
<213>  Artificial Sequence

<220>
<223>  Synthetic sequence

<400>  21
caccgtgcag agcaagatga gcgacgtgaa ggtgtccgcc ctgaaggaaa aag              53


<210>  22
<211>  51
<212>  DNA
<213>  Artificial Sequence

<220>
<223>  Synthetic sequence

<400>  22
catcttgctc tgcacggtgg ccacctttga tgcatcggta atgccagccg c                51


<210>  23
<211>  53
<212>  DNA
<213>  Artificial Sequence

<220>
<223>  Synthetic sequence

<400>  23
gaagctgcag aacaacgagc tgagccccaa ggtgtccgcc ctgaaggaaa aag              53


<210>  24
<211>  52
<212>  DNA
<213>  Artificial Sequence

<220>
<223>  Synthetic sequence

<400>  24
gctcgttgtt ctgcagcttc acggcgcttg atgcatcggt aatgccagcc gc               52


<210>  25
<211>  53
<212>  DNA
<213>  Artificial Sequence

<220>
<223>  Synthetic sequence

<400>  25
gaggctgcag gccggcaacg ccaccgagaa ggtgtccgcc ctgaaggaaa aag                53

<210>  26
<211>  51
<212>  DNA
<213>  Artificial Sequence

<220>
<223>  Synthetic sequence

<400>  26
gttgccggcc tgcagcctca cggtggctga tgcatcggta atgccagccg c                  51

<210>  27
<211>  53
<212>  DNA
<213>  Artificial Sequence

<220>
<223>  Synthetic sequence

<400>  27
catgctgcag agcgccgacg cccagagcaa ggtgtccgcc ctgaaggaaa aag                53

<210>  28
<211>  51
<212>  DNA
<213>  Artificial Sequence

<220>
<223>  Synthetic sequence

<400>  28
gtcggcgctc tgcagcatgg gctcccttga tgcatcggta atgccagccg c                  51

<210>  29
<211>  54
<212>  DNA
<213>  Artificial Sequence

<220>
<223>  Synthetic sequence

<400>  29
ggaagaggag gggcgccgac accagcatca aggtgtccgc cctgaaggaa aaag                54

<210>  30
<211>  51
<212>  DNA
<213>  Artificial Sequence

<220>
<223>  Synthetic sequence

<400>  30
gtcggcgccc ctcctcttcc tctcctttga tgcatcggta atgccagccg c                  51

<210>  31
<211>  54
<212>  DNA
<213>  Artificial Sequence

<220>
<223>  Synthetic sequence

<400>  31
gcggcaagag gagctggccc cccagcgaga aggtgtccgc cctgaaggaa aaag          54


<210>  32
<211>  52
<212>  DNA
<213>  Artificial Sequence

<220>
<223>  Synthetic sequence

<400>  32
ggggccagct cctcttgccg ctcctggttg atgcatcggt aatgccagcc gc            52


<210>  33
<211>  53
<212>  DNA
<213>  Artificial Sequence

<220>
<223>  Synthetic sequence

<400>  33
gaagcagagg agcccccagg acaaccagaa ggtgtccgcc ctgaaggaaa aag           53


<210>  34
<211>  51
<212>  DNA
<213>  Artificial Sequence

<220>
<223>  Synthetic sequence

<400>  34
ctgggggctc ctctgcttct cgggctctga tgcatcggta atgccagccg c             51


<210>  35
<211>  55
<212>  DNA
<213>  Artificial Sequence

<220>
<223>  Synthetic sequence

<400>  35
gtgaagagga ggggcggcgg caccggcgag aaggtgtccg ccctgaagga aaaag         55


<210>  36

<211>  51
<212>  DNA
<213>  Artificial Sequence

<220>
<223>  Synthetic sequence

<400>  36
gccgccgccc ctcctcttca ccaggcctga tgcatcggta atgccagccg c                51


<210>  37
<211>  54
<212>  DNA
<213>  Artificial Sequence

<220>
<223>  Synthetic sequence

<400>  37
gggccggcgc cggcatcatc gagaccccca aggtgtccgc cctgaaggaa aaag             54


<210>  38
<211>  51
<212>  DNA
<213>  Artificial Sequence

<220>
<223>  Synthetic sequence

<400>  38
gatgatgccg gcgccggccc tgtcctctga tgcatcggta atgccagccg c                51


<210>  39
<211>  54
<212>  DNA
<213>  Artificial Sequence

<220>
<223>  Synthetic sequence

<400>  39
caccctgtag ctgggggcgc agccggccca aggtgtccgc cctgaaggaa aaag             54


<210>  40
<211>  51
<212>  DNA
<213>  Artificial Sequence

<220>
<223>  Synthetic sequence

<400>  40
gctgggggcg cagccggccc tggtggctga tgcatcggta atgccagccg c                51


<210>  41
<211>  54
<212>  DNA
<213>  Artificial Sequence

<220>
<223>  Synthetic sequence

<400>  41
gggccggcgg ctacatcttc agcagcgaca aggtgtccgc cctgaaggaa aaag          54


<210>  42
<211>  51
<212>  DNA
<213>  Artificial Sequence

<220>
<223>  Synthetic sequence

<400>  42
gaagatgtag ccgccggccc tgtccagtga tgcatcggta atgccagccg c             51


<210>  43
<211>  47
<212>  DNA
<213>  Artificial Sequence

<220>
<223>  Synthetic sequence

<400>  43
cactcttggg aaatttggac aaaaggtgtc cgccctgaag gaaaaag                  47


<210>  44
<211>  46
<212>  DNA
<213>  Artificial Sequence

<220>
<223>  Synthetic sequence

<400>  44
caaatttccc aagagtggtg attgatgcat cggtaatgcc agccgc                   46


<210>  45
<211>  56
<212>  DNA
<213>  Artificial Sequence

<220>
<223>  Synthetic sequence

<400>  45
cgccagcgag cagggcgaga tccagtgggt gaaggtgtcc gccctgaagg aaaaag        56


<210>  46
<211>  54
<212>  DNA
<213>  Artificial Sequence

<220>
<223>  Synthetic sequence

<400>  46
gatctcgccc tgctcgctgg cgaagtagct tgatgcatcg gtaatgccag ccgc          54


<210>  47
<211>  1077
<212>  DNA
<213>  Artificial Sequence

<220>
<223>  Synthetic sequence

<400>  47
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc agaggacagg gccggcgccg gcatcatcga gacccccaag     300

gtgtccgccc tgaaggaaaa agtaagcgca ctgaaagaaa aggtgagcgc gctgaaggag     360

aaagtgagcg ccctgaaaga gaaagtctct gcccttaagg aggatatcga gggcagagga     420

agtctgctaa catgcggtga cgtcgaggag aatcctggcc caaagcttgc caccatgcgc     480

aaaggcgaag aactgtttac cggcattgtg ccgattctgg tggaactgga tggcgatgtg     540

aacggccata aattttttgt gcgcggcgaa ggcgaaggcg atgcgaccat tggcaaactg     600

agcctgaaat ttatttgcac caccggcaaa ctgccggtgc cgtggccgac cctggtgacc     660

accctgacct atggcgtgca gtgctttagc cgctatccgg atcatatgaa acgccatgat     720

ttttttaaaa gcgcgatgcc ggaaggctat gtgcaggaac gcaccattta ttttaaagat     780

gatggcacct ataaaacccg cgcggaagtg aaatttgaag gcgataccct ggtgaaccgc     840

attgaactga aaggcattga ttttaaagaa gatggcaaca ttctgggcca taaactggaa     900

tataacttta acagccataa agtgtatatt accgcggata aacagaacaa cggcattaaa     960

gcgaacttta ccattcgcca taacgtggaa gatggcagcg tgcagctggc ggatcattat    1020

cagcagaaca ccccgattgg cgatggcccg gttcttcttc ctggcggccg ctctaga       1077


<210>  48
<211>  1077
<212>  DNA
<213>  Artificial Sequence

<220>
<223>  Synthetic sequence

<400>  48
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc agccaccagg gccggctgcg cccccagcta cagggtgaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 49
<211> 1077
<212> DNA
<213> Artificial Sequence

<220>
<223> Synthetic sequence

<400> 49
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc actggacagg gccggcggct acatcttcag cagcgacaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 50
<211> 1161
<212> DNA
<213> Artificial Sequence

<220>
<223> Synthetic sequence

<400> 50
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcagagga cagggccggc 360

gccggcatca tcgagacccc caaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a                                              1161

<210>  51
<211>  1161
<212>  DNA
<213>  Artificial Sequence

<220>
<223>  Synthetic sequence

<400>  51
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa     180

gttagcgctc tggaaaaaga agtgtctgca ctcgagaaag aagtaagtgc ccttgagaag     240

gaggtgtccg cactcgagaa ggaggtcagc gccctggaaa aggaaaagcg agaccatatg     300

gttttgcttg agtatgttac agcggctggc attaccgatg catcagccac cagggccggc     360

tgcgccccca gctacagggt gaaggtgtcc gccctgaagg aaaaagtaag cgcactgaaa     420

gaaaaggtga gcgcgctgaa ggagaaagtg agcgccctga aagagaaagt ctctgccctt     480

aaggaggata tcgagggcag aggaagtctg ctaacatgcg gtgacgtcga ggagaatcct     540

ggcccaaagc ttgccaccat gcgcaaaggc gaagaactgt ttaccggcat tgtgccgatt     600

ctggtggaac tggatggcga tgtgaacggc cataaatttt ttgtgcgcgg cgaaggcgaa     660

ggcgatgcga ccattggcaa actgagcctg aaatttattt gcaccaccgg caaactgccg     720

gtgccgtggc cgaccctggt gaccaccctg acctatggcg tgcagtgctt tagccgctat     780

ccggatcata tgaaacgcca tgattttttt aaaagcgcga tgccggaagg ctatgtgcag     840

gaacgcacca tttattttaa agatgatggc acctataaaa cccgcgcgga agtgaaattt     900

gaaggcgata ccctggtgaa ccgcattgaa ctgaaaggca ttgattttaa agaagatggc     960

aacattctgg gccataaact ggaatataac tttaacagcc ataaagtgta tattaccgcg    1020

gataaacaga acaacggcat taaagcgaac tttaccattc gccataacgt ggaagatggc    1080

agcgtgcagc tggcggatca ttatcagcag aacaccccga ttggcgatgg cccggttctt    1140

cttcctggcg gccgctctag a                                              1161

<210>  52
<211>  1161
<212>  DNA
<213>  Artificial Sequence

<220>
<223>  Synthetic sequence

<400>  52

atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcactgga cagggccggc 360

ggctacatct tcagcagcga caaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 53
<211> 1152
<212> DNA
<213> Artificial Sequence

<220>
<223> Synthetic sequence

<400> 53
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaatcac cactcttggg 360

aaatttggac aaaaggtgtc cgccctgaag gaaaaagtaa gcgcactgaa agaaaaggtg 420

agcgcgctga aggagaaagt gagcgccctg aaagagaaag tctctgccct taaggaggat 480

atcgagggca gaggaagtct gctaacatgc ggtgacgtcg aggagaatcc tggcccaaag 540

cttgccacca tgcgcaaagg cgaagaactg tttaccggca ttgtgccgat tctggtggaa 600

ctggatggcg atgtgaacgg ccataaattt tttgtgcgcg gcgaaggcga aggcgatgcg 660

accattggca aactgagcct gaaatttatt tgcaccaccg gcaaactgcc ggtgccgtgg 720

ccgaccctgg tgaccaccct gacctatggc gtgcagtgct ttagccgcta tccggatcat 780

atgaaacgcc atgatttttt taaaagcgcg atgccggaag gctatgtgca ggaacgcacc 840

atttatttta aagatgatgg cacctataaa acccgcgcgg aagtgaaatt tgaaggcgat 900

accctggtga accgcattga actgaaaggc attgatttta aagaagatgg caacattctg 960

ggccataaac tggaatataa ctttaacagc cataaagtgt atattaccgc ggataaacag 1020

aacaacggca ttaaagcgaa ctttaccatt cgccataacg tggaagatgg cagcgtgcag 1080

ctggcggatc attatcagca gaacaccccg attggcgatg gcccggttct tcttcctggc 1140

ggccgctcta ga 1152

<210> 54
<211> 1164
<212> DNA
<213> Artificial Sequence

<220>
<223> Synthetic sequence

<400> 54
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaagcta cttcgccagc 360

gagcagggcg agatccagtg ggtgaaggtg tccgccctga aggaaaaagt aagcgcactg 420

aaagaaaagg tgagcgcgct gaaggagaaa gtgagcgccc tgaaagagaa agtctctgcc 480

cttaaggagg atatcgaggg cagaggaagt ctgctaacat gcggtgacgt cgaggagaat 540

cctggcccaa agcttgccac catgcgcaaa ggcgaagaac tgtttaccgg cattgtgccg 600

attctggtgg aactggatgg cgatgtgaac ggccataaat tttttgtgcg cggcgaaggc 660

gaaggcgatg cgaccattgg caaactgagc ctgaaattta tttgcaccac cggcaaactg 720

ccggtgccgt ggccgaccct ggtgaccacc ctgacctatg gcgtgcagtg ctttagccgc 780

tatccggatc atatgaaacg ccatgatttt tttaaaagcg cgatgccgga aggctatgtg 840

caggaacgca ccatttattt taaagatgat ggcacctata aaacccgcgc ggaagtgaaa 900

tttgaaggcg ataccctggt gaaccgcatt gaactgaaag gcattgattt taaagaagat 960

ggcaacattc tgggccataa actggaatat aactttaaca gccataaagt gtatattacc 1020

gcggataaac agaacaacgg cattaaagcg aactttacca ttcgccataa cgtggaagat 1080

ggcagcgtgc agctggcgga tcattatcag cagaacaccc cgattggcga tggcccggtt 1140

cttcttcctg gcggccgctc taga 1164

<210> 55
<211> 1161
<212> DNA
<213> Artificial Sequence

<220>
<223> Synthetic sequence

<400> 55
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaggga gctgaacggc 360

ggcgcctaca ccaggtacgt gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210>  56
<211>  1161
<212>  DNA
<213>  Artificial Sequence

<220>
<223>  Synthetic sequence

<400>  56
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa     180

gttagcgctc tggaaaaaga agtgtctgca ctcgagaaag aagtaagtgc ccttgagaag     240

gaggtgtccg cactcgagaa ggaggtcagc gccctggaaa aggaaaagcg agaccatatg     300

gttttgcttg agtatgttac agcggctggc attaccgatg catcattcac cctgaagggc     360

ggcgccccca ccaaggtgac caaggtgtcc gccctgaagg aaaaagtaag cgcactgaaa     420

gaaaaggtga gcgcgctgaa ggagaaagtg agcgccctga aagagaaagt ctctgccctt     480

aaggaggata tcgagggcag aggaagtctg ctaacatgcg gtgacgtcga ggagaatcct     540

ggcccaaagc ttgccaccat gcgcaaaggc gaagaactgt ttaccggcat tgtgccgatt     600

ctggtggaac tggatggcga tgtgaacggc cataaatttt ttgtgcgcgg cgaaggcgaa     660

ggcgatgcga ccattggcaa actgagcctg aaatttattt gcaccaccgg caaactgccg     720

gtgccgtggc cgaccctggt gaccaccctg acctatggcg tgcagtgctt tagccgctat     780

ccggatcata tgaaacgcca tgattttttt aaaagcgcga tgccggaagg ctatgtgcag     840

gaacgcacca tttattttaa agatgatggc acctataaaa cccgcgcgga agtgaaattt     900

gaaggcgata ccctggtgaa ccgcattgaa ctgaaaggca ttgattttaa agaagatggc     960

aacattctgg gccataaact ggaatataac tttaacagcc ataaagtgta tattaccgcg    1020

gataaacaga acaacggcat taaagcgaac tttaccattc gccataacgt ggaagatggc    1080

agcgtgcagc tggcggatca ttatcagcag aacaccccga ttggcgatgg cccggttctt    1140

cttcctggcg gccgctctag a                                              1161

<210>  57
<211>  1161
<212>  DNA
<213>  Artificial Sequence

<220>
<223>  Synthetic sequence

<400>  57
atggaccccg tggtggtgct gggcctgtgc ctgagctgcc tgctgctgct gagcctgtgg      60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaatcgc cctgaagggc 360

ggcaagatcg tgaacaactg gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 58
<211> 1161
<212> DNA
<213> Artificial Sequence

<220>
<223> Synthetic sequence

<400> 58
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaccag cgccgtgctg 360

cagagcggct tcaggaagat gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 59
<211> 1161
<212> DNA
<213> Artificial Sequence

<220>
<223> Synthetic sequence

<400> 59
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaagcgg cgtgaccttc 360

cagagcgccg tgaagaggac caaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 60
<211> 1161
<212> DNA
<213> Artificial Sequence

<220>
<223> Synthetic sequence

<400> 60
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaaggt ggccaccgtg 360

cagagcaaga tgagcgacgt gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210>  61
<211>  1161
<212>  DNA
<213>  Artificial Sequence

<220>
<223>  Synthetic sequence

<400>  61
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa     180

gttagcgctc tggaaaaaga agtgtctgca ctcgagaaag aagtaagtgc ccttgagaag     240

gaggtgtccg cactcgagaa ggaggtcagc gccctggaaa aggaaaagcg agaccatatg     300

gttttgcttg agtatgttac agcggctggc attaccgatg catcaagcgc cgtgaagctg     360

cagaacaacg agctgagccc caaggtgtcc gccctgaagg aaaaagtaag cgcactgaaa     420

gaaaaggtga gcgcgctgaa ggagaaagtg agcgccctga aagagaaagt ctctgccctt     480

aaggaggata tcgagggcag aggaagtctg ctaacatgcg gtgacgtcga ggagaatcct     540

ggcccaaagc ttgccaccat gcgcaaaggc gaagaactgt ttaccggcat tgtgccgatt     600

ctggtggaac tggatggcga tgtgaacggc cataaatttt ttgtgcgcgg cgaaggcgaa     660

ggcgatgcga ccattggcaa actgagcctg aaatttattt gcaccaccgg caaactgccg     720

gtgccgtggc cgaccctggt gaccaccctg acctatggcg tgcagtgctt tagccgctat     780

ccggatcata tgaaacgcca tgattttttt aaaagcgcga tgccggaagg ctatgtgcag     840

gaacgcacca tttattttaa agatgatggc acctataaaa cccgcgcgga agtgaaattt     900

gaaggcgata ccctggtgaa ccgcattgaa ctgaaaggca ttgattttaa agaagatggc     960

aacattctgg gccataaact ggaatataac tttaacagcc ataaagtgta tattaccgcg    1020

gataaacaga acaacggcat taaagcgaac tttaccattc gccataacgt ggaagatggc    1080

agcgtgcagc tggcggatca ttatcagcag aacaccccga ttggcgatgg cccggttctt    1140

cttcctggcg gccgctctag a                                              1161


<210>  62
<211>  1161
<212>  DNA
<213>  Artificial Sequence

<220>
<223>  Synthetic sequence

<400>  62
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcagccac cgtgaggctg 360

caggccggca acgccaccga gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 63
<211> 1161
<212> DNA
<213> Artificial Sequence

<220>
<223> Synthetic sequence

<400> 63
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaggga gcccatgctg 360

cagagcgccg acgcccagag caaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 64
<211> 1161
<212> DNA
<213> Artificial Sequence

<220>
<223> Synthetic sequence

<400> 64
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaagga gaggaagagg 360

aggggcgccg acaccagcat caaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 65
<211> 1161
<212> DNA
<213> Artificial Sequence

<220>
<223> Synthetic sequence

<400> 65
atggaccccg tggtggtgct gggcctgtgc ctgagctgcc tgctgctgct gagcctgtgg 60

aagcagagcc acggcggcgg caagatggac ctgcctgacg accactacct gtccacccag 120

accatcctgt ccaaggacct gaactccgga ctcagatctg gcagcggtct cgagatggaa 180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaaccag gagcggcaag 360

aggagctggc cccccagcga gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 66
<211> 1161

<212>  DNA
<213>  Artificial Sequence

<220>
<223>  Synthetic sequence

<400>  66
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa     180

gttagcgctc tggaaaaaga agtgtctgca ctcgagaaag aagtaagtgc ccttgagaag     240

gaggtgtccg cactcgagaa ggaggtcagc gccctggaaa aggaaaagcg agaccatatg     300

gttttgcttg agtatgttac agcggctggc attaccgatg catcagagcc cgagaagcag     360

aggagccccc aggacaacca gaaggtgtcc gccctgaagg aaaaagtaag cgcactgaaa     420

gaaaaggtga gcgcgctgaa ggagaaagtg agcgccctga aagagaaagt ctctgccctt     480

aaggaggata tcgagggcag aggaagtctg ctaacatgcg gtgacgtcga ggagaatcct     540

ggcccaaagc ttgccaccat gcgcaaaggc gaagaactgt ttaccggcat tgtgccgatt     600

ctggtggaac tggatggcga tgtgaacggc cataaatttt ttgtgcgcgg cgaaggcgaa     660

ggcgatgcga ccattggcaa actgagcctg aaatttattt gcaccaccgg caaactgccg     720

gtgccgtggc cgaccctggt gaccaccctg acctatggcg tgcagtgctt tagccgctat     780

ccggatcata tgaaacgcca tgattttttt aaaagcgcga tgccggaagg ctatgtgcag     840

gaacgcacca tttattttaa agatgatggc acctataaaa cccgcgcgga agtgaaattt     900

gaaggcgata ccctggtgaa ccgcattgaa ctgaaaggca ttgattttaa agaagatggc     960

aacattctgg gccataaact ggaatataac tttaacagcc ataaagtgta tattaccgcg    1020

gataaacaga acaacggcat taaagcgaac tttaccattc gccataacgt ggaagatggc    1080

agcgtgcagc tggcggatca ttatcagcag aacaccccga ttggcgatgg cccggttctt    1140

cttcctggcg gccgctctag a                                              1161

<210>  67
<211>  1161
<212>  DNA
<213>  Artificial Sequence

<220>
<223>  Synthetic sequence

<400>  67
atggaccccg tggtggtgct gggcctgtgc ctgagctgcc tgctgctgct gagcctgtgg      60

aagcagagcc acggcggcgg caagatggac ctgcctgacg accactacct gtccacccag     120

accatcctgt ccaaggacct gaactccgga ctcagatctg gcagcggtct cgagatggaa     180

gttagcgctc tggaaaaaga agtgtctgca ctcgagaaag aagtaagtgc ccttgagaag 240

gaggtgtccg cactcgagaa ggaggtcagc gccctggaaa aggaaaagcg agaccatatg 300

gttttgcttg agtatgttac agcggctggc attaccgatg catcaggcct ggtgaagagg 360

aggggcggcg gcaccggcga gaaggtgtcc gccctgaagg aaaaagtaag cgcactgaaa 420

gaaaaggtga gcgcgctgaa ggagaaagtg agcgccctga aagagaaagt ctctgccctt 480

aaggaggata tcgagggcag aggaagtctg ctaacatgcg gtgacgtcga ggagaatcct 540

ggcccaaagc ttgccaccat gcgcaaaggc gaagaactgt ttaccggcat tgtgccgatt 600

ctggtggaac tggatggcga tgtgaacggc cataaatttt ttgtgcgcgg cgaaggcgaa 660

ggcgatgcga ccattggcaa actgagcctg aaatttattt gcaccaccgg caaactgccg 720

gtgccgtggc cgaccctggt gaccaccctg acctatggcg tgcagtgctt tagccgctat 780

ccggatcata tgaaacgcca tgattttttt aaaagcgcga tgccggaagg ctatgtgcag 840

gaacgcacca tttattttaa agatgatggc acctataaaa cccgcgcgga agtgaaattt 900

gaaggcgata ccctggtgaa ccgcattgaa ctgaaaggca ttgattttaa agaagatggc 960

aacattctgg gccataaact ggaatataac tttaacagcc ataaagtgta tattaccgcg 1020

gataaacaga acaacggcat taaagcgaac tttaccattc gccataacgt ggaagatggc 1080

agcgtgcagc tggcggatca ttatcagcag aacaccccga ttggcgatgg cccggttctt 1140

cttcctggcg gccgctctag a 1161

<210> 68
<211> 1068
<212> DNA
<213> Artificial Sequence

<220>
<223> Synthetic sequence

<400> 68
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aatcaccact cttgggaaat ttggacaaaa ggtgtccgcc 300

ctgaaggaaa aagtaagcgc actgaaagaa aaggtgagcg cgctgaagga gaaagtgagc 360

gccctgaaag agaaagtctc tgcccttaag gaggatatcg agggcagagg aagtctgcta 420

acatgcggtg acgtcgagga gaatcctggc ccaaagcttg ccaccatgcg caaaggcgaa 480

gaactgttta ccggcattgt gccgattctg gtggaactgg atggcgatgt gaacggccat 540

aaattttttg tgcgcggcga aggcgaaggc gatgcgacca ttggcaaact gagcctgaaa 600

tttatttgca ccaccggcaa actgccggtg ccgtggccga ccctggtgac caccctgacc 660

tatggcgtgc agtgctttag ccgctatccg gatcatatga aacgccatga tttttttaaa 720

agcgcgatgc cggaaggcta tgtgcaggaa cgcaccattt attttaaaga tgatggcacc 780

tataaaaccc gcgcggaagt gaaatttgaa ggcgataccc tggtgaaccg cattgaactg 840

aaaggcattg attttaaaga agatggcaac attctgggcc ataaactgga atataacttt 900

aacagccata aagtgtatat taccgcggat aaacagaaca acggcattaa agcgaacttt 960

accattcgcc ataacgtgga agatggcagc gtgcagctgg cggatcatta tcagcagaac 1020

accccgattg gcgatggccc ggttcttctt cctggcggcc gctctaga 1068

<210> 69
<211> 1080
<212> DNA
<213> Artificial Sequence

<220>
<223> Synthetic sequence

<400> 69
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aagctacttc gccagcgagc agggcgagat ccagtgggtg 300

aaggtgtccg ccctgaagga aaaagtaagc gcactgaaag aaaaggtgag cgcgctgaag 360

gagaaagtga gcgccctgaa agagaaagtc tctgccctta aggaggatat cgagggcaga 420

ggaagtctgc taacatgcgg tgacgtcgag gagaatcctg gcccaaagct tgccaccatg 480

cgcaaaggcg aagaactgtt taccggcatt gtgccgattc tggtggaact ggatggcgat 540

gtgaacggcc ataaattttt tgtgcgcggc gaaggcgaag gcgatgcgac cattggcaaa 600

ctgagcctga aatttatttg caccaccggc aaactgccgg tgccgtggcc gaccctggtg 660

accaccctga cctatggcgt gcagtgcttt agccgctatc cggatcatat gaaacgccat 720

gattttttta aaagcgcgat gccggaaggc tatgtgcagg aacgcaccat ttattttaaa 780

gatgatggca cctataaaac ccgcgcggaa gtgaaatttg aaggcgatac cctggtgaac 840

cgcattgaac tgaaaggcat tgattttaaa gaagatggca acattctggg ccataaactg 900

gaatataact ttaacagcca taaagtgtat attaccgcgg ataaacagaa caacggcatt 960

aaagcgaact ttaccattcg ccataacgtg gaagatggca gcgtgcagct ggcggatcat 1020

tatcagcaga acaccccgat tggcgatggc ccggttcttc ttcctggcgg ccgctctaga 1080

<210>  70
<211>  1101
<212>  DNA
<213>  Artificial Sequence

<220>
<223>  Synthetic sequence

<400>  70
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcagagga cagggccggc     300

gccggcatca tcgagacccc caaggtgtcc gccctgaagg aaaaagtaag cgcactgaaa     360

gaaaaggtga gcgcgctgaa ggagaaagtg agcgccctga aagagaaagt ctctgccctt     420

aaggaggata tcgagggcag aggaagtctg ctaacatgcg gtgacgtcga ggagaatcct     480

ggcccaaagc ttgccaccat gcgcaaaggc gaagaactgt ttaccggcat tgtgccgatt     540

ctggtggaac tggatggcga tgtgaacggc cataaatttt ttgtgcgcgg cgaaggcgaa     600

ggcgatgcga ccattggcaa actgagcctg aaatttattt gcaccaccgg caaactgccg     660

gtgccgtggc cgaccctggt gaccaccctg acctatggcg tgcagtgctt tagccgctat     720

ccggatcata tgaaacgcca tgattttttt aaaagcgcga tgccggaagg ctatgtgcag     780

gaacgcacca tttattttaa agatgatggc acctataaaa cccgcgcgga agtgaaattt     840

gaaggcgata ccctggtgaa ccgcattgaa ctgaaaggca ttgattttaa agaagatggc     900

aacattctgg gccataaact ggaatataac tttaacagcc ataaagtgta tattaccgcg     960

gataaacaga acaacggcat taaagcgaac tttaccattc gccataacgt ggaagatggc    1020

agcgtgcagc tggcggatca ttatcagcag aacaccccga ttggcgatgg cccggttctt    1080

cttcctggcg gccgctctag a                                              1101

<210>  71
<211>  1101
<212>  DNA
<213>  Artificial Sequence

<220>
<223>  Synthetic sequence

<400>  71
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg 240

gttttgcttg agtatgttac agcggctggc attaccgatg catcagccac cagggccggc 300

tgcgccccca gctacagggt gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 72
<211> 1101
<212> DNA
<213> Artificial Sequence

<220>
<223> Synthetic sequence

<400> 72
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcactgga cagggccggc 300

ggctacatct tcagcagcga caaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 73
<211> 1092
<212> DNA
<213> Artificial Sequence

<220>
<223> Synthetic sequence

<400> 73
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaatcac cactcttggg 300

aaatttggac aaaaggtgtc cgccctgaag gaaaaagtaa gcgcactgaa agaaaaggtg 360

agcgcgctga aggagaaagt gagcgccctg aaagagaaag tctctgccct taaggaggat 420

atcgagggca gaggaagtct gctaacatgc ggtgacgtcg aggagaatcc tggcccaaag 480

cttgccacca tgcgcaaagg cgaagaactg tttaccggca ttgtgccgat tctggtggaa 540

ctggatggcg atgtgaacgg ccataaattt tttgtgcgcg gcgaaggcga aggcgatgcg 600

accattggca aactgagcct gaaatttatt tgcaccaccg gcaaactgcc ggtgccgtgg 660

ccgaccctgg tgaccaccct gacctatggc gtgcagtgct ttagccgcta tccggatcat 720

atgaaacgcc atgatttttt taaaagcgcg atgccggaag gctatgtgca ggaacgcacc 780

atttatttta aagatgatgg cacctataaa acccgcgcgg aagtgaaatt tgaaggcgat 840

accctggtga accgcattga actgaaaggc attgatttta aagaagatgg caacattctg 900

ggccataaac tggaatataa ctttaacagc cataaagtgt atattaccgc ggataaacag 960

aacaacggca ttaaagcgaa ctttaccatt cgccataacg tggaagatgg cagcgtgcag 1020

ctggcggatc attatcagca gaacaccccg attggcgatg gcccggttct tcttcctggc 1080

ggccgctcta ga                                                          1092

<210>  74
<211>  1104
<212>  DNA
<213>  Artificial Sequence

<220>
<223>  Synthetic sequence

<400>  74
atgggctgct gcttcagcaa gaccatggac ctgcctgacg accactacct gtccacccag        60

accatcctgt ccaaggacct gaactccgga ctcagatctg gcagcggtct cgagatggaa       120

gttagcgctc tggaaaaaga agtgtctgca ctcgagaaag aagtaagtgc ccttgagaag       180

gaggtgtccg cactcgagaa ggaggtcagc gccctggaaa aggaaaagcg agaccatatg       240

gttttgcttg agtatgttac agcggctggc attaccgatg catcaagcta cttcgccagc       300

gagcagggcg agatccagtg ggtgaaggtg tccgccctga aggaaaaagt aagcgcactg       360

aaagaaaagg tgagcgcgct gaaggagaaa gtgagcgccc tgaaagagaa agtctctgcc       420

cttaaggagg atatcgaggg cagaggaagt ctgctaacat gcggtgacgt cgaggagaat       480

cctggcccaa agcttgccac catgcgcaaa ggcgaagaac tgtttaccgg cattgtgccg       540

attctggtgg aactggatgg cgatgtgaac ggccataaat tttttgtgcg cggcgaaggc       600

gaaggcgatg cgaccattgg caaactgagc ctgaaattta tttgcaccac cggcaaactg       660

ccggtgccgt ggccgaccct ggtgaccacc ctgacctatg gcgtgcagtg ctttagccgc       720

tatccggatc atatgaaacg ccatgatttt tttaaaagcg cgatgccgga aggctatgtg       780

caggaacgca ccatttattt taaagatgat ggcacctata aaacccgcgc ggaagtgaaa       840

tttgaaggcg ataccctggt gaaccgcatt gaactgaaag gcattgattt taaagaagat       900

ggcaacattc tgggccataa actggaatat aactttaaca gccataaagt gtatattacc       960

gcggataaac agaacaacgg cattaaagcg aactttacca ttcgccataa cgtggaagat      1020

ggcagcgtgc agctggcgga tcattatcag cagaacaccc cgattggcga tggcccggtt      1080

cttcttcctg gcggccgctc taga                                            1104

<210>  75
<211>  1101
<212>  DNA
<213>  Artificial Sequence

<220>
<223>  Synthetic sequence

<400>  75
atgggctgct gcttcagcaa gaccatggac ctgcctgacg accactacct gtccacccag        60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaaggga gctgaacggc 300

ggcgcctaca ccaggtacgt gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 76
<211> 1101
<212> DNA
<213> Artificial Sequence

<220>
<223> Synthetic sequence

<400> 76
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcattcac cctgaagggc 300

ggcgccccca ccaaggtgac caaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 77
<211> 1101
<212> DNA
<213> Artificial Sequence

<220>
<223> Synthetic sequence

<400> 77
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaatcgc cctgaagggc 300

ggcaagatcg tgaacaactg gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 78
<211> 1101
<212> DNA
<213> Artificial Sequence

<220>
<223> Synthetic sequence

<400> 78
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaaccag cgccgtgctg 300

cagagcggct tcaggaagat gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 79
<211> 1101
<212> DNA
<213> Artificial Sequence

<220>
<223> Synthetic sequence

<400>  79
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcaagcgg cgtgaccttc     300

cagagcgccg tgaagaggac caaggtgtcc gccctgaagg aaaaagtaag cgcactgaaa     360

gaaaaggtga gcgcgctgaa ggagaaagtg agcgccctga aagagaaagt ctctgccctt     420

aaggaggata tcgagggcag aggaagtctg ctaacatgcg gtgacgtcga ggagaatcct     480

ggcccaaagc ttgccaccat gcgcaaaggc gaagaactgt ttaccggcat tgtgccgatt     540

ctggtggaac tggatggcga tgtgaacggc cataaatttt ttgtgcgcgg cgaaggcgaa     600

ggcgatgcga ccattggcaa actgagcctg aaatttattt gcaccaccgg caaactgccg     660

gtgccgtggc cgaccctggt gaccaccctg acctatggcg tgcagtgctt tagccgctat     720

ccggatcata tgaaacgcca tgattttttt aaaagcgcga tgccggaagg ctatgtgcag     780

gaacgcacca tttattttaa agatgatggc acctataaaa cccgcgcgga agtgaaattt     840

gaaggcgata ccctggtgaa ccgcattgaa ctgaaaggca ttgattttaa agaagatggc     900

aacattctgg gccataaact ggaatataac tttaacagcc ataaagtgta tattaccgcg     960

gataaacaga acaacggcat taaagcgaac tttaccattc gccataacgt ggaagatggc    1020

agcgtgcagc tggcggatca ttatcagcag aacaccccga ttggcgatgg cccggttctt    1080

cttcctggcg gccgctctag a                                              1101

<210>  80
<211>  1101
<212>  DNA
<213>  Artificial Sequence

<220>
<223>  Synthetic sequence

<400>  80
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcaaaggt ggccaccgtg     300

cagagcaaga tgagcgacgt gaaggtgtcc gccctgaagg aaaaagtaag cgcactgaaa     360

gaaaaggtga gcgcgctgaa ggagaaagtg agcgccctga aagagaaagt ctctgccctt     420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 81
<211> 1101
<212> DNA
<213> Artificial Sequence

<220>
<223> Synthetic sequence

<400> 81
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaagcgc cgtgaagctg 300

cagaacaacg agctgagccc caaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 82
<211> 1101
<212> DNA
<213> Artificial Sequence

<220>
<223> Synthetic sequence

<400> 82
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcagccac cgtgaggctg 300

caggccggca acgccaccga gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 83
<211> 1101
<212> DNA
<213> Artificial Sequence

<220>
<223>  Synthetic sequence

<400>  83
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcaaggga gcccatgctg     300

cagagcgccg acgcccagag caaggtgtcc gccctgaagg aaaaagtaag cgcactgaaa     360

gaaaaggtga gcgcgctgaa ggagaaagtg agcgccctga aagagaaagt ctctgccctt     420

aaggaggata tcgagggcag aggaagtctg ctaacatgcg gtgacgtcga ggagaatcct     480

ggcccaaagc ttgccaccat gcgcaaaggc gaagaactgt ttaccggcat tgtgccgatt     540

ctggtggaac tggatggcga tgtgaacggc cataaatttt ttgtgcgcgg cgaaggcgaa     600

ggcgatgcga ccattggcaa actgagcctg aaatttattt gcaccaccgg caaactgccg     660

gtgccgtggc cgaccctggt gaccaccctg acctatggcg tgcagtgctt tagccgctat     720

ccggatcata tgaaacgcca tgattttttt aaaagcgcga tgccggaagg ctatgtgcag     780

gaacgcacca tttattttaa agatgatggc acctataaaa cccgcgcgga agtgaaattt     840

gaaggcgata ccctggtgaa ccgcattgaa ctgaaaggca ttgattttaa agaagatggc     900

aacattctgg gccataaact ggaatataac tttaacagcc ataaagtgta tattaccgcg     960

gataaacaga acaacggcat taaagcgaac tttaccattc gccataacgt ggaagatggc    1020

agcgtgcagc tggcggatca ttatcagcag aacaccccga ttggcgatgg cccggttctt    1080

cttcctggcg gccgctctag a                                              1101

<210>  84
<211>  1101
<212>  DNA
<213>  Artificial Sequence

<220>
<223>  Synthetic sequence

<400>  84
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcaaggga gaggaagagg     300

aggggcgccg acaccagcat caaggtgtcc gccctgaagg aaaaagtaag cgcactgaaa     360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 85
<211> 1101
<212> DNA
<213> Artificial Sequence

<220>
<223> Synthetic sequence

<400> 85
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcaaccag gagcggcaag 300

aggagctggc cccccagcga gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 86
<211> 1101
<212> DNA
<213> Artificial Sequence

<220>
<223> Synthetic sequence

<400> 86
atgggctgct gcttcagcaa gaccatggac ctgcctgacg accactacct gtccacccag 60

accatcctgt ccaaggacct gaactccgga ctcagatctg gcagcggtct cgagatggaa 120

gttagcgctc tggaaaaaga agtgtctgca ctcgagaaag aagtaagtgc ccttgagaag 180

gaggtgtccg cactcgagaa ggaggtcagc gccctggaaa aggaaaagcg agaccatatg 240

gttttgcttg agtatgttac agcggctggc attaccgatg catcagagcc cgagaagcag 300

aggagccccc aggacaacca gaaggtgtcc gccctgaagg aaaaagtaag cgcactgaaa 360

gaaaaggtga gcgcgctgaa ggagaaagtg agcgccctga aagagaaagt ctctgccctt 420

aaggaggata tcgagggcag aggaagtctg ctaacatgcg gtgacgtcga ggagaatcct 480

ggcccaaagc ttgccaccat gcgcaaaggc gaagaactgt ttaccggcat tgtgccgatt 540

ctggtggaac tggatggcga tgtgaacggc cataaatttt ttgtgcgcgg cgaaggcgaa 600

ggcgatgcga ccattggcaa actgagcctg aaatttattt gcaccaccgg caaactgccg 660

gtgccgtggc cgaccctggt gaccaccctg acctatggcg tgcagtgctt tagccgctat 720

ccggatcata tgaaacgcca tgattttttt aaaagcgcga tgccggaagg ctatgtgcag 780

gaacgcacca tttattttaa agatgatggc acctataaaa cccgcgcgga agtgaaattt 840

gaaggcgata ccctggtgaa ccgcattgaa ctgaaaggca ttgattttaa agaagatggc 900

aacattctgg gccataaact ggaatataac tttaacagcc ataaagtgta tattaccgcg 960

gataaacaga acaacggcat taaagcgaac tttaccattc gccataacgt ggaagatggc 1020

agcgtgcagc tggcggatca ttatcagcag aacaccccga ttggcgatgg cccggttctt 1080

cttcctggcg gccgctctag a 1101

<210> 87
<211> 1101

<212>  DNA
<213>  Artificial Sequence

<220>
<223>  Synthetic sequence

<400>  87
atgggctgct gcttcagcaa gaccatggac ctgcctgacg accactacct gtccacccag      60

accatcctgt ccaaggacct gaactccgga ctcagatctg gcagcggtct cgagatggaa     120

gttagcgctc tggaaaaaga agtgtctgca ctcgagaaag aagtaagtgc ccttgagaag     180

gaggtgtccg cactcgagaa ggaggtcagc gccctggaaa aggaaaagcg agaccatatg     240

gttttgcttg agtatgttac agcggctggc attaccgatg catcaggcct ggtgaagagg     300

aggggcggcg gcaccggcga gaaggtgtcc gccctgaagg aaaaagtaag cgcactgaaa     360

gaaaaggtga gcgcgctgaa ggagaaagtg agcgccctga aagagaaagt ctctgccctt     420

aaggaggata tcgagggcag aggaagtctg ctaacatgcg gtgacgtcga ggagaatcct     480

ggcccaaagc ttgccaccat gcgcaaaggc gaagaactgt ttaccggcat tgtgccgatt     540

ctggtggaac tggatggcga tgtgaacggc cataaatttt ttgtgcgcgg cgaaggcgaa     600

ggcgatgcga ccattggcaa actgagcctg aaatttattt gcaccaccgg caaactgccg     660

gtgccgtggc cgaccctggt gaccaccctg acctatggcg tgcagtgctt tagccgctat     720

ccggatcata tgaaacgcca tgattttttt aaaagcgcga tgccggaagg ctatgtgcag     780

gaacgcacca tttattttaa agatgatggc acctataaaa cccgcgcgga agtgaaattt     840

gaaggcgata ccctggtgaa ccgcattgaa ctgaaaggca ttgattttaa agaagatggc     900

aacattctgg gccataaact ggaatataac tttaacagcc ataaagtgta tattaccgcg     960

gataaacaga acaacggcat taaagcgaac tttaccattc gccataacgt ggaagatggc    1020

agcgtgcagc tggcggatca ttatcagcag aacaccccga ttggcgatgg cccggttctt    1080

cttcctggcg gccgctctag a                                              1101


<210>  88
<211>  1077
<212>  DNA
<213>  Artificial Sequence

<220>
<223>  Synthetic sequence

<400>  88
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc aagggagctg aacggcggcg cctacaccag gtacgtgaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 89
<211> 1077
<212> DNA
<213> Artificial Sequence

<220>
<223> Synthetic sequence

<400> 89
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc attcaccctg aagggcggcg cccccaccaa ggtgaccaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 90
<211> 1077
<212> DNA
<213> Artificial Sequence

<220>
<223> Synthetic sequence

<400> 90
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aatcgccctg aagggcggca agatcgtgaa caactggaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 91
<211> 1077
<212> DNA
<213> Artificial Sequence

<220>
<223>  Synthetic sequence

<400>  91
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc aaccagcgcc gtgctgcaga gcggcttcag gaagatgaag     300

gtgtccgccc tgaaggaaaa agtaagcgca ctgaaagaaa aggtgagcgc gctgaaggag     360

aaagtgagcg ccctgaaaga gaaagtctct gcccttaagg aggatatcga gggcagagga     420

agtctgctaa catgcggtga cgtcgaggag aatcctggcc caaagcttgc caccatgcgc     480

aaaggcgaag aactgtttac cggcattgtg ccgattctgg tggaactgga tggcgatgtg     540

aacggccata aattttttgt gcgcggcgaa ggcgaaggcg atgcgaccat tggcaaactg     600

agcctgaaat ttatttgcac caccggcaaa ctgccggtgc cgtggccgac cctggtgacc     660

accctgacct atggcgtgca gtgctttagc cgctatccgg atcatatgaa acgccatgat     720

ttttttaaaa gcgcgatgcc ggaaggctat gtgcaggaac gcaccattta ttttaaagat     780

gatggcacct ataaaacccg cgcggaagtg aaatttgaag gcgataccct ggtgaaccgc     840

attgaactga aaggcattga ttttaaagaa gatggcaaca ttctgggcca taaactggaa     900

tataacttta acagccataa agtgtatatt accgcggata aacagaacaa cggcattaaa     960

gcgaacttta ccattcgcca taacgtggaa gatggcagcg tgcagctggc ggatcattat    1020

cagcagaaca ccccgattgg cgatggcccg gttcttcttc ctggcggccg ctctaga       1077


<210>  92
<211>  1077
<212>  DNA
<213>  Artificial Sequence

<220>
<223>  Synthetic sequence

<400>  92
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc aagcggcgtg accttccaga gcgccgtgaa gaggaccaag     300

gtgtccgccc tgaaggaaaa agtaagcgca ctgaaagaaa aggtgagcgc gctgaaggag     360

aaagtgagcg ccctgaaaga gaaagtctct gcccttaagg aggatatcga gggcagagga     420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 93
<211> 1077
<212> DNA
<213> Artificial Sequence

<220>
<223> Synthetic sequence

<400> 93
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aaaggtggcc accgtgcaga gcaagatgag cgacgtgaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 94
<211> 1077
<212> DNA
<213> Artificial Sequence

<220>
<223> Synthetic sequence

<400> 94
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aagcgccgtg aagctgcaga acaacgagct gagccccaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 95
<211> 1077
<212> DNA
<213> Artificial Sequence

<220>
<223> Synthetic sequence

<400> 95
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc agccaccgtg aggctgcagg ccggcaacgc caccgagaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 96
<211> 1077
<212> DNA
<213> Artificial Sequence

<220>
<223> Synthetic sequence

<400> 96
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aaggagcccc atgctgcaga gcgccgacgc ccagagcaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 97
<211> 1077
<212> DNA
<213> Artificial Sequence

<220>
<223> Synthetic sequence

<400> 97
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aaaggagagg aagaggaggg gcgccgacac cagcatcaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210>  98
<211>  1077
<212>  DNA
<213>  Artificial Sequence

<220>
<223>  Synthetic sequence

<400>  98
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc aaccaggagc ggcaagagga gctggccccc cagcgagaag     300

gtgtccgccc tgaaggaaaa agtaagcgca ctgaaagaaa aggtgagcgc gctgaaggag     360

aaagtgagcg ccctgaaaga gaaagtctct gcccttaagg aggatatcga gggcagagga     420

agtctgctaa catgcggtga cgtcgaggag aatcctggcc caaagcttgc caccatgcgc     480

aaaggcgaag aactgtttac cggcattgtg ccgattctgg tggaactgga tggcgatgtg     540

aacggccata aattttttgt gcgcggcgaa ggcgaaggcg atgcgaccat tggcaaactg     600

agcctgaaat ttatttgcac caccggcaaa ctgccggtgc cgtggccgac cctggtgacc     660

accctgacct atggcgtgca gtgctttagc cgctatccgg atcatatgaa acgccatgat     720

ttttttaaaa gcgcgatgcc ggaaggctat gtgcaggaac gcaccattta ttttaaagat     780

gatggcacct ataaaacccg cgcggaagtg aaatttgaag gcgataccct ggtgaaccgc     840

attgaactga aaggcattga ttttaaagaa gatggcaaca ttctgggcca taaactggaa     900

tataacttta acagccataa agtgtatatt accgcggata aacagaacaa cggcattaaa     960

gcgaacttta ccattcgcca taacgtggaa gatggcagcg tgcagctggc ggatcattat    1020

cagcagaaca ccccgattgg cgatggcccg gttcttcttc ctggcggccg ctctaga       1077


<210>  99
<211>  1077
<212>  DNA
<213>  Artificial Sequence

<220>
<223>  Synthetic sequence

<400>  99
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg 240

gctggcatta ccgatgcatc agagcccgag aagcagagga gcccccagga caaccagaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 100
<211> 1077
<212> DNA
<213> Artificial Sequence

<220>
<223> Synthetic sequence

<400> 100
atggacctgc ctgacgacca ctacctgtcc acccagacca tcctgtccaa ggacctgaac 60

tccggactca gatctggcag cggtctcgag atggaagtta gcgctctgga aaaagaagtg 120

tctgcactcg agaaagaagt aagtgccctt gagaaggagg tgtccgcact cgagaaggag 180

gtcagcgccc tggaaaagga aaagcgagac catatggttt tgcttgagta tgttacagcg 240

gctggcatta ccgatgcatc aggcctggtg aagaggaggg gcggcggcac cggcgagaag 300

gtgtccgccc tgaaggaaaa agtaagcgca ctgaaagaaa aggtgagcgc gctgaaggag 360

aaagtgagcg ccctgaaaga gaaagtctct gcccttaagg aggatatcga gggcagagga 420

agtctgctaa catgcggtga cgtcgaggag aatcctggcc caaagcttgc caccatgcgc 480

aaaggcgaag aactgtttac cggcattgtg ccgattctgg tggaactgga tggcgatgtg 540

aacggccata aattttttgt gcgcggcgaa ggcgaaggcg atgcgaccat tggcaaactg 600

agcctgaaat ttatttgcac caccggcaaa ctgccggtgc cgtggccgac cctggtgacc 660

accctgacct atggcgtgca gtgctttagc cgctatccgg atcatatgaa acgccatgat 720

ttttttaaaa gcgcgatgcc ggaaggctat gtgcaggaac gcaccattta ttttaaagat 780

gatggcacct ataaaacccg cgcggaagtg aaatttgaag gcgataccct ggtgaaccgc 840

attgaactga aaggcattga ttttaaagaa gatggcaaca ttctgggcca taaactggaa 900

tataacttta acagccataa agtgtatatt accgcggata aacagaacaa cggcattaaa 960

gcgaacttta ccattcgcca taacgtggaa gatggcagcg tgcagctggc ggatcattat 1020

cagcagaaca ccccgattgg cgatggcccg gttcttcttc ctggcggccg ctctaga 1077

<210> 101
<211> 8638
<212> DNA
<213> Artificial Sequence

<220>
<223> Synthetic sequence

<400> 101
aatgtagtct tatgcaatac tcttgtagtc ttgcaacatg gtaacgatga gttagcaaca 60

tgccttacaa ggagagaaaa agcaccgtgc atgccgattg gtggaagtaa ggtggtacga 120

tcgtgcctta ttaggaaggc aacagacggg tctgacatgg attggacgaa ccactgaatt 180

gccgcattgc agagatattg tatttaagtg cctagctcga tacataaacg ggtctctctg 240

gttagaccag atctgagcct gggagctctc tggctaacta gggaacccac tgcttaagcc 300

tcaataaagc ttgccttgag tgcttcaagt agtgtgtgcc cgtctgttgt gtgactctgg 360

taactagaga tccctcagac ccttttagtc agtgtggaaa atctctagca gtggcgcccg 420

aacagggact tgaaagcgaa agggaaacca gaggagctct ctcgacgcag gactcggctt 480

gctgaagcgc gcacggcaag aggcgagggg cggcgactgg tgagtacgcc aaaaattttg 540

actagcggag gctagaagga gagagatggg tgcgagagcg tcagtattaa gcgggggaga 600

attagatcgc gatgggaaaa aattcggtta aggccagggg gaaagaaaaa atataaatta 660

aaacatatag tatgggcaag cagggagcta gaacgattcg cagttaatcc tggcctgtta 720

gaaacatcag aaggctgtag acaaatactg ggacagctac aaccatccct tcagacagga 780

tcagaagaac ttagatcatt atataataca gtagcaaccc tctattgtgt gcatcaaagg 840

atagagataa aagacaccaa ggaagcttta gacaagatag aggaagagca aaacaaaagt 900

aagaccaccg cacagcaagc ggccgctgat cttcagacct ggaggaggag atatgaggga 960

caattggaga agtgaattat ataaatataa agtagtaaaa attgaaccat taggagtagc 1020

acccaccaag gcaaagagaa gagtggtgca gagagaaaaa agagcagtgg gaataggagc 1080

tttgttcctt gggttcttgg gagcagcagg aagcactatg ggcgcagcgt caatgacgct 1140

gacggtacag gccagacaat tattgtctgg tatagtgcag cagcagaaca atttgctgag 1200

ggctattgag gcgcaacagc atctgttgca actcacagtc tggggcatca agcagctcca 1260

ggcaagaatc ctggctgtgg aaagatacct aaaggatcaa cagctcctgg ggatttgggg 1320

ttgctctgga aaactcattt gcaccactgc tgtgccttgg aatgctagtt ggagtaataa 1380

atctctggaa cagatttgga atcacacgac ctggatggag tgggacagag aaattaacaa 1440

ttacacaagc ttaatacact ccttaattga agaatcgcaa aaccagcaag aaaagaatga 1500

acaagaatta ttggaattag ataaatgggc aagtttgtgg aattggttta acataacaaa 1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggtatcggtt 1800

aacttttaaa agaaaagggg ggattggggg gtacagtgca ggggaaagaa tagtagacat 1860

aatagcaaca gacatacaaa ctaaagaatt acaaaaacaa attacaaaat tcaaaatttt 1920

atcgataagc ttgggagttc cgcgttacat aacttacggt aaatggcccg cctggctgac 1980

cgcccaacga cccccgccca ttgacgtcaa taatgacgta tgttcccata gtaacgccaa 2040

tagggacttt ccattgacgt caatgggtgg agtatttacg gtaaactgcc cacttggcag 2100

tacatcaagt gtatcatatg ccaagtacgc cccctattga cgtcaatgac ggtaaatggc 2160

ccgcctggca ttatgcccag tacatgacct tatgggactt tcctacttgg cagtacatct 2220

acgtattagt catcgctatt accatggtga tgcggttttg gcagtacatc aatgggcgtg 2280

gatagcggtt tgactcacgg ggatttccaa gtctccaccc cattgacgtc aatgggagtt 2340

tgttttggca ccaaaatcaa cgggactttc caaaatgtcg taacaactcc gccccattga 2400

cgcaaatggg cggtaggcgt gtacggtggg aggtctatat aagcagagct cgtttagtga 2460

accgtcagat cgcctggaga cgccatccac gctgttttga cctccataga agacaccgcc 2520

cgggcgtacg ggatccatgg accccgtggt ggtgctgggc ctgtgcctga gctgcctgct 2580

gctgctgagc ctgtggaagc agagccacgg cggcggcaag atggacctgc ctgacgacca 2640

ctacctgtcc acccagacca tcctgtccaa ggacctgaac tccggactca gatctggcag 2700

cggtctcgag atggaagtta gcgctctgga aaaagaagtg tctgcactcg agaaagaagt 2760

aagtgccctt gagaaggagg tgtccgcact cgagaaggag gtcagcgccc tggaaaagga 2820

aaagcgagac catatggttt tgcttgagta tgttacagcg gctggcatta ccgatgcatc 2880

aagctacttc gccagcgagc agggcgagat ccagtgggtg aaggtgtccg ccctgaagga 2940

aaaagtaagc gcactgaaag aaaaggtgag cgcgctgaag gagaaagtga gcgccctgaa 3000

agagaaagtc tctgccctta aggaggatat cgagggcaga ggaagtctgc taacatgcgg 3060

tgacgtcgag gagaatcctg gcccaaagct tgccaccatg cgcaaaggcg aagaactgtt 3120

taccggcatt gtgccgattc tggtggaact ggatggcgat gtgaacggcc ataaattttt 3180

tgtgcgcggc gaaggcgaag gcgatgcgac cattggcaaa ctgagcctga aatttatttg 3240

caccaccggc aaactgccgg tgccgtggcc gaccctggtg accaccctga cctatggcgt 3300

gcagtgcttt agccgctatc cggatcatat gaaacgccat gattttttta aaagcgcgat 3360

gccggaaggc tatgtgcagg aacgcaccat ttattttaaa gatgatggca cctataaaac 3420

ccgcgcggaa gtgaaatttg aaggcgatac cctggtgaac cgcattgaac tgaaaggcat 3480

tgattttaaa gaagatggca acattctggg ccataaactg gaatataact ttaacagcca 3540

taaagtgtat attaccgcgg ataaacagaa caacggcatt aaagcgaact ttaccattcg 3600

ccataacgtg gaagatggca gcgtgcagct ggcggatcat tatcagcaga acaccccgat 3660

tggcgatggc ccggttcttc ttcctggcgg ccgctctaga gagggcagag gaagtctgct 3720

aacatgcggt gacgtcgagg agaatcctgg cccagaattg atggtgagca agggcgagga 3780

ggataacatg gccatcatca aggagttcat gcgcttcaag gtgcacatgg agggctccgt 3840

gaacggccac gagttcgaga tcgagggcga gggcgagggc cgcccctacg agggcaccca 3900

gaccgccaag ctgaaggtga ccaagggtgg ccccctgccc ttcgcctggg acatcctgtc 3960

ccctcagttc atgtacggct ccaaggccta cgtgaagcac cccgccgaca tccccgacta 4020

cttgaagctg tccttccccg agggcttcaa gtgggagcgc gtgatgaact tcgaggacgg 4080

cggcgtggtg accgtgaccc aggactcctc cctgcaggac ggcgagttca tctacaaggt 4140

gaagctgcgc ggcaccaact tcccctccga cggccccgta atgcagaaga agaccatggg 4200

ctgggaggcc tcctccgagc ggatgtaccc cgaggacggc gccctgaagg gcgagatcaa 4260

gcagaggctg aagctgaagg acggcggcca ctacgacgct gaggtcaaga ccacctacaa 4320

ggccaagaag cccgtgcagc tgcccggcgc ctacaacgtc aacatcaagt tggacatcac 4380

ctcccacaac gaggactaca ccatcgtgga acagtacgaa cgcgccgagg gccgccactc 4440

caccggcggc atggacgagc tgtacaagta ataacacatc gacaatcaac ctctggatta 4500

caaaatttgt gaaagattga ctggtattct taactatgtt gctcctttta cgctatgtgg 4560

atacgctgct ttaatgcctt tgtatcatgc tattgcttcc cgtatggctt tcattttctc 4620

ctccttgtat aaatcctggt tgctgtctct ttatgaggag ttgtggcccg ttgtcaggca 4680

acgtggcgtg gtgtgcactg tgtttgctga cgcaaccccc actggttggg gcattgccac 4740

cacctgtcag ctcctttccg ggactttcgc tttccccctc cctattgcca cggcggaact 4800

catcgccgcc tgccttgccc gctgctggac aggggctcgg ctgttgggca ctgacaattc 4860

cgtggtgttg tcggggaagc tgacgtcctt tccatggctg ctcgcctgtg ttgccacctg 4920

gattctgcgc gggacgtcct tctgctacgt cccttcggcc ctcaatccag cggaccttcc 4980

ttcccgcggc ctgctgccgg ctctgcggcc tcttccgcgt cttcgccttc gccctcagac 5040

gagtcggatc tccctttggg ccgcctcccc gcctggaatt cgagctcggt acctttaaga 5100

ccaatgactt acaaggcagc tgtagatctt agccactttt taaaagaaaa ggggggactg 5160

gaagggctaa ttcactccca acgaagacaa gatctgcttt ttgcttgtac tgggtctctc 5220

tggttagacc agatctgagc ctgggagctc tctggctaac tagggaaccc actgcttaag 5280

cctcaataaa gcttgccttg agtgcttcaa gtagtgtgtg cccgtctgtt gtgtgactct 5340

ggtaactaga gatccctcag acccttttag tcagtgtgga aaatctctag cagtagtagt 5400

tcatgtcatc ttattattca gtatttataa cttgcaaaga aatgaatatc agagagtgag 5460

aggaacttgt ttattgcagc ttataatggt tacaaataaa gcaatagcat cacaaatttc 5520

acaaataaag catttttttc actgcattct agttgtggtt tgtccaaact catcaatgta 5580

tcttatcatg tctggctcta gctatcccgc ccctaactcc gcccatcccg cccctaactc 5640

cgcccagttc cgcccattct ccgccccatg gctgactaat tttttttatt tatgcagagg 5700

ccgaggccgc ctcggcctct gagctattcc agaagtagtg aggaggcttt tttggaggcc 5760

tagggacgta cccaattcgc cctatagtga gtcgtattac gcgcgctcac tggccgtcgt 5820

tttacaacgt cgtgactggg aaaaccctgg cgttacccaa cttaatcgcc ttgcagcaca 5880

tccccctttc gccagctggc gtaatagcga agaggcccgc accgatcgcc cttcccaaca 5940

gttgcgcagc ctgaatggcg aatgggacgc gccctgtagc ggcgcattaa gcgcggcggg 6000

tgtggtggtt acgcgcagcg tgaccgctac acttgccagc gccctagcgc ccgctccttt 6060

cgctttcttc ccttcctttc tcgccacgtt cgccggcttt ccccgtcaag ctctaaatcg 6120

ggggctccct ttagggttcc gatttagtgc tttacggcac ctcgacccca aaaaacttga 6180

ttagggtgat ggttcacgta gtgggccatc gccctgatag acggtttttc gccctttgac 6240

gttggagtcc acgttcttta atagtggact cttgttccaa actggaacaa cactcaaccc 6300

tatctcggtc tattcttttg atttataagg gattttgccg atttcggcct attggttaaa 6360

aaatgagctg atttaacaaa aatttaacgc gaattttaac aaaatattaa cgcttacaat 6420

ttaggtggca cttttcgggg aaatgtgcgc ggaaccccta tttgtttatt tttctaaata 6480

cattcaaata tgtatccgct catgagacaa taaccctgat aaatgcttca ataatattga 6540

aaaaggaaga gtatgagtat tcaacatttc cgtgtcgccc ttattccctt ttttgcggca 6600

ttttgccttc ctgtttttgc tcacccagaa acgctggtga aagtaaaaga tgctgaagat 6660

cagttgggtg cacgagtggg ttacatcgaa ctggatctca acagcggtaa gatccttgag 6720

agttttcgcc ccgaagaacg ttttccaatg atgagcactt ttaaagttct gctatgtggc 6780

gcggtattat cccgtattga cgccgggcaa gagcaactcg gtcgccgcat acactattct 6840

cagaatgact tggttgagta ctcaccagtc acagaaaagc atcttacgga tggcatgaca 6900

gtaagagaat tatgcagtgc tgccataacc atgagtgata acactgcggc caacttactt 6960

ctgacaacga tcggaggacc gaaggagcta accgcttttt tgcacaacat gggggatcat 7020

gtaactcgcc ttgatcgttg ggaaccggag ctgaatgaag ccataccaaa cgacgagcgt 7080

gacaccacga tgcctgtagc aatggcaaca acgttgcgca aactattaac tggcgaacta 7140

cttactctag cttcccggca acaattaata gactggatgg aggcggataa agttgcagga 7200

ccacttctgc gctcggccct tccggctggc tggtttattg ctgataaatc tggagccggt 7260

gagcgtgggt ctcgcggtat cattgcagca ctggggccag atggtaagcc ctcccgtatc 7320

gtagttatct acacgacggg gagtcaggca actatggatg aacgaaatag acagatcgct 7380

gagataggtg cctcactgat taagcattgg taactgtcag accaagttta ctcatatata 7440

ctttagattg atttaaaact tcatttttaa tttaaaagga tctaggtgaa gatccttttt 7500

gataatctca tgaccaaaat cccttaacgt gagttttcgt tccactgagc gtcagacccc 7560

gtagaaaaga tcaaaggatc ttcttgagat cctttttttc tgcgcgtaat ctgctgcttg 7620

caaacaaaaa aaccaccgct accagcggtg gtttgtttgc cggatcaaga gctaccaact 7680

ctttttccga aggtaactgg cttcagcaga gcgcagatac caaatactgt tcttctagtg 7740

tagccgtagt taggccacca cttcaagaac tctgtagcac cgcctacata cctcgctctg 7800

ctaatcctgt taccagtggc tgctgccagt ggcgataagt cgtgtcttac cgggttggac 7860

tcaagacgat agttaccgga taaggcgcag cggtcgggct gaacgggggg ttcgtgcaca 7920

cagcccagct tggagcgaac gacctacacc gaactgagat acctacagcg tgagctatga 7980

gaaagcgcca cgcttcccga agggagaaag gcggacaggt atccggtaag cggcagggtc 8040

ggaacaggag agcgcacgag ggagcttcca gggggaaacg cctggtatct ttatagtcct 8100

gtcgggtttc gccacctctg acttgagcgt cgatttttgt gatgctcgtc aggggggcgg 8160

agcctatgga aaaacgccag caacgcggcc tttttacggt tcctggcctt ttgctggcct 8220

tttgctcaca tgttctttcc tgcgttatcc cctgattctg tggataaccg tattaccgcc 8280

tttgagtgag ctgataccgc tcgccgcagc cgaacgaccg agcgcagcga gtcagtgagc 8340

gaggaagcgg aagagcgccc aatacgcaaa ccgcctctcc ccgcgcgttg gccgattcat 8400

taatgcagct ggcacgacag gtttcccgac tggaaagcgg gcagtgagcg caacgcaatt 8460

aatgtgagtt agctcactca ttaggcaccc caggctttac actttatgct tccggctcgt 8520

atgttgtgtg gaattgtgag cggataacaa tttcacacag gaaacagcta tgaccatgat 8580

tacgccaagc gcgcaattaa ccctcactaa agggaacaaa agctggagct gcaagctt 8638

<210> 102
<211> 8578
<212> DNA

<213>  Artificial Sequence

<220>
<223>  Synthetic sequence

<400>  102
aatgtagtct tatgcaatac tcttgtagtc ttgcaacatg gtaacgatga gttagcaaca      60

tgccttacaa ggagagaaaa agcaccgtgc atgccgattg gtggaagtaa ggtggtacga     120

tcgtgcctta ttaggaaggc aacagacggg tctgacatgg attggacgaa ccactgaatt     180

gccgcattgc agagatattg tatttaagtg cctagctcga tacataaacg ggtctctctg     240

gttagaccag atctgagcct gggagctctc tggctaacta gggaacccac tgcttaagcc     300

tcaataaagc ttgccttgag tgcttcaagt agtgtgtgcc cgtctgttgt gtgactctgg     360

taactagaga tccctcagac ccttttagtc agtgtggaaa atctctagca gtggcgcccg     420

aacagggact tgaaagcgaa agggaaacca gaggagctct ctcgacgcag gactcggctt     480

gctgaagcgc gcacggcaag aggcgagggg cggcgactgg tgagtacgcc aaaaattttg     540

actagcggag gctagaagga gagagatggg tgcgagagcg tcagtattaa gcgggggaga     600

attagatcgc gatgggaaaa aattcggtta aggccagggg gaaagaaaaa atataaatta     660

aaacatatag tatgggcaag cagggagcta gaacgattcg cagttaatcc tggcctgtta     720

gaaacatcag aaggctgtag acaaatactg ggacagctac aaccatccct tcagacagga     780

tcagaagaac ttagatcatt atataataca gtagcaaccc tctattgtgt gcatcaaagg     840

atagagataa aagacaccaa ggaagcttta gacaagatag aggaagagca aaacaaaagt     900

aagaccaccg cacagcaagc ggccgctgat cttcagacct ggaggaggag atatgaggga     960

caattggaga agtgaattat ataaatataa agtagtaaaa attgaaccat taggagtagc    1020

acccaccaag gcaaagagaa gagtggtgca gagagaaaaa agagcagtgg gaataggagc    1080

tttgttcctt gggttcttgg gagcagcagg aagcactatg ggcgcagcgt caatgacgct    1140

gacggtacag gccagacaat tattgtctgg tatagtgcag cagcagaaca atttgctgag    1200

ggctattgag gcgcaacagc atctgttgca actcacagtc tggggcatca agcagctcca    1260

ggcaagaatc ctggctgtgg aaagatacct aaaggatcaa cagctcctgg ggatttgggg    1320

ttgctctgga aaactcattt gcaccactgc tgtgccttgg aatgctagtt ggagtaataa    1380

atctctggaa cagatttgga atcacacgac ctggatggag tgggacagag aaattaacaa    1440

ttacacaagc ttaatacact ccttaattga agaatcgcaa aaccagcaag aaaagaatga    1500

acaagaatta ttggaattag ataaatgggc aagtttgtgg aattggttta acataacaaa    1560

ttggctgtgg tatataaaat tattcataat gatagtagga ggcttggtag gtttaagaat    1620

agtttttgct gtactttcta tagtgaatag agttaggcag ggatattcac cattatcgtt    1680

tcagacccac ctcccaaccc cgaggggacc cgacaggccc gaaggaatag aagaagaagg    1740

tggagagaga gacagagaca gatccattcg attagtgaac ggatctcgac ggtatcggtt 1800

aacttttaaa agaaaagggg ggattggggg gtacagtgca ggggaaagaa tagtagacat 1860

aatagcaaca gacatacaaa ctaaagaatt acaaaaacaa attacaaaat tcaaaatttt 1920

atcgataagc ttgggagttc cgcgttacat aacttacggt aaatggcccg cctggctgac 1980

cgcccaacga cccccgccca ttgacgtcaa taatgacgta tgttcccata gtaacgccaa 2040

tagggacttt ccattgacgt caatgggtgg agtatttacg gtaaactgcc cacttggcag 2100

tacatcaagt gtatcatatg ccaagtacgc cccctattga cgtcaatgac ggtaaatggc 2160

ccgcctggca ttatgcccag tacatgacct tatgggactt tcctacttgg cagtacatct 2220

acgtattagt catcgctatt accatggtga tgcggttttg gcagtacatc aatgggcgtg 2280

gatagcggtt tgactcacgg ggatttccaa gtctccaccc cattgacgtc aatgggagtt 2340

tgttttggca ccaaaatcaa cgggactttc caaaatgtcg taacaactcc gccccattga 2400

cgcaaatggg cggtaggcgt gtacggtggg aggtctatat aagcagagct cgtttagtga 2460

accgtcagat cgcctggaga cgccatccac gctgttttga cctccataga agacaccgcc 2520

cgggcgtacg ggatccatgg gctgctgctt cagcaagacc atggacctgc ctgacgacca 2580

ctacctgtcc acccagacca tcctgtccaa ggacctgaac tccggactca gatctggcag 2640

cggtctcgag atggaagtta gcgctctgga aaaagaagtg tctgcactcg agaaagaagt 2700

aagtgccctt gagaaggagg tgtccgcact cgagaaggag gtcagcgccc tggaaaagga 2760

aaagcgagac catatggttt tgcttgagta tgttacagcg gctggcatta ccgatgcatc 2820

aagctacttc gccagcgagc agggcgagat ccagtgggtg aaggtgtccg ccctgaagga 2880

aaaagtaagc gcactgaaag aaaaggtgag cgcgctgaag gagaaagtga gcgccctgaa 2940

agagaaagtc tctgccctta aggaggatat cgagggcaga ggaagtctgc taacatgcgg 3000

tgacgtcgag gagaatcctg gcccaaagct tgccaccatg cgcaaaggcg aagaactgtt 3060

taccggcatt gtgccgattc tggtggaact ggatggcgat gtgaacggcc ataaattttt 3120

tgtgcgcggc gaaggcgaag gcgatgcgac cattggcaaa ctgagcctga aatttatttg 3180

caccaccggc aaactgccgg tgccgtggcc gaccctggtg accaccctga cctatggcgt 3240

gcagtgcttt agccgctatc cggatcatat gaaacgccat gattttttta aaagcgcgat 3300

gccggaaggc tatgtgcagg aacgcaccat ttattttaaa gatgatggca cctataaaac 3360

ccgcgcggaa gtgaaatttg aaggcgatac cctggtgaac cgcattgaac tgaaaggcat 3420

tgattttaaa gaagatggca acattctggg ccataaactg gaatataact ttaacagcca 3480

taaagtgtat attaccgcgg ataaacagaa caacggcatt aaagcgaact ttaccattcg 3540

ccataacgtg gaagatggca gcgtgcagct ggcggatcat tatcagcaga acaccccgat 3600

tggcgatggc ccggttcttc ttcctggcgg ccgctctaga gagggcagag gaagtctgct 3660

aacatgcggt gacgtcgagg agaatcctgg cccagaattg atggtgagca agggcgagga 3720

ggataacatg gccatcatca aggagttcat gcgcttcaag gtgcacatgg agggctccgt 3780

gaacggccac gagttcgaga tcgagggcga gggcgagggc cgcccctacg agggcaccca 3840

gaccgccaag ctgaaggtga ccaagggtgg ccccctgccc ttcgcctggg acatcctgtc 3900

ccctcagttc atgtacggct ccaaggccta cgtgaagcac cccgccgaca tccccgacta 3960

cttgaagctg tccttccccg agggcttcaa gtgggagcgc gtgatgaact tcgaggacgg 4020

cggcgtggtg accgtgaccc aggactcctc cctgcaggac ggcgagttca tctacaaggt 4080

gaagctgcgc ggcaccaact tcccctccga cggccccgta atgcagaaga agaccatggg 4140

ctgggaggcc tcctccgagc ggatgtaccc cgaggacggc gccctgaagg gcgagatcaa 4200

gcagaggctg aagctgaagg acggcggcca ctacgacgct gaggtcaaga ccacctacaa 4260

ggccaagaag cccgtgcagc tgcccggcgc ctacaacgtc aacatcaagt tggacatcac 4320

ctcccacaac gaggactaca ccatcgtgga acagtacgaa cgcgccgagg gccgccactc 4380

caccggcggc atggacgagc tgtacaagta ataacacatc gacaatcaac ctctggatta 4440

caaaatttgt gaaagattga ctggtattct taactatgtt gctcctttta cgctatgtgg 4500

atacgctgct ttaatgcctt tgtatcatgc tattgcttcc cgtatggctt tcattttctc 4560

ctccttgtat aaatcctggt tgctgtctct ttatgaggag ttgtggcccg ttgtcaggca 4620

acgtggcgtg gtgtgcactg tgtttgctga cgcaaccccc actggttggg gcattgccac 4680

cacctgtcag ctcctttccg ggactttcgc tttccccctc cctattgcca cggcggaact 4740

catcgccgcc tgccttgccc gctgctggac aggggctcgg ctgttgggca ctgacaattc 4800

cgtggtgttg tcggggaagc tgacgtcctt tccatggctg ctcgcctgtg ttgccacctg 4860

gattctgcgc gggacgtcct tctgctacgt cccttcggcc ctcaatccag cggaccttcc 4920

ttcccgcggc ctgctgccgg ctctgcggcc tcttccgcgt cttcgccttc gccctcagac 4980

gagtcggatc tccctttggg ccgcctcccc gcctggaatt cgagctcggt acctttaaga 5040

ccaatgactt acaaggcagc tgtagatctt agccactttt taaaagaaaa ggggggactg 5100

gaagggctaa ttcactccca acgaagacaa gatctgcttt ttgcttgtac tgggtctctc 5160

tggttagacc agatctgagc ctgggagctc tctggctaac tagggaaccc actgcttaag 5220

cctcaataaa gcttgccttg agtgcttcaa gtagtgtgtg cccgtctgtt gtgtgactct 5280

ggtaactaga gatccctcag acccttttag tcagtgtgga aaatctctag cagtagtagt 5340

tcatgtcatc ttattattca gtatttataa cttgcaaaga aatgaatatc agagagtgag 5400

aggaacttgt ttattgcagc ttataatggt tacaaataaa gcaatagcat cacaaatttc 5460

acaaataaag catttttttc actgcattct agttgtggtt tgtccaaact catcaatgta 5520

tcttatcatg tctggctcta gctatcccgc ccctaactcc gcccatcccg cccctaactc 5580

cgcccagttc cgcccattct ccgccccatg gctgactaat tttttttatt tatgcagagg 5640

ccgaggccgc ctcggcctct gagctattcc agaagtagtg aggaggcttt tttggaggcc 5700

tagggacgta cccaattcgc cctatagtga gtcgtattac gcgcgctcac tggccgtcgt 5760

tttacaacgt cgtgactggg aaaaccctgg cgttacccaa cttaatcgcc ttgcagcaca 5820

tccccctttc gccagctggc gtaatagcga agaggcccgc accgatcgcc cttcccaaca 5880

gttgcgcagc ctgaatggcg aatgggacgc gccctgtagc ggcgcattaa gcgcggcggg 5940

tgtggtggtt acgcgcagcg tgaccgctac acttgccagc gccctagcgc ccgctccttt 6000

cgctttcttc ccttcctttc tcgccacgtt cgccggcttt ccccgtcaag ctctaaatcg 6060

ggggctccct ttagggttcc gatttagtgc tttacggcac ctcgacccca aaaaacttga 6120

ttagggtgat ggttcacgta gtgggccatc gccctgatag acggtttttc gccctttgac 6180

gttggagtcc acgttcttta atagtggact cttgttccaa actggaacaa cactcaaccc 6240

tatctcggtc tattcttttg atttataagg gattttgccg atttcggcct attggttaaa 6300

aaatgagctg atttaacaaa aatttaacgc gaattttaac aaaatattaa cgcttacaat 6360

ttaggtggca cttttcgggg aaatgtgcgc ggaaccccta tttgtttatt tttctaaata 6420

cattcaaata tgtatccgct catgagacaa taaccctgat aaatgcttca ataatattga 6480

aaaaggaaga gtatgagtat tcaacatttc cgtgtcgccc ttattccctt ttttgcggca 6540

ttttgccttc ctgtttttgc tcacccagaa acgctggtga aagtaaaaga tgctgaagat 6600

cagttgggtg cacgagtggg ttacatcgaa ctggatctca acagcggtaa gatccttgag 6660

agttttcgcc ccgaagaacg ttttccaatg atgagcactt ttaaagttct gctatgtggc 6720

gcggtattat cccgtattga cgccgggcaa gagcaactcg gtcgccgcat acactattct 6780

cagaatgact tggttgagta ctcaccagtc acagaaaagc atcttacgga tggcatgaca 6840

gtaagagaat tatgcagtgc tgccataacc atgagtgata acactgcggc caacttactt 6900

ctgacaacga tcggaggacc gaaggagcta accgcttttt tgcacaacat gggggatcat 6960

gtaactcgcc ttgatcgttg ggaaccggag ctgaatgaag ccataccaaa cgacgagcgt 7020

gacaccacga tgcctgtagc aatggcaaca acgttgcgca aactattaac tggcgaacta 7080

cttactctag cttcccggca acaattaata gactggatgg aggcggataa agttgcagga 7140

ccacttctgc gctcggccct tccggctggc tggtttattg ctgataaatc tggagccggt 7200

gagcgtgggt ctcgcggtat cattgcagca ctggggccag atggtaagcc ctcccgtatc 7260

gtagttatct acacgacggg gagtcaggca actatggatg aacgaaatag acagatcgct 7320

gagataggtg cctcactgat taagcattgg taactgtcag accaagttta ctcatatata 7380

ctttagattg atttaaaact tcatttttaa tttaaaagga tctaggtgaa gatccttttt 7440

gataatctca tgaccaaaat cccttaacgt gagttttcgt tccactgagc gtcagacccc 7500

gtagaaaaga tcaaaggatc ttcttgagat cctttttttc tgcgcgtaat ctgctgcttg 7560

caaacaaaaa aaccaccgct accagcggtg gtttgtttgc cggatcaaga gctaccaact 7620

ctttttccga aggtaactgg cttcagcaga gcgcagatac caaatactgt tcttctagtg 7680

tagccgtagt taggccacca cttcaagaac tctgtagcac cgcctacata cctcgctctg 7740

ctaatcctgt taccagtggc tgctgccagt ggcgataagt cgtgtcttac cgggttggac 7800

tcaagacgat agttaccgga taaggcgcag cggtcgggct gaacgggggg ttcgtgcaca 7860

cagcccagct tggagcgaac gacctacacc gaactgagat acctacagcg tgagctatga 7920

gaaagcgcca cgcttcccga agggagaaag gcggacaggt atccggtaag cggcagggtc 7980

ggaacaggag agcgcacgag ggagcttcca gggggaaacg cctggtatct ttatagtcct 8040

gtcgggtttc gccacctctg acttgagcgt cgatttttgt gatgctcgtc aggggggcgg 8100

agcctatgga aaaacgccag caacgcggcc tttttacggt tcctggcctt ttgctggcct 8160

tttgctcaca tgttctttcc tgcgttatcc cctgattctg tggataaccg tattaccgcc 8220

tttgagtgag ctgataccgc tcgccgcagc cgaacgaccg agcgcagcga gtcagtgagc 8280

gaggaagcgg aagagcgccc aatacgcaaa ccgcctctcc ccgcgcgttg gccgattcat 8340

taatgcagct ggcacgacag gtttcccgac tggaaagcgg gcagtgagcg caacgcaatt 8400

aatgtgagtt agctcactca ttaggcaccc caggctttac actttatgct tccggctcgt 8460

atgttgtgtg gaattgtgag cggataacaa tttcacacag gaaacagcta tgaccatgat 8520

tacgccaagc gcgcaattaa ccctcactaa agggaacaaa agctggagct gcaagctt 8578

<210> 103
<211> 8554
<212> DNA
<213> Artificial Sequence

<220>
<223> Synthetic sequence

<400> 103
aatgtagtct tatgcaatac tcttgtagtc ttgcaacatg gtaacgatga gttagcaaca 60

tgccttacaa ggagagaaaa agcaccgtgc atgccgattg gtggaagtaa ggtggtacga 120

tcgtgcctta ttaggaaggc aacagacggg tctgacatgg attggacgaa ccactgaatt 180

gccgcattgc agagatattg tatttaagtg cctagctcga tacataaacg ggtctctctg 240

gttagaccag atctgagcct gggagctctc tggctaacta gggaacccac tgcttaagcc 300

tcaataaagc ttgccttgag tgcttcaagt agtgtgtgcc cgtctgttgt gtgactctgg 360

taactagaga tccctcagac ccttttagtc agtgtggaaa atctctagca gtggcgcccg 420

aacagggact tgaaagcgaa agggaaacca gaggagctct ctcgacgcag gactcggctt 480

gctgaagcgc gcacggcaag aggcgagggg cggcgactgg tgagtacgcc aaaaattttg 540

actagcggag gctagaagga gagagatggg tgcgagagcg tcagtattaa gcgggggaga 600

attagatcgc gatgggaaaa aattcggtta aggccagggg gaaagaaaaa atataaatta 660

aaacatatag tatgggcaag cagggagcta gaacgattcg cagttaatcc tggcctgtta 720

gaaacatcag aaggctgtag acaaatactg ggacagctac aaccatccct tcagacagga 780

tcagaagaac ttagatcatt atataataca gtagcaaccc tctattgtgt gcatcaaagg 840

atagagataa aagacaccaa ggaagcttta gacaagatag aggaagagca aaacaaaagt 900

aagaccaccg cacagcaagc ggccgctgat cttcagacct ggaggaggag atatgaggga 960

caattggaga agtgaattat ataaatataa agtagtaaaa attgaaccat taggagtagc 1020

acccaccaag gcaaagagaa gagtggtgca gagagaaaaa agagcagtgg gaataggagc 1080

tttgttcctt gggttcttgg gagcagcagg aagcactatg ggcgcagcgt caatgacgct 1140

gacggtacag gccagacaat tattgtctgg tatagtgcag cagcagaaca atttgctgag 1200

ggctattgag gcgcaacagc atctgttgca actcacagtc tggggcatca agcagctcca 1260

ggcaagaatc ctggctgtgg aaagatacct aaaggatcaa cagctcctgg ggatttgggg 1320

ttgctctgga aaactcattt gcaccactgc tgtgccttgg aatgctagtt ggagtaataa 1380

atctctggaa cagatttgga atcacacgac ctggatggag tgggacagag aaattaacaa 1440

ttacacaagc ttaatacact ccttaattga agaatcgcaa aaccagcaag aaaagaatga 1500

acaagaatta ttggaattag ataaatgggc aagtttgtgg aattggttta acataacaaa 1560

ttggctgtgg tatataaaat tattcataat gatagtagga ggcttggtag gtttaagaat 1620

agtttttgct gtactttcta tagtgaatag agttaggcag ggatattcac cattatcgtt 1680

tcagacccac ctcccaaccc cgaggggacc cgacaggccc gaaggaatag aagaagaagg 1740

tggagagaga gacagagaca gatccattcg attagtgaac ggatctcgac ggtatcggtt 1800

aacttttaaa agaaaagggg ggattggggg gtacagtgca ggggaaagaa tagtagacat 1860

aatagcaaca gacatacaaa ctaaagaatt acaaaaacaa attacaaaat tcaaaatttt 1920

atcgataagc ttgggagttc cgcgttacat aacttacggt aaatggcccg cctggctgac 1980

cgcccaacga cccccgccca ttgacgtcaa taatgacgta tgttcccata gtaacgccaa 2040

tagggacttt ccattgacgt caatgggtgg agtatttacg gtaaactgcc cacttggcag 2100

tacatcaagt gtatcatatg ccaagtacgc cccctattga cgtcaatgac ggtaaatggc 2160

ccgcctggca ttatgcccag tacatgacct tatgggactt tcctacttgg cagtacatct 2220

acgtattagt catcgctatt accatggtga tgcggttttg gcagtacatc aatgggcgtg 2280

gatagcggtt tgactcacgg ggatttccaa gtctccaccc cattgacgtc aatgggagtt 2340

tgttttggca ccaaaatcaa cgggactttc caaaatgtcg taacaactcc gccccattga 2400

cgcaaatggg cggtaggcgt gtacggtggg aggtctatat aagcagagct cgtttagtga 2460

accgtcagat cgcctggaga cgccatccac gctgttttga cctccataga agacaccgcc 2520

cgggcgtacg ggatccatgg acctgcctga cgaccactac ctgtccaccc agaccatcct 2580

gtccaaggac ctgaactccg gactcagatc tggcagcggt ctcgagatgg aagttagcgc 2640

tctggaaaaa gaagtgtctg cactcgagaa agaagtaagt gcccttgaga aggaggtgtc 2700

cgcactcgag aaggaggtca gcgccctgga aaaggaaaag cgagaccata tggttttgct 2760

tgagtatgtt acagcggctg gcattaccga tgcatcaagc tacttcgcca gcgagcaggg 2820

cgagatccag tgggtgaagg tgtccgccct gaaggaaaaa gtaagcgcac tgaaagaaaa 2880

ggtgagcgcg ctgaaggaga aagtgagcgc cctgaaagag aaagtctctg cccttaagga 2940

ggatatcgag ggcagaggaa gtctgctaac atgcggtgac gtcgaggaga atcctggccc 3000

aaagcttgcc accatgcgca aaggcgaaga actgtttacc ggcattgtgc cgattctggt 3060

ggaactggat ggcgatgtga acggccataa attttttgtg cgcggcgaag gcgaaggcga 3120

tgcgaccatt ggcaaactga gcctgaaatt tatttgcacc accggcaaac tgccggtgcc 3180

gtggccgacc ctggtgacca ccctgaccta tggcgtgcag tgctttagcc gctatccgga 3240

tcatatgaaa cgccatgatt tttttaaaag cgcgatgccg gaaggctatg tgcaggaacg 3300

caccatttat tttaaagatg atggcaccta taaaacccgc gcggaagtga aatttgaagg 3360

cgataccctg gtgaaccgca ttgaactgaa aggcattgat tttaaagaag atggcaacat 3420

tctgggccat aaactggaat ataactttaa cagccataaa gtgtatatta ccgcggataa 3480

acagaacaac ggcattaaag cgaactttac cattcgccat aacgtggaag atggcagcgt 3540

gcagctggcg gatcattatc agcagaacac cccgattggc gatggcccgg ttcttcttcc 3600

tggcggccgc tctagagagg gcagaggaag tctgctaaca tgcggtgacg tcgaggagaa 3660

tcctggccca gaattgatgg tgagcaaggg cgaggaggat aacatggcca tcatcaagga 3720

gttcatgcgc ttcaaggtgc acatggaggg ctccgtgaac ggccacgagt tcgagatcga 3780

gggcgagggc gagggccgcc cctacgaggg cacccagacc gccaagctga aggtgaccaa 3840

gggtggcccc ctgcccttcg cctgggacat cctgtcccct cagttcatgt acggctccaa 3900

ggcctacgtg aagcaccccg ccgacatccc cgactacttg aagctgtcct tccccgaggg 3960

cttcaagtgg gagcgcgtga tgaacttcga ggacggcggc gtggtgaccg tgacccagga 4020

ctcctccctg caggacggcg agttcatcta caaggtgaag ctgcgcggca ccaacttccc 4080

ctccgacggc cccgtaatgc agaagaagac catgggctgg gaggcctcct ccgagcggat 4140

gtaccccgag gacggcgccc tgaagggcga gatcaagcag aggctgaagc tgaaggacgg 4200

cggccactac gacgctgagg tcaagaccac ctacaaggcc aagaagcccg tgcagctgcc 4260

cggcgcctac aacgtcaaca tcaagttgga catcacctcc cacaacgagg actacaccat 4320

cgtggaacag tacgaacgcg ccgagggccg ccactccacc ggcggcatgg acgagctgta 4380

caagtaataa cacatcgaca atcaacctct ggattacaaa atttgtgaaa gattgactgg 4440

tattcttaac tatgttgctc cttttacgct atgtggatac gctgctttaa tgcctttgta 4500

tcatgctatt gcttcccgta tggctttcat tttctcctcc ttgtataaat cctggttgct 4560

gtctctttat gaggagttgt ggcccgttgt caggcaacgt ggcgtggtgt gcactgtgtt 4620

tgctgacgca acccccactg gttggggcat tgccaccacc tgtcagctcc tttccgggac 4680

tttcgctttc cccctcccta ttgccacggc ggaactcatc gccgcctgcc ttgcccgctg 4740

ctggacaggg gctcggctgt tgggcactga caattccgtg gtgttgtcgg ggaagctgac 4800

gtcctttcca tggctgctcg cctgtgttgc cacctggatt ctgcgcggga cgtccttctg 4860

ctacgtccct tcggccctca atccagcgga ccttccttcc cgcggcctgc tgccggctct 4920

gcggcctctt ccgcgtcttc gccttcgccc tcagacgagt cggatctccc tttgggccgc 4980

ctccccgcct ggaattcgag ctcggtacct ttaagaccaa tgacttacaa ggcagctgta 5040

gatcttagcc actttttaaa agaaaagggg ggactggaag ggctaattca ctcccaacga 5100

agacaagatc tgctttttgc ttgtactggg tctctctggt tagaccagat ctgagcctgg 5160

gagctctctg gctaactagg gaacccactg cttaagcctc aataaagctt gccttgagtg 5220

cttcaagtag tgtgtgcccg tctgttgtgt gactctggta actagagatc cctcagaccc 5280

ttttagtcag tgtggaaaat ctctagcagt agtagttcat gtcatcttat tattcagtat 5340

ttataacttg caaagaaatg aatatcagag agtgagagga acttgtttat tgcagcttat 5400

aatggttaca aataaagcaa tagcatcaca aatttcacaa ataaagcatt tttttcactg 5460

cattctagtt gtggtttgtc caaactcatc aatgtatctt atcatgtctg gctctagcta 5520

tcccgcccct aactccgccc atcccgcccc taactccgcc cagttccgcc cattctccgc 5580

cccatggctg actaattttt tttatttatg cagaggccga ggccgcctcg gcctctgagc 5640

tattccagaa gtagtgagga ggcttttttg gaggcctagg gacgtaccca attcgcccta 5700

tagtgagtcg tattacgcgc gctcactggc cgtcgtttta caacgtcgtg actgggaaaa 5760

ccctggcgtt acccaactta atcgccttgc agcacatccc cctttcgcca gctggcgtaa 5820

tagcgaagag gcccgcaccg atcgcccttc ccaacagttg cgcagcctga atggcgaatg 5880

ggacgcgccc tgtagcggcg cattaagcgc ggcgggtgtg gtggttacgc gcagcgtgac 5940

cgctacactt gccagcgccc tagcgcccgc tcctttcgct ttcttccctt cctttctcgc 6000

cacgttcgcc ggctttcccc gtcaagctct aaatcggggg ctccctttag ggttccgatt 6060

tagtgcttta cggcacctcg accccaaaaa acttgattag ggtgatggtt cacgtagtgg 6120

gccatcgccc tgatagacgg tttttcgccc tttgacgttg gagtccacgt tctttaatag 6180

tggactcttg ttccaaactg gaacaacact caaccctatc tcggtctatt cttttgattt 6240

ataagggatt ttgccgattt cggcctattg gttaaaaaat gagctgattt aacaaaaatt 6300

taacgcgaat tttaacaaaa tattaacgct tacaatttag gtggcacttt tcggggaaat 6360

gtgcgcggaa cccctatttg tttatttttc taaatacatt caaatatgta tccgctcatg 6420

agacaataac cctgataaat gcttcaataa tattgaaaaa ggaagagtat gagtattcaa 6480

catttccgtg tcgcccttat tccctttttt gcggcatttt gccttcctgt ttttgctcac 6540

ccagaaacgc tggtgaaagt aaaagatgct gaagatcagt tgggtgcacg agtgggttac 6600

atcgaactgg atctcaacag cggtaagatc cttgagagtt ttcgccccga agaacgtttt 6660

ccaatgatga gcacttttaa agttctgcta tgtggcgcgg tattatcccg tattgacgcc 6720

gggcaagagc aactcggtcg ccgcatacac tattctcaga atgacttggt tgagtactca 6780

ccagtcacag aaaagcatct tacggatggc atgacagtaa gagaattatg cagtgctgcc 6840

ataaccatga gtgataacac tgcggccaac ttacttctga caacgatcgg aggaccgaag 6900

gagctaaccg cttttttgca caacatgggg gatcatgtaa ctcgccttga tcgttgggaa 6960

ccggagctga atgaagccat accaaacgac gagcgtgaca ccacgatgcc tgtagcaatg 7020

gcaacaacgt tgcgcaaact attaactggc gaactactta ctctagcttc ccggcaacaa 7080

ttaatagact ggatggaggc ggataaagtt gcaggaccac ttctgcgctc ggcccttccg 7140

gctggctggt ttattgctga taaatctgga gccggtgagc gtgggtctcg cggtatcatt 7200

gcagcactgg ggccagatgg taagccctcc cgtatcgtag ttatctacac gacggggagt 7260

caggcaacta tggatgaacg aaatagacag atcgctgaga taggtgcctc actgattaag 7320

cattggtaac tgtcagacca agtttactca tatatacttt agattgattt aaaacttcat 7380

ttttaattta aaaggatcta ggtgaagatc ctttttgata atctcatgac caaaatccct 7440

taacgtgagt tttcgttcca ctgagcgtca gaccccgtag aaaagatcaa aggatcttct 7500

tgagatcctt tttttctgcg cgtaatctgc tgcttgcaaa caaaaaaacc accgctacca 7560

gcggtggttt gtttgccgga tcaagagcta ccaactcttt ttccgaaggt aactggcttc 7620

agcagagcgc agataccaaa tactgttctt ctagtgtagc cgtagttagg ccaccacttc 7680

aagaactctg tagcaccgcc tacatacctc gctctgctaa tcctgttacc agtggctgct 7740

gccagtggcg ataagtcgtg tcttaccggg ttggactcaa gacgatagtt accggataag 7800

gcgcagcggt cgggctgaac ggggggttcg tgcacacagc ccagcttgga gcgaacgacc 7860

tacaccgaac tgagatacct acagcgtgag ctatgagaaa gcgccacgct tcccgaaggg 7920

agaaaggcgg acaggtatcc ggtaagcggc agggtcggaa caggagagcg cacgagggag 7980

cttccagggg gaaacgcctg gtatctttat agtcctgtcg ggtttcgcca cctctgactt 8040

gagcgtcgat ttttgtgatg ctcgtcaggg gggcggagcc tatggaaaaa cgccagcaac 8100

gcggcctttt tacggttcct ggccttttgc tggccttttg ctcacatgtt ctttcctgcg 8160

ttatcccctg attctgtgga taaccgtatt accgcctttg agtgagctga taccgctcgc 8220

cgcagccgaa cgaccgagcg cagcgagtca gtgagcgagg aagcggaaga gcgcccaata 8280

cgcaaaccgc ctctccccgc gcgttggccg attcattaat gcagctggca cgacaggttt 8340

cccgactgga aagcgggcag tgagcgcaac gcaattaatg tgagttagct cactcattag 8400

gcaccccagg ctttacactt tatgcttccg gctcgtatgt tgtgtggaat tgtgagcgga 8460

taacaatttc acacaggaaa cagctatgac catgattacg ccaagcgcgc aattaaccct 8520

cactaaaggg aacaaaagct ggagctgcaa gctt 8554

<210> 104
<211> 84
<212> DNA
<213> Artificial Sequence

<220>
<223> Synthetic sequence

<400> 104
atggaccccg tggtggtgct gggcctgtgc ctgagctgcc tgctgctgct gagcctgtgg 60

aagcagagcc acggcggcgg caag 84

<210> 105
<211> 27
<212> PRT
<213> Artificial Sequence

<220>
<223> Synthetic sequence

<400> 105

Met Asp Pro Val Val Val Leu Gly Leu Cys Leu Ser Cys Leu Leu Leu
1               5                   10                  15

Leu Ser Leu Trp Gln Ser His Gly Gly Gly Lys
            20                  25

<210> 106
<211> 24
<212> DNA
<213> Artificial Sequence

<220>
<223> Synthetic sequence

<400> 106

atgggctgct gcttcagcaa gacc                                              24

<210>  107
<211>  8
<212>  PRT
<213>  Artificial Sequence

<220>
<223>  Synthetic sequence

<400>  107

Met Gly Cys Cys Phe Ser Lys Thr
1               5

<210>  108
<211>  36
<212>  DNA
<213>  Artificial Sequence

<220>
<223>  Synthetic sequence

<400>  108
gaggacaggg ccggcgccgg catcatcgag accccc                                  36

<210>  109
<211>  36
<212>  DNA
<213>  Artificial Sequence

<220>
<223>  Synthetic sequence

<400>  109
gccaccaggg ccggctgcgc ccccagctac agggtg                                  36

<210>  110
<211>  36
<212>  DNA
<213>  Artificial Sequence

<220>
<223>  Synthetic sequence

<400>  110
ctggacaggg ccggcggcta catcttcagc agcgac                                  36

<210>  111
<211>  27
<212>  DNA
<213>  Artificial Sequence

<220>
<223>  Synthetic sequence

<400>  111
atcaccactc ttgggaaatt tggacaa                                            27

<210>  112
<211>  39
<212>  DNA
<213>  Artificial Sequence

<220>
<223>  Synthetic sequence

<400>  112
agctacttcg ccagcgagca gggcgagatc cagtgggtg                              39


<210>  113
<211>  36
<212>  DNA
<213>  Artificial Sequence

<220>
<223>  Synthetic sequence

<400>  113
agggagctga acggcggcgc ctacaccagg tacgtg                                 36


<210>  114
<211>  36
<212>  DNA
<213>  Artificial Sequence

<220>
<223>  Synthetic sequence

<400>  114
ttcaccctga agggcggcgc ccccaccaag gtgacc                                 36


<210>  115
<211>  36
<212>  DNA
<213>  Artificial Sequence

<220>
<223>  Synthetic sequence

<400>  115
atcgccctga agggcggcaa gatcgtgaac aactgg                                 36


<210>  116
<211>  36
<212>  DNA
<213>  Artificial Sequence

<220>
<223>  Synthetic sequence

<400>  116
accagcgccg tgctgcagag cggcttcagg aagatg                                 36


<210>  117

<211>  36
<212>  DNA
<213>  Artificial Sequence

<220>
<223>  Synthetic sequence

<400>  117
agcggcgtga ccttccagag cgccgtgaag aggacc                                36


<210>  118
<211>  36
<212>  DNA
<213>  Artificial Sequence

<220>
<223>  Synthetic sequence

<400>  118
aaggtggcca ccgtgcagag caagatgagc gacgtg                                36


<210>  119
<211>  36
<212>  DNA
<213>  Artificial Sequence

<220>
<223>  Synthetic sequence

<400>  119
agcgccgtga agctgcagaa caacgagctg agcccc                                36


<210>  120
<211>  36
<212>  DNA
<213>  Artificial Sequence

<220>
<223>  Synthetic sequence

<400>  120
gccaccgtga ggctgcaggc cggcaacgcc accgag                                36


<210>  121
<211>  36
<212>  DNA
<213>  Artificial Sequence

<220>
<223>  Synthetic sequence

<400>  121
agggagccca tgctgcagag cgccgacgcc cagagc                                36


<210>  122
<211>  36
<212>  DNA
<213>  Artificial Sequence

<220>
<223>  Synthetic sequence

<400>  122
aaggagagga agaggagggg cgccgacacc agcatc                                36


<210>  123
<211>  36
<212>  DNA
<213>  Artificial Sequence

<220>
<223>  Synthetic sequence

<400>  123
accaggagcg gcaagaggag ctggcccccc agcgag                                36


<210>  124
<211>  36
<212>  DNA
<213>  Artificial Sequence

<220>
<223>  Synthetic sequence

<400>  124
gagcccgaga agcagaggag cccccaggac aaccag                                36


<210>  125
<211>  36
<212>  DNA
<213>  Artificial Sequence

<220>
<223>  Synthetic sequence

<400>  125
ggcctggtga agaggagggg cggcggcacc ggcgag                                36


<210>  126
<211>  9
<212>  PRT
<213>  Artificial Sequence

<220>
<223>  Synthetic sequence

<400>  126

Ile Thr Thr Leu Gly Lys Phe Gly Gln
1               5


<210>  127
<211>  8
<212>  PRT
<213>  Artificial Sequence

<220>
<223>  Synthetic sequence

<400>  127

Glu Ala Leu Phe Gln Gly Pro Lys
1               5


<210>  128
<211>  13
<212>  PRT
<213>  Artificial Sequence

<220>
<223>  Synthetic sequence

<400>  128

Ser Tyr Phe Ala Ser Glu Gln Gly Glu Ile Gln Trp Val
1               5                   10


<210>  129
<211>  8
<212>  PRT
<213>  Artificial Sequence

<220>
<223>  Synthetic sequence

<400>  129

Arg Ala Gly Ala Tyr Ile Phe Ser
1               5


<210>  130
<211>  12
<212>  PRT
<213>  Artificial Sequence

<220>
<223>  Synthetic sequence

<400>  130

Arg Glu Leu Asn Gly Gly Ala Tyr Thr Arg Tyr Val
1               5                   10


<210>  131
<211>  12
<212>  PRT
<213>  Artificial Sequence

<220>
<223>  Synthetic sequence

<400>  131

Phe Thr Leu Lys Gly Gly Ala Pro Thr Lys Val Thr
1               5                   10

<210>  132
<211>  12
<212>  PRT
<213>  Artificial Sequence

<220>
<223>  Synthetic sequence

<400>  132

Ile Ala Leu Lys Gly Gly Lys Ile Val Asn Asn Trp
1               5                   10


<210>  133
<211>  12
<212>  PRT
<213>  Artificial Sequence

<220>
<223>  Synthetic sequence

<400>  133

Thr Ser Ala Val Leu Gln Ser Gly Phe Arg Lys Met
1               5                   10


<210>  134
<211>  12
<212>  PRT
<213>  Artificial Sequence

<220>
<223>  Synthetic sequence

<400>  134

Lys Val Ala Thr Val Gln Ser Lys Met Ser Asp Val
1               5                   10


<210>  135
<211>  12
<212>  PRT
<213>  Artificial Sequence

<220>
<223>  Synthetic sequence

<400>  135

Ser Ala Val Lys Leu Gln Asn Asn Glu Leu Ser Pro
1               5                   10


<210>  136
<211>  12
<212>  PRT
<213>  Artificial Sequence

<220>
<223>  Synthetic sequence

<400>  136

Ala Thr Val Arg Leu Gln Ala Gly Asn Ala Thr Glu
1               5                   10


<210>  137
<211>  12
<212>  PRT
<213>  Artificial Sequence

<220>
<223>  Synthetic sequence

<400>  137

Arg Glu Pro Met Leu Gln Ser Ala Asp Ala Gln Ser
1               5                   10


<210>  138
<211>  3
<212>  PRT
<213>  Artificial Sequence

<220>
<223>  Synthetic sequence


<400>  138

Ser Gly Val Thr Phe Gln Ser Ala Val Lys Arg Thr
1               5                   10


<210>  139
<211>  11
<212>  PRT
<213>  Artificial Sequence

<220>
<223>  Synthetic sequence

<400>  139

Ser Gly Val Thr Phe Gln Gly Lys Phe Lys Lys
1               5                   10


<210>  140
<211>  8
<212>  PRT
<213>  Artificial Sequence

<220>
<223>  Synthetic sequence

<400>  140

Tyr Ala Lys Arg Gly Gly Val Phe

1               5


<210>  141
<211>  12
<212>  PRT
<213>  Artificial Sequence

<220>
<223>  Synthetic sequence

<400>  141

Lys Glu Arg Lys Arg Arg Gly Ala Asp Thr Ser Ile
1               5                   10


<210>  142
<211>  12
<212>  PRT
<213>  Artificial Sequence

<220>
<223>  Synthetic sequence

<400>  142

Thr Arg Ser Gly Lys Arg Ser Trp Pro Pro Ser Glu
1               5                   10


<210>  143
<211>  12
<212>  PRT
<213>  Artificial Sequence

<220>
<223>  Synthetic sequence

<400>  143

Glu Pro Glu Lys Gln Arg Ser Pro Gln Asp Asn Gln
1               5                   10


<210>  144
<211>  12
<212>  PRT
<213>  Artificial Sequence

<220>
<223>  Synthetic sequence

<400>  144

Gly Leu Val Lys Arg Arg Gly Gly Gly Thr Gly Glu
1               5                   10


<210>  145
<211>  1041
<212>  DNA
<213>  Artificial Sequence

<220>
<223>  Synthetic sequence

<400>  145
atggacctgc ctgacgacca ctacctgtcc acccagacca tcctgtccaa ggacctgaac      60

tccggactca gatctggcag cggtctcgag atggaagtta gcgctctgga aaaagaagtg     120

tctgcactcg agaaagaagt aagtgccctt gagaaggagg tgtccgcact cgagaaggag     180

gtcagcgccc tggaaaagga aaagcgagac catatggttt tgcttgagta tgttacagcg     240

gctggcatta ccgatgcatc aaaggtgtcc gccctgaagg aaaaagtaag cgcactgaaa     300

gaaaaggtga gcgcgctgaa ggagaaagtg agcgccctga aagagaaagt ctctgccctt     360

aaggaggata tcgagggcag aggaagtctg ctaacatgcg gtgacgtcga ggagaatcct     420

ggcccaaagc ttgccaccat gcgcaaaggc gaagaactgt ttaccggcat tgtgccgatt     480

ctggtggaac tggatggcga tgtgaacggc cataaatttt ttgtgcgcgg cgaaggcgaa     540

ggcgatgcga ccattggcaa actgagcctg aaatttattt gcaccaccgg caaactgccg     600

gtgccgtggc cgaccctggt gaccaccctg acctatggcg tgcagtgctt tagccgctat     660

ccggatcata tgaaacgcca tgattttttt aaaagcgcga tgccggaagg ctatgtgcag     720

gaacgcacca tttattttaa agatgatggc acctataaaa cccgcgcgga agtgaaattt     780

gaaggcgata ccctggtgaa ccgcattgaa ctgaaaggca ttgattttaa agaagatggc     840

aacattctgg gccataaact ggaatataac tttaacagcc ataaagtgta tattaccgcg     900

gataaacaga acaacggcat taaagcgaac tttaccattc gccataacgt ggaagatggc     960

agcgtgcagc tggcggatca ttatcagcag aacaccccga ttggcgatgg cccggttctt    1020

cttcctggcg gccgctctag a                                              1041


<210>  146
<211>  707
<212>  DNA
<213>  Artificial Sequence

<220>
<223>  Synthetic sequence

<400>  146
tgagcaaggg cgaggaggat aacatggcca tcatcaagga gttcatgcgc ttcaaggtgc      60

acatggaggg ctccgtgaac ggccacgagt tcgagatcga gggcgagggc gagggccgcc     120

cctacgaggg cacccagacc gccaagctga aggtgaccaa gggtggcccc ctgcccttcg     180

cctgggacat cctgtcccct cagttcatgt acggctccaa ggcctacgtg aagcaccccg     240

ccgacatccc cgactacttg aagctgtcct tccccgaggg cttcaagtgg gagcgcgtga     300

tgaacttcga ggacggcggc gtggtgaccg tgacccagga ctcctccctg caggacggcg     360

agttcatcta caaggtgaag ctgcgcggca ccaacttccc ctccgacggc cccgtaatgc 420

agaagaagac catgggctgg gaggcctcct ccgagcggat gtaccccgag gacggcgccc 480

tgaagggcga gatcaagcag aggctgaagc tgaaggacgg cggccactac gacgctgagg 540

tcaagaccac ctacaaggcc aagaagcccg tgcagctgcc cggcgcctac aacgtcaaca 600

tcaagttgga catcacctcc cacaacgagg actacaccat cgtggaacag tacgaacgcg 660

ccgagggccg ccactccacc ggcggcatgg acgagctgta caagtaa 707

<210> 147
<211> 54
<212> DNA
<213> Artificial Sequence

<220>
<223> Synthetic sequence

<400> 147
gagggcagag gaagtctgct aacatgcggt gacgtcgagg agaatcctgg ccca 54

## Description

The invention relates to novel means and processes for the detection of a virus in a biological sample comprising cells infected by the virus. In particular, the invention relates to a reporter protein designed for specific activation by viral components, in particular by viral proteins, such as viral protease, suitable for the detection of virus associated with such viral components, in a biological sample comprising cells sensitive to virus infection. Or rendered sensitive to virus infection. The invention hence relates to an *in vitro* method of detecting a virus or a virus infection in a biological sample, in particular in a sample that is regarded as intact, i.e., that does not require treatment to get access to the virus or the virus components, including to the viral genome.

Currently the detection of viruses relies on either visual signs of infection, such as cytopathic effects (CPE) when a virus infection results in cell death towards the end of the infection cycle, or it relies on methods that do not allow for conditions that keep the sample intact, referred to as live conditions. Such methods include, but are not limited to, plaque assays, TCID50s, PCR-based methods or sequencing methods.

With a view to design detection means based on fluorescence of a reporter protein, wherein the detection means would be suitable to enable detection of any type of viruses independently of their replication pathway in host cells and without interfering with the expression of viral components and integrity of the viral genome, the inventors have considered providing altered fluorescent proteins that would be activated in a biological sample in the presence of the virus to be detected or its viral proteins. In a preferred aspect the invention makes use of the indirect detection of the presence of proteins involved in achieving proper replication and assembly of viral particles, or mediating particle disassembly upon virus entry into a newly infected cell, especially makes use of detection of viral proteases in the assayed biological sample. Besides their biological function in the assembly and disassembly of virus particles, proteases additionally give rise to proteolytic cleavage in cells infected by the virus.

Accordingly the invention relies on genetically modified fluorescent proteins suitable for use as reporter proteins for infection of a sample by a determined virus, wherein the modification in the protein enables the fluorescent protein to become a sensor for a determined viral protease, especially of a protease of a virus infecting human or animals (human virus or animal virus). Such fluorescent proteins have been prepared for the purpose of the invention by modifying known split fluorescent proteins. In particular the genetically modified fluorescent protein suitable for use as reporter protein for infection of a sample by a determined virus is engineered in such a way that assaying the viral infection of a biological sample does not require prior purification of the viral protease interacting with the genetically modified fluorescent reporter protein. Otherwise stated the genetically modified fluorescent protein suitable for use as reporter protein for infection by a determined virus advantageously enables to carry out the detection when viral proteins other than the sought protease are present in the detected sample.

Systems derived from Spilt Fluorescent Protein (FP) are built according to the invention by engineering the fluorescent protein in order to confer to the fluorescent protein a conditional reporter activity that is based on occurrence of determined specific cleavage events in the engineered fluorescent protein that prove necessary to allow the reconstitution of a functional fluorescent FP. According to the invention, cleavage events in the sequence of the engineered FP are elicited by the internalization or the expression in a host cell expressing or transformed with the engineered FP system, of a protein of a pathogen (such as a viral protein, a bacterial protein or a parasitic protein), especially a protease, more particularly a viral protease. In a particular embodiment a pathogen protease such as a viral protease of a human or animal virus that specifically targets the cleavage site engineered in the FP system is present in the hot cell of the virus expressing this protease and activates the FP system to allow it to fluoresce. In another embodiment, the engineered FP system is expressed in a host cell that is contacted with a sample suspected of containing the virus. Advantageously the invention enables to detect virus infection in a sample obtained from a subject without requiring purification of the virus protease for contacting it with the engineered FP system and assessing infection of the sample.

According to a particular aspect, the invention relates to a process for the detection of an infection, especially a viral infection in a biological sample previously taken from a human subject or an animal, wherein the process comprises the step of transforming cells of the sample with a FP system comprising a fluorescent reporter protein that would be able to become fluorescent in the cell when its protease cleavage site is targeted and cleaved by a determined viral protease present in the cell and detecting whether fluorescence of the reporter protein is present.

According to another aspect, the invention relates to a process for the detection of an infection, especially a viral infection in a biological sample previously taken from a human subject or an animal, wherein the process comprises the step of providing cells expressing a FP system comprising a fluorescent reporter protein that would be able to become fluorescent in the cell when its protease cleavage site is targeted and cleaved by a determined viral protease present in an assayed biological sample contacted with said cells and detecting whether fluorescence of the reporter protein is present.

Applied to the detection of virus infection with the process of the invention may be efficient as it would detect virus presence a few hours after infection, in a specific and sensitive manner and would not require treating or denaturing the cell sample to isolate the virus material or its proteins.

As an example of the FP system suitable to carry out the invention an inactive form of split GFP, flip-GFP, has been chosen to illustrate the preparation of an engineered novel flip-GFP expressed in a host cell where it exhibits fluorescence under conditional elicitation by viral proteases. Flip-GFP according to the invention is accordingly an illustration of a particular protease sensor wherein the protease originates from a pathogen, in particular a virus.

GFP (also designated split-GFP) was cloned from *Aequorea Victoria* and has been shown to comprise 238 amino acids (with a cut-off site at position 214/215) that form a barrel structure with 11 beta strands (β-strands) with a central alpha helix (α-helix). The sequence of the GFP has been published (Tsien R.Y. Annu Rev. Biochem, 1998, 67, 509-44) and is available under accession number Q6YGZ20 (uniprot). GFP can be split into three parts. One part contains beta-strands 1 to 9 and the alpha helix (designated β1-9 or GFP1-9 - amino acids 1-193), a second part contains the 10^{th} β-strand (β10 - amino acids 194-212) and the third part contains the 11^{th} β-strand (β11 - amino acids 213-233). β1-9 contains three amino acids (Ser65, Tyr66, Gly67) that undergo cyclisation, dehydration and oxidation during maturation to form the chromophore whereas β11 contains the highly conserved amino acid residue Glu222 that catalyzes chromophore maturation (Sniegowski, J.A. J.Biol. Chem 2005, 280 (28), 26248). The activation of the fluorescence is elicited when β10 and β11 are linked together or are in close proximity and then bind to β1-9 that leads to the development of fluorescence within several tens of minutes (Cabantous, S. et al. Sci. Rep. 2013, 3, 2854). In the native structure of GFP, β10-11 forms an anti-parallel beta-strands which fits well within the rest of the structure, β1-9. The structure of the GFP was flipped in order to achieve parallel β10-11 structure so that it does not spontaneously fit β1-9 but rather required a signal that was chosen to be provided by protease cleavage at a specific protease cleavage site inserted into the sequence of β10-11: cleavage allowed flipping back (or reverting orientation) of β11 strand in an anti-parallel structure with β1, thereby enabling self-assembly with β1-9 and increase in fluorescence. The thus engineered reporter was named Flip-GFP and considered an inactive form of split-GFP that needed an external signal to become activated. It was used for imaging spatiotemporal dynamics of apoptosis in Zebrafish and in *Drosophila* (Zhang Q. et al J. Am. Chem. Soc., 2019, 141, 4526-4530)*.* avGFP has a maximum excitation peak at 396-398nm (corresponding to the neutral state of Tyr66 in the chromophore) and a lower excitation peak at 476-478nm (corresponding to the deprotonated anionic state of Tyr66 and the emission peak is at 510nm (Pedelacq J.D. and Cabantous S. Int. J. Mol. Sci. 2019, 20, 3479).
It has been observed in the art that GFP fused to other proteins in order to report on their activity may exhibit drawbacks such as folding defects that impact its fluorescence and reporter capability. Accordingly improved GFP such as superfolder GFP variants obtained by mutation of specific positions have been developed the folding of which is not affected by fusion to poorly folded proteins (Pedelacq J.D. et al Nature Biotechnology 2006, 24,1, 79-88). Such improved variants of GFP may be used in the invention.

In a first aspect, the invention relates to a nucleic acid construct which comprises (a) a recombinant transgene that encodes a recombinant inactive form of a fluorescent reporter protein, and a cleavage site for a viral protease, and (b) a nucleic acid coding for a detectable expression control protein, wherein the nucleic acid sequences of (a) and (b) are operably assembled in said operon under the control of a single promoter and optionally of additional control sequence(s) for transcription and/or translation and wherein the nucleic acid sequences of (a) and (b) are optionally separated by the sequence of a polyprotein separating site such as the sequence of the separating 2A-peptide originating from Thosea asigna virus capsid.

In a particular embodiment, the invention relates to a nucleic acid construct which comprises an operon wherein the operon comprises (a) a recombinant transgene that encodes a recombinant inactive form of a fluorescent reporter protein, and a cleavage site for a viral protease, and (b) a nucleic acid coding for a detectable expression control protein, wherein the nucleic acid sequences of (a) and (b) are operably assembled in said operon under the control of a single promoter and optionally of additional control sequence(s) for transcription and/or translation and wherein the nucleic acid sequences of (a) and (b) are optionally separated by the sequence of a polyprotein separating site such as the sequence of the separating 2A-peptide originating from Thosea asigna virus capsid.

A nucleic acid construct of the invention is in particular a DNA construct, especially a double-stranded DNA construct. It may in particular be a cDNA molecule.

The expression *"fluorescent protein"* (FP) in particular a *"fluorescent reporter protein"* or the likes according to the invention means a protein that is able to fluoresce in a cell when expressed in proper condition that enable maturation of its chromophore (also designated fluorophore). In the context of the invention, a first category of fluorescent proteins encompasses a fluorescent reporter protein that becomes active to fluoresce when it assembles as an active form of the protein. "*Assembly*" (assembled) means that the structural domains of the protein provide for a structure that reproduces the wild-type or the native conformation of the protein that allows maturation of the chromophore. Assembly may require that multiple structural domains in the proteins be correctly assembled. A second category of fluorescent proteins for use according to the invention encompasses expression control proteins that fluoresce as soon as the nucleic acid construct of the invention is expressed. Such fluorescent protein for control of expression are well known from the person skilled in the art.

The expression *"active fluorescent reporter protein"* is used interchangeably with the terms *"fluorescent reporter protein"* and designates the fluorescent protein that has adopted a functional conformation after specific site cleavage of the inactive fluorescent protein following contact or interaction with a suitable protease recognizing said specific site, according to the invention.

The expression *"inactive fluorescent reporter protein"* relates to the fluorescent reporter protein the amino sequence of which has been modified such that (i) its structure domains cannot assemble as in the wild type or the native form of the fluorescent protein to allow maturation of its chromophore and that (ii) it contains an amino acid sequence of a cleavage site for a protease in accordance with the disclosure provided herein. As a consequence of the presence of this cleavage site, upon cleavages the inactive fluorescent protein reconstitutes its wild type or native structure and fluoresce thereby becoming active or functional.

Accordingly, the invention relates to a particular nucleic acid construct wherein the recombinant transgene encoding the inactive form of the fluorescent reporter protein comprises a nucleotide sequence of an altered form of the Open Reading Frame that encodes the active form of the fluorescent reporter protein and wherein said alteration in the ORF comprises switching position in the ORF of at least one nucleotide sequence encoding specific structure domains of the active form of the fluorescent protein to prevent assembly of the expressed structure domains as a functional protein enabling maturation of the chromophore and wherein the nucleotide sequence encoding the inactive form of the fluorescent reporter protein additionally comprises a nucleotide sequence encoding a cleavage site for a determined protease.

The expression *"host cell"* refers to a cell that is present in the biological sample either naturally or after introduction by technical available means in order to enable suitable conditions for the expression or the display of the inactive and the active forms of the fluorescent protein. In a particular embodiment, the host cell is a cell that can be infected by the pathogen targeted the cleavage site in the inactive fluorescent protein. Especially, the host cell is a cell that can be infected by a human or an animal virus. In a particular embodiment, the host cell may be a cell line transformed (especially transected or transduced) with the nucleic acid construct as disclosed herein.

The expression *"operably linked"* or "operatively/operably linked" "operatively/operably assembled" or "operatively/operably cloned", refers to the functional cloning, or insertion, of polynucleotides within the nucleic acid construct of the invention. In a particular embodiment the nucleic acid sequences encoding the flip-GFP switched domains (designated flip-GFP10-11 in the figures) of the inactive form of the FP are cloned in a different open reading frame from the open reading frame of the sequence encoding the cleavage site for the viral protease. The sequences encoding the other polypeptides of the transgene, including the separating peptide and the expression control protein are cloned in the same reading frame as the reading frame of the nucleic acid sequences encoding the flip-GFP switched domains (designated flip-GFP10-11 in the figures) of the inactive form of the FP. The polynucleotides are operably linked when they can be expressed from the nucleic acid construct, as recombinant polypeptides such as in the inactive form of the fluorescent reporter protein or as individualized polypeptides such as the expression control protein.

The expression of the encoded fluorescent reporter protein and the expression of the control protein may be under the control of the same promoter that may be any known promoter including CMV promoter as exemplified herein. In a particular embodiment, the promoter of the transgene is active in cells selected from the group of prokaryotic cells, in particular in bacterial cells or in archaeal cells, and/or is active in eukaryotic cells, in particular in mammalian cells or in insect cells.

A fluorescent protein according to this embodiment is a protein that comprises multiple structural domains that need to be assembled and fold in native conformation to allow fluorescence to develop as a result of providing a mature chromophore that has acquires visible absorbance of light and fluorescence.

In a particular embodiment, the nucleic acid construct is such that the active fluorescent reporter protein is an active flip-GFP and the inactive fluorescent reporter protein is an inactive flip-GFP.

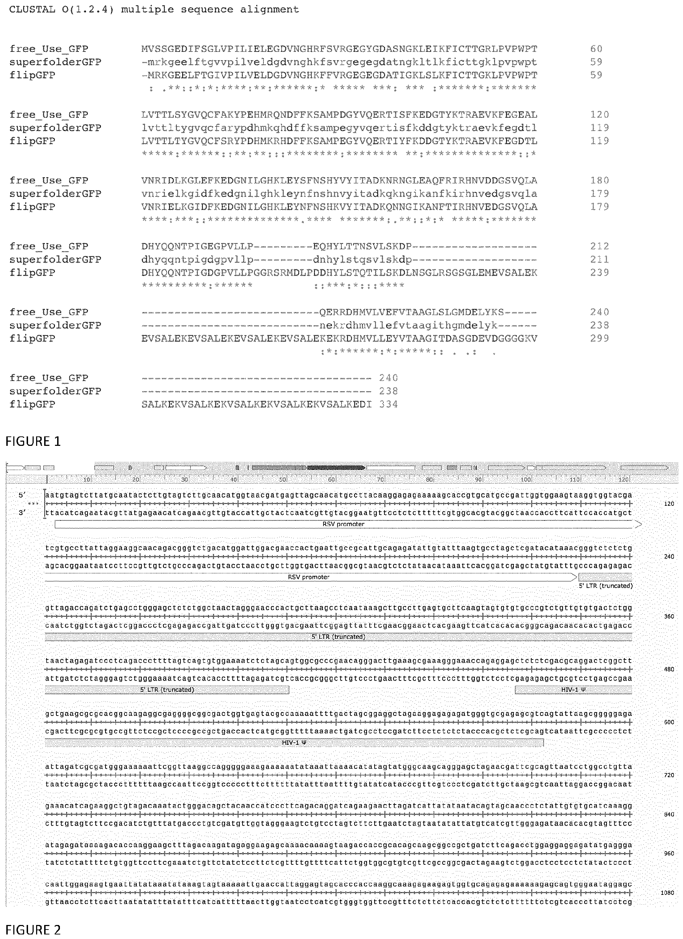

A particular embodiment of the fluorescent protein used in the invention is the GFP as disclosed above based on the protein originally identified in *Aeoquorea* jellyfish. Beyond the disclosed GFP having the polypeptide sequence disclosed by Tsien R.Y, various isoforms of the sequence of *Aeoquorea* GFP have been identified in the art. They may interchangeably be considered for the purpose of the novel flip-GFP construction according to the invention. besides GFP from other organisms have also been identified such as GFP originating from anthozoa *Renilla,* or GFP of other coelenterates such as *Obelia* or *Phialidium.* In addition, mutated GFP having improved properties when used as reporter protein have been made available in the art, including the proteins as disclosed in Figure 1. All these forms of GFP may be used to derive the novel flip-GFP construct for use in the invention.

*"switching position"* relative to the ORF of at least one structure domain of the fluorescent reporter protein to achieve an inactive form of the protein may involve permutation of the nucleotide sequence coding for at least two structure domains of the protein or permutation of sequences encoding contiguous groups of domains in the ORF coding for the protein, in the nucleic acid construct of the invention. This is illustrated by the nucleotide sequence of the flip-GFP where the nucleotide sequences encoding respectively the GFP1-9 and GFP10-11 domains are permutated in the nucleic acid construct of the invention.

In a further particular embodiment of a nucleic acid construct according to the invention the recombinant transgene comprises from 5'-end to 3'-end polynucleotides encoding the beta10 strand of GFP, a linker having from 3 to 15, in particular about 10, amino acid residues, the E5 domain of GFP, the beta11 strand of GFP, the cleavage site of the viral protease and the K5 domain of the GFP, the sequence of a polyprotein separating site such as the sequence of the separating 2A-peptide originating from Thosea asigna virus capsid, a polynucleotide encoding the beta1-9 strand of GFP wherein these polynucleotides together encode the inactive form of the recombinant GFP with the viral protease cleavage site.

Amino acid sequence of a 2A-peptide originating from Thosea asigna virus capsid may be the sequence of SEQ ID No.147.

In an embodiment of a nucleic acid construct according to the invention the transgene further comprises upstream from the sequence encoding the inactive fluorescent reporter protein in particular the inactive flip-GFP, a signal peptide, in particular a signal peptide for retention of the expressed polypeptides in the endoplasmic reticulum (ER retention signal) or a signal peptide for targeting the expressed polypeptides to the cell membrane (membrane targeting signal), especially a Cytochrome P450 ER retention signal of sequence MDPVVVLGLCLSCLLLLSLWQSHGGGK (SEQ ID No.105) or a membrane targeting signal of sequence MGCCFSKT (SEQ ID No.107).

As an illustration to carry out the invention, the polynucleotide encoding the signal peptide may contain or consist of the sequence of ATGGACCCCGTGGTGGTGCTGGGCCTGTGCCTGAGCTGCCTGCTGCTGCTGAGCCTGT GGAAGCAGAGCCACGGCGGCGGCAAG (SEQ ID No.104) encoding the Cytochrome P450 ER retention signal peptide or may contain or consist of the sequence of ATGGGCTGCTGCTTCAGCAAGACC (SEQ ID No.106) encoding the membrane targeting signal peptide.

Any alternative signal peptide known to the person skilled in the art and suitable to adress the expressed polypeptides to a specific cellular compartment, especially to the cell membrane or to the endoplasmic reticulum may be used to perform the invention. The sequence encoding a signal peptide in the nucleic acid construct of the invention enables expression of the polypeptides in a specific cell compartment and as a consequence enables their accessibility for the protease of the virus when contacted with the assayed sample.

As mentioned hereabove a nucleic acid construct according to the invention encodes an expression control protein.

The expression *"expression control protein"* defines a protein that may be detectable by any means in particular by fluorescence in conditions that would prevent detrimental, overlapping between fluorescence of the expression control protein and fluorescence of the fluorescent reporter protein. In particular, the fluorescence of expression control protein and of the FP may take place in different light ranges or may be of a different color. The expression control protein may be used to ascertain the expression of the fluorescent reporter protein in conditions where fluorescence of the sample may interfere with the expected fluorescence of the FP. Accordingly expression of the expression control protein may ensure that the detected fluorescence alleged to arise from the FP is not merely a background fluorescence.

The expression control protein may be a fluorescent protein with a fluorophore of a color that is different from the color of the fluorescent reporter protein. In particular the expression control protein may be an mCherry protein or mTurquoise protein or cyan fluorescent protein (ECFP), yellow fluorescent protein such as mVenus, in particular the nucleic acid construct contains the polynucleotide of SEQ ID No. 146 coding for mCherry.

In the nucleic acid construct of the invention, the sequence encoding the cleavage site in the fluorescent reporter protein should be inserted in the sequence encoding the inactive form of the fluorescent protein at a position of the sequence that would not hamper the maturation of the chromophore in the expressed activated protein said position being further chosen to enable restoration of the active conformation of the fluorescent protein after cleavage. In a particular embodiment, insertion site for the cleavage site sequence may be at the junction of structural domains. Illustration of the insertion position of the cleavage site is especially provided for the flip-GFP and thus gives guidance for the person skilled in the art to derive alternative embodiments.

The expression *"cleavage site"* refers to a peptide or polypeptide functional as a site to allow cleavage of its amino acid sequence when recognized by a specific enzyme, i.e. a pathogen protease, in particular a viral protease. In a particular embodiment the cleavage site is said to be *"specific"* when it is efficiently recognized and cleaved by a protease of a single virus or by a protease shared by members of a determined family or group of pathogens such as a class or a group of viruses. A cleavage site has a wild type sequence originating from a known pathogen, in particular a known virus of a group of viruses or has a modified sequence, such as a consensus sequence suitable to improve recognition and cleavage by the relevant protease of several pathogens in a determined group of pathogens, especially viruses. The cleavage site comprises amino acid residues surrounding the precise residues where the cleavage takes place in the target sequence for the protease. In a particular embodiment the cleavage site comprises more than 8 amino acid residues, in particular more than 10, especially 12 amino acid residues. In a particular embodiment the number of amino acid residues is similar in the N-terminal and C-terminal part of the sequence relative to the location of the residue where the cleavage takes place. In a particular embodiment the amino acid sequence of the cleavage site may be derived from the native sequence targeted by the selected protease in a viral strain. In another particular embodiment, variation in the amino acid residues relative to the native sequence may be performed.

In a particular embodiment of the nucleic acid construct, the cleavage site contained in the inactive fluorescent protein is recognized by proteases of a determined virus family or genus selected in the group of Alphaviruses, Coronaviruses, Enteroviruses, Retroviruses and Flaviviruses.

These families of viruses are of particular interest for the purpose of early detection of infection. Families of viruses such as these cited above or sub-families thereof may share features including similar sequences for cleavage site of proteases, such similarity being sufficient to allow cross-recognition by proteases of the various virus members of the family or the sub-family. Shared cleavage sites for proteases in a family or sub-family or even in a genus may allow the detection of viruses at the level of their family, sub-family or respectively genus and accordingly be in particular useful to obtain a primary indication of the cause of an infection in a subject especially when it can be obtained at an early stage of the infection.

The inventors have in particular selected sequences of peptides or polypeptides that bear the cleavage site for a protease active in viruses belonging to these families.

Accordingly in a second aspect, the invention concerns a polynucleotide suitable for the preparation of the nucleic acid constructs of the invention, wherein such polynucleotide comprises or consists in the sequence coding for an inactive fluorescent reporter protein wherein the sequence insert for the cleavage site for a protease is selected among cleavage site recognized by proteases of a determined virus family selected in the group of Alphaviruses, Coronaviruses, Enteroviruses, Retroviruses and Flaviviruses. Alternatively the polynucleotide encoding the cleavage site may be selected for its capability to be recognized by the protease of a specific virus or a specific virus strain.

According to a particular embodiment of the nucleic acid construct; the nucleic acid sequence for the protease cleavage site encodes an amino acid sequence selected from the group of ITTLGKFGQ (SEQ ID No.126) for the Enterovirus 2A protease, EALFQGPK (SEQ ID No.127) or SYFASEQGEIQWV (SEQ ID No.128) for the Enterovirus 3C protease, RAGAYIFS (SEQ ID No.129) for Alphaviruses, RELNGGAYTRYV (SEQ ID No.130), FTLKGGAPTKVT (SEQ ID No.131), IALKGGKIVNNW (SEQ ID No.132), TSAVLQSGFRKM (SEQ ID No.133), KVATVQSKMSDV (SEQ ID No.134), SAVKLQNNELSP (SEQ ID No.135), ATVRLQAGNATE (SEQ ID No.136), REPMLQSADAQS (SEQ ID No.137) and SGVTFQSAVKRT (SEQ ID No.138) for SARS-CoV-2 coronavirus, SGVTFQGKFKK (SEQ ID No.139) for SARS virus coronavirus, YAKRGGVF (SEQ ID No140) for Flaviviruses, and more particularly KERKRRGADTSI (SEQ ID No.141), TRSGKRSWPPSE (SEQ ID No. 142), EPEKQRSPQDNQ (SEQ ID No.143), GLVKRRGGGTGE (SEQ ID No.144) for ZIKA viruses.

The above cited peptide forming cleavage site for the SARS-CoV-2 coronavirus are specific for cleavage by a protease of the virus in particular by the main protease of the virus also designated M^{pro,}, nsp5 or 3CL^{pro} that requires the minimal LQ consensus sequence for cleavage (after the Q residue) or alternatively by the protease designated nsp3.

In another particular embodiment of the nucleic acid construct, the polynucleotide encoding the viral protease cleavage site consists of a polynucleotide selected from the group of : GAGGACAGGGCCGGCGCCGGCATCATCGAGACCCCC for CHKV-1 (SEQ ID No.108) or GCCACCAGGGCCGGCTGCGCCCCCAGCTACAGGGTG for CHKV-2 (SEQ ID No.109) or CTGGACAGGGCCGGCGGCTACATCTTCAGCAGCGAC for CHKV-3 (SEQ ID No.110) or,
ATCACCACTCTTGGGAAATTTGGACAA for EV71_2A (SEQ ID No.111) or AGCTACTTCGCCAGCGAGCAGGGCGAGATCCAGTGGGTG for EV71_3C (SEQ ID No.112) or,
AGGGAGCTGAACGGCGGCGCCTACACCAGGTACGTG for SARSCoV2_1 (SEQ ID No.113) or TTCACCCTGAAGGGCGGCGCCCCCACCAAGGTGACC for SARSCoV2_2 (SEQ ID No.114) or ATCGCCCTGAAGGGCGGCAAGATCGTGAACAACTGG for SARSCoV2_3 (SEQ ID No.115) or ACCAGCGCCGTGCTGCAGAGCGGCTTCAGGAAGATG for SARSCoV2_4 (SEQ ID No.116) or AGCGGCGTGACCTTCCAGAGCGCCGTGAAGAGGACC for SARSCoV2_5 (SEQ ID No.117) or AAGGTGGCCACCGTGCAGAGCAAGATGAGCGACGTG for SARSCoV2_6 (SEQ ID No.118) or AGCGCCGTGAAGCTGCAGAACAACGAGCTGAGCCCC for SARSCoV2_7 (SEQ ID No.119) or GCCACCGTGAGGCTGCAGGCCGGCAACGCCACCGAG for SARSCoV2_8 (SEQ ID No.120) or AGGGAGCCCATGCTGCAGAGCGCCGACGCCCAGAGC for SARSCoV2_9 (SEQ ID No.121) or, AAGGAGAGGAAGAGGAGGGGCGCCGACACCAGCATC for ZIKV_1 (SEQ ID No.122) or ACCAGGAGCGGCAAGAGGAGCTGGCCCCCCAGCGAG for ZIKV_2 (SEQ ID No.123) or GAGCCCGAGAAGCAGAGGAGCCCCCAGGACAACCAG for ZIKV_3 (SEQ ID No.124) or GGCCTGGTGAAGAGGAGGGGCGGCGGCACCGGCGAG for ZIKV_4 (SEQ ID No.125).

In a particular embodiment, the nucleic acid construct comprises a polynucleotide that encodes a flip-GFP of the sequence of SEQ ID No.145 recombined with one of these nucleotide sequences coding for one or more of the above cleavage sites. In a particular embodiment the sequence encoding the one or multiple cleavage sites for the viral protease is inserted at position 262 in said sequence.

In another embodiment, the novel flip-GFP is encoded by the sequence of SEQ ID No. 145 and the sequence of the cleavage site for the protease, in particular a sequence chosen among the illustrated sequences herein for viral cleavage sites is inserted at position 262 in said sequence.

According to a particular embodiment, the polynucleotide for insertion in the recombinant transgene to encode the recombinant inactive recombinant flip-GFP is selected from the group of SEQ ID No. 47 to SEQ ID No.100 respectively for flipGFP_CHKV_1, flipGFP_CHKV₋2, flipGFP_ER_CHKV_1, flipGFP_ER_CHKV_2, flipGFP_ER_CHKV_3, flipGFP_ER_EV71_2A, flipGFP_ER_EV71_3C, flipGFP_ER_SARSCoV2_1, flipGFP_ER_SARSCoV2_2, flipGFP_ER_SARSCoV2_3, flipGFP_ER_SARSCoV2 4, flipGFP_ER₋SARSCoV2_5, flipGFP_ER_SARSCoV2_6, flipGFP_ER_SARSCoV2_7, flipGFP_ER₋SARSCoV2_8, flipGFP_ER_SARSCoV2_9, flipGFP_ER_ZIKV_1, flipGFP_ER_ZIKV_2, flipGFP_ER_ZIKV_3, flipGFP_ER_ZIKV_4, flipGFP_EV71_2A, flipGFP_EV71_3C, flipGFP_Membrane_CHKV_1, flipGFP_Membrane_CHKV_2, flipGFP_Membrane_CHKV_3, flipGFP_Membrane_EV71_2A, flipGFP_Membrane-EV71_3C, flipGFP_Membrane_SARSCoV2_1, flipGFP_Membrane_SARSCoV2_2, flipGFP_Membrane_SARSCoV2_3, flipGFP_Membrane_SARSCoV2_4, flipGFP_Membrane_SARSCoV2_5, flipGFP_Membrane_SARSCoV2_6, flipGFP-Membrane_SARSCoV2_7, flipGFP_Membrane_SARSCoV2_8, flipGFP_Membrane_SARSCoV2_9, flipGFP_Membrane_ZIKV_1, flipGFP_Membrane_ZIKV_2, flipGFP_Membrane_ZIKV_3, flipGFP_Membrane_ZIKV_4, flipGFP_SARSCoV2_1, flipGFP_SARSCoV2_2, flipGFP _SARSCoV2_3, flipGFP_SARSCoV2_4, flipGFP_SARSCoV2_5, flipGFP _SARSCoV2_6, flipGFP_SARSCoV2_7, flipGFP_SARSCoV2_8, flipGFP _SARSCoV2_9, flipGFP_ZIKV_1, flipGFP _ZIKV_2, flipGFP_ZIKV_3, flipGFP_ZIKV_4.

The invention also relates to each of the above nucleic acid sequences encoding a recombinant inactive flip-GFP as such.

The invention also relates to a set of at least two nucleic acid constructs as defined herein, or of recombinant nucleic acids as defined herein or of polynucleotides as defined herein.

The invention also concerns a transformation vector which comprises a nucleic acid construct or a recombinant nucleic acids or a polynucleotide, in particular a vector which is a plasmid for transfection, especially a lentiviral vector plasmid.

In a particular embodiment, when the nucleic acid construct is a lentiviral vector plasmid, the polynucleotide sequences are operably linked within the cDNA encoding the genome of the lentivirus vector. Accordingly, in the context of the invention the nucleic acid sequence of the polynucleotide encoding a fluorescent reporter protein is fused within the sequence of the plasmid comprising the genome of the lentiviral vector in such a manner that the construct enables expression of infectious recombinant viral particles (in particular pseudotyped recombinant viral particles) of the lentiviral vector and enables such recombinant lentiviral particles to express the fluorescent protein in appropriate conditions. The nucleic acid construct accordingly comprises the nucleotide sequence necessary for replication of the recombinant vector genome and for the expression of the encoded proteins.

The expression *"lentiviral vector plasmid*" means the plasmid bearing the genome of the lentiviral vector. In particular the lentiviral vector is a HIV derived vector, especially a HIV-1 derived vector: in this case the lentiviral vector plasmid comprises the cDNA of the nucleic acid sequences of the HIV that are necessary to enable replication of the vector genome recombined with heterologous sequence(s) such as the transgene encoding the inactive fluorescent protein comprised therein. Lentiviral vectors are well known from the person skilled in the art and their preparation based on HIV-1 virus has been disclosed in the art including in Zennou V. et al (Cell, 2000, 101, 173-185), Iglesias M.C. et al (Mol. Ther, 2007, 15: 1203-1210)*.* A lentivector plasmid based on HIV-1 genome in particular contains the HIV-1 cis-active elements (LTR (long terminal repeat), encapsidation signal Ψ, RRE and advantageously DNA Flap cPPT-CTS) operably linked with the transgene encoding the fluorescent protein under the control of a selected promoter, especially non-HIV promoter. In order to produce a lentiviral vector (i.e. vector particles or recombinant virus particles) the vector plasmid is transfected in cells suitable for assembly of the vector particles together with one or several further plasmids expressing HIV-1 genes necessary for the genome encapsidation, in particular one or a plurality of plasmids collectively comprising HIV-1 genes gag, pol, tat and rev and furthermore with an envelope expression plasmid encoding an envelope glycoprotein, in particular an envelope glycoprotein that does not originated from HIV and that may be obtained from the Vesicular Stomatitis Virus (VSV), e.g. a VSV-G protein. Specific description of the preparation of lentiviral plasmid vectors and complementation vectors based on HIV is disclosed in the art, in particular in patent applications WO.

According to a particular embodiment, the vector is a lentiviral vector plasmid selected from the group of pLentiPuro_flipGFP_ER_3C (SEQ ID No.101), pLentiPuro_flipGFP_Membrane_3C (SEQ ID No.102), and pLentiPuro-flipGFP-3C (SEQ ID No.103).

In an alternative embodiment, the nucleotide sequence encoding the cleavage site of the enterovirus 3C in one of the above sequences of the vector is substituted for a nucleotide sequence encoding a cleavage site selected from the group of ITTLGKFGQ (SEQ ID No.126) for the Enterovirus 2A protease, EALFQGPK (SEQ ID No.127) or SYFASEQGEIQWV (SEQ ID No.128) for the Enterovirus 3C protease, RAGAYIFS (SEQ ID No.129) for Alphaviruses, RELNGGAYTRYV (SEQ ID No.130), FTLKGGAPTKVT (SEQ ID No.131), IALKGGKIVNNW (SEQ ID No.132), TSAVLQSGFRKM (SEQ ID No.133), KVATVQSKMSDV (SEQ ID No.134), SAVKLQNNELSP (SEQ ID No.135), ATVRLQAGNATE (SEQ ID No.136), REPMLQSADAQS (SEQ ID No.137) and SGVTFQSAVKRT (SEQ ID No.138) for SARS-CoV-2 coronavirus, SGVTFQGKFKK (SEQ ID No.139) for SARS virus coronavirus, YAKRGGVF (SEQ ID No140) for Flaviviruses, and more particularly KERKRRGADTSI (SEQ ID No.141), TRSGKRSWPPSE (SEQ ID No. 142), EPEKQRSPQDNQ (SEQ ID No.143), GLVKRRGGGTGE (SEQ ID No.144) for ZIKA viruses.

In an alternative embodiment, the the nucleotide sequence encoding the cleavage site of the enterovirus 3C in one of the above sequences of the vector is substituted for a nucleotide sequence selected from the group of GAGGACAGGGCCGGCGCCGGCATCATCGAGACCCCC for CHKV-1 (SEQ ID No.108) or GCCACCAGGGCCGGCTGCGCCCCCAGCTACAGGGTG for CHKV-2 (SEQ ID No.109) or CTGGACAGGGCCGGCGGCTACATCTTCAGCAGCGAC for CHKV-3 (SEQ ID No.110) or, ATCACCACTCTTGGGAAATTTGGACAA for EV71_2A (SEQ ID No.111) or AGCTACTTCGCCAGCGAGCAGGGCGAGATCCAGTGGGTG for EV71_3C (SEQ ID No.112) or,
AGGGAGCTGAACGGCGGCGCCTACACCAGGTACGTG for SARSCoV2_1 (SEQ ID No.113) or TTCACCCTGAAGGGCGGCGCCCCCACCAAGGTGACC for SARSCoV2_2 (SEQ ID No.114) or ATCGCCCTGAAGGGCGGCAAGATCGTGAACAACTGG for SARSCoV2_3 (SEQ ID No.115) or ACCAGCGCCGTGCTGCAGAGCGGCTTCAGGAAGATG for SARSCoV2_4 (SEQ ID No.116) or AGCGGCGTGACCTTCCAGAGCGCCGTGAAGAGGACC for SARSCoV2_5 (SEQ ID No.117) or AAGGTGGCCACCGTGCAGAGCAAGATGAGCGACGTG for SARSCoV2_6 (SEQ ID No.118) or AGCGCCGTGAAGCTGCAGAACAACGAGCTGAGCCCC for SARSCoV2_7 (SEQ ID No.119) or GCCACCGTGAGGCTGCAGGCCGGCAACGCCACCGAG for SARSCoV2_8 (SEQ ID No.120) or AGGGAGCCCATGCTGCAGAGCGCCGACGCCCAGAGC for SARSCoV2_9 (SEQ ID No.121) or,
AAGGAGAGGAAGAGGAGGGGCGCCGACACCAGCATC for ZIKV_1 (SEQ ID No.122) or ACCAGGAGCGGCAAGAGGAGCTGGCCCCCCAGCGAG for ZIKV_2 (SEQ ID No.123) or GAGCCCGAGAAGCAGAGGAGCCCCCAGGACAACCAG for ZIKV_3 (SEQ ID No.124) or GGCCTGGTGAAGAGGAGGGGCGGCGGCACCGGCGAG for ZIKV_4 (SEQ ID No.125) .

The vector is used for the transformation of host cells for the expression of the inactive form of the fluorescent reporter protein. When the vector is a lentiviral vector plasmid it is used for the preparation of recombinant lentiviral particles in a host cell having recourse to complementation plasmids bearing the nucleotide sequences for the expression of necessary structural proteins according to methods known in the art. The vector may be designed for transient expression of the nucleic acid construct of the invention of for stable expression of said nucleic acid construct. The lentiviral vector particles may in particular be advantageously used for the stable expression of the nucleic acid construct of the invention.

The invention also relates to a cell which is a prokaryotic or a eukaryotic cell or cell line transformed with the recombinant nucleic acid according to the invention or with a transformation vector. In a specific embodiment, the cell is selected for its sensibility to infection by a determined human virus targeting the cleavage site of the inactive fluorescent protein expressed in the cell, and said cell is optionally a stable cell line. A stable cell line may advantageously stably express the nucleic acid construct of the invention. It may be obtained after transduction with recombinant lentiviral vector particles encoding the nucleic acid construct of the invention wherein said construct provides the sequence coding for the recombinant inactive fluorescent reporter protein to the nucleus of the host cells and allow its insertion in its genome.

The invention also relates to recombinant viral vector particles, in particular HIV-1 vector particles which comprise as their genome a nucleic acid construct as defined herein. Such recombinant viral particles may be used for transduction of cells in a biological sample assayed for the detection of a virus wherein the targeted virus is one recognizing the cleavage site introduced in the inactive fluorescent reporter protein. Alternatively, the recombinant viral particles may be used for transduction of host cells that are used to assay the biological sample in which detection of viral infection is performed and wherein the targeted virus is one recognizing the cleavage site introduced in the inactive fluorescent reporter protein.

The invention is also directed to a recombinant fluorescent reporter protein which is encoded by a nucleic acid construct or a recombinant nucleic acid as disclosed herein.

A particular inactive recombinant fluorescent reporter protein according to the invention is characterized by its amino acid sequence which is the sequence encoded by any polynucleotide selected from the group of SEQ ID No. 47 to SEQ ID No. 100.

The invention also concerns a polynucleotide encoding a viral protease cleavage site which is selected from the group of GAGGACAGGGCCGGCGCCGGCATCATCGAGACCCCC for CHKV-1 (SEQ ID No.108) or GCCACCAGGGCCGGCTGCGCCCCCAGCTACAGGGTG for CHKV-2 (SEQ ID No.109) or CTGGACAGGGCCGGCGGCTACATCTTCAGCAGCGAC for CHKV-3 (SEQ ID No.110) or, ATCACCACTCTTGGGAAATTTGGACAA for EV71_2A (SEQ ID No.111) or AGCTACTTCGCCAGCGAGCAGGGCGAGATCCAGTGGGTG for EV71_3C (SEQ ID No.112) or,
AGGGAGCTGAACGGCGGCGCCTACACCAGGTACGTG for SARSCoV2_1 (SEQ ID No.113) or TTCACCCTGAAGGGCGGCGCCCCCACCAAGGTGACC for SARSCoV2_2 (SEQ ID No.114) or ATCGCCCTGAAGGGCGGCAAGATCGTGAACAACTGG for SARSCoV2_3 (SEQ ID No.115) or ACCAGCGCCGTGCTGCAGAGCGGCTTCAGGAAGATG for SARSCoV2_4 (SEQ ID No.116) or AGCGGCGTGACCTTCCAGAGCGCCGTGAAGAGGACC for SARSCoV2_5 (SEQ ID No.117) or AAGGTGGCCACCGTGCAGAGCAAGATGAGCGACGTG for SARSCoV2_6 (SEQ ID No.118) or AGCGCCGTGAAGCTGCAGAACAACGAGCTGAGCCCC for SARSCoV2_7 (SEQ ID No.119) or GCCACCGTGAGGCTGCAGGCCGGCAACGCCACCGAG for SARSCoV2_8 (SEQ ID No.120) or AGGGAGCCCATGCTGCAGAGCGCCGACGCCCAGAGC for SARSCoV2_9 (SEQ ID No.121) or, AAGGAGAGGAAGAGGAGGGGCGCCGACACCAGCATC for ZIKV_1 (SEQ ID No.122) or ACCAGGAGCGGCAAGAGGAGCTGGCCCCCCAGCGAG for ZIKV_2 (SEQ ID No.123) or GAGCCCGAGAAGCAGAGGAGCCCCCAGGACAACCAG for ZIKV_3 (SEQ ID No.124) or GGCCTGGTGAAGAGGAGGGGCGGCGGCACCGGCGAG for ZIKV_4 (SEQ ID No.125).

The invention also relates to a polypeptide which is a cleavage site for a virus protease and which is selected from the group of SEQ ID No. 126 to SEQ No.144.

The invention also contemplates the use of a nucleic acid construct or of a vector or a protein disclosed herein, for *in vitro* detection of a viral infection in a biological sample of a human or animal subject, wherein the detection targets a virus expressing a protease recognizing the cleavage site inserted in the recombinant inactive fluorescent reporter protein.
The invention also relates to an *in vitro* method of detecting or monitoring a pathogen infection, in particular a virus infection, in a biological sample previously obtained from a human or an animal subject, which comprises the steps of:
a. Providing cells according to the invention that express either transiently or stably an inactive fluorescent reporter protein comprising a cleavage site for a protease of the virus to be detected,
b. Contacting said cells with the assayed biological sample in condition that enable the virus when present in the sample, to infect the cells and its protease to cleave the protease cleavage site,
c. Allowing fluorescence to increase in the biological sample following activation of the inactive fluorescent reporter protein by cleavage of the viral protease cleavage site in step b. and measuring said fluorescence of the reporter protein,
d. Optionally comparing the fluorescence level of the active fluorescent reporter protein to a standard or fluorescent control protein expressed in the cells and optionally concluding on virus infectious activity in the subject and/or quantitating the virus.

Alternatively an *in vitro* method of detecting or monitoring a pathogen infection, in particular a virus infection, in a biological sample previously obtained from a human or an animal subject, which comprises the steps of:
a. Providing an inactive fluorescent protein as a reporter protein expressed from the nucleic acid construct of any one of the disclosed embodiments, together with a control protein expressed from the same nucleic acid construct wherein the inactive fluorescent reporter protein comprises a cleavage site for a protease of the virus to be detected,
b. Contacting said inactive fluorescent reporter protein with the assayed biological sample in condition that enable the virus protease to target and to cleave the protease cleavage site in the inactive fluorescent reporter protein,
c. Allowing fluorescence to increase in the biological sample following activation of the inactive fluorescent reporter protein by cleavage in step b. and measuring said fluorescence,
d. Optionally comparing the fluorescence level to a standard or to the fluorescence of the control protein and optionally concluding on the virus infectious activity in the subject and/or quantitating the virus.

These methods may be used for the diagnosis of a human subject or an animal for an infection by a determined pathogen, especially a determined virus, including at an early stage of infection when the pathogen load is still low and its proteases are however expressed sufficiently to enable the fluorescence of the assayed sample to increase over the background fluorescence of the sample, as a result of the method carried out.

The methods may also be used for monitoring the infection by a pathogen, in particular a virus in a subject that is undergoing therapy for this infection.

A particular embodiment of the method of detecting or monitoring a virus infection in a biological sample previously obtained from a human or an animal subject, is targets infection by a virus selected in the group of Alphaviruses, Coronaviruses, Enteroviruses, Retroviruses and Flaviviruses, in particular a virus which is a coronavirus, especially is SARS-CoV-2 (or SARS-2) responsible for Covid-19.

Furthermore, the invention concerns a laboratory animal for experimental or clinical observation of the response to a pathogen infection, in particular a virus infection, wherein the animal has been transformed to enable its genome to express a nucleic acid construct or a recombinant nucleic acids, either transiently or stably, the animal being in particular a rodent, an insect or a non-human mammal.

The invention will be further described in the examples and figures that follow that illustrate particular non limitative embodiments.

### Legend of the Figures

Figure 1: sequence alignment for GFP

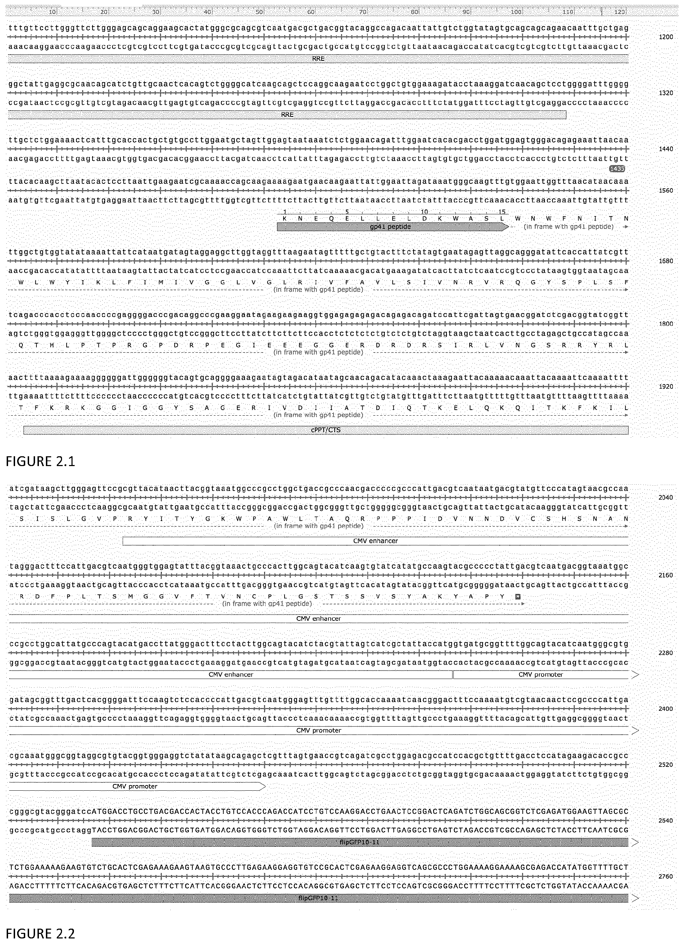

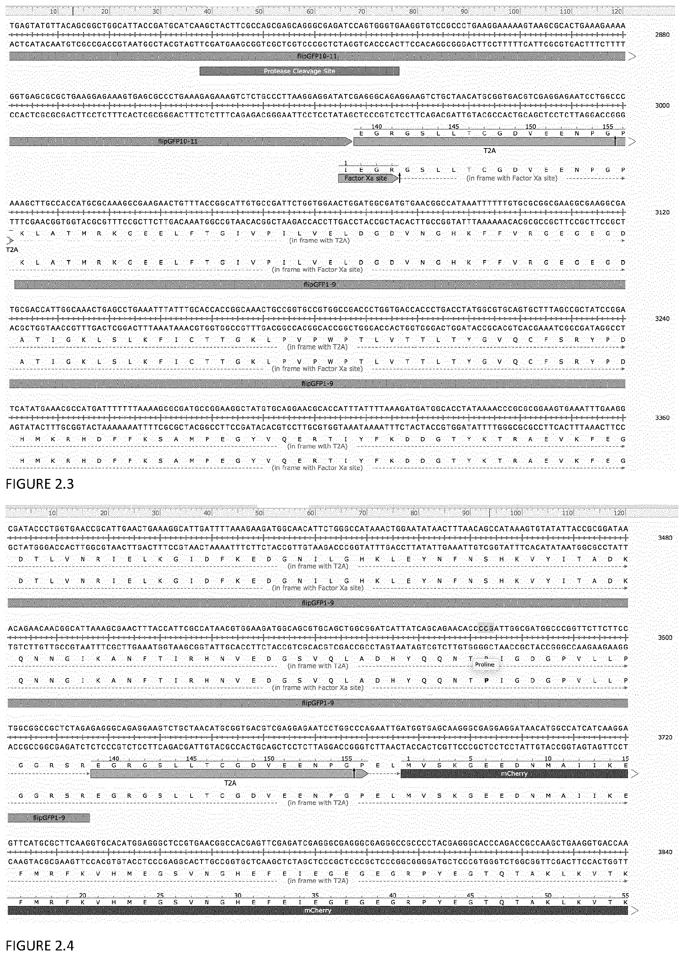

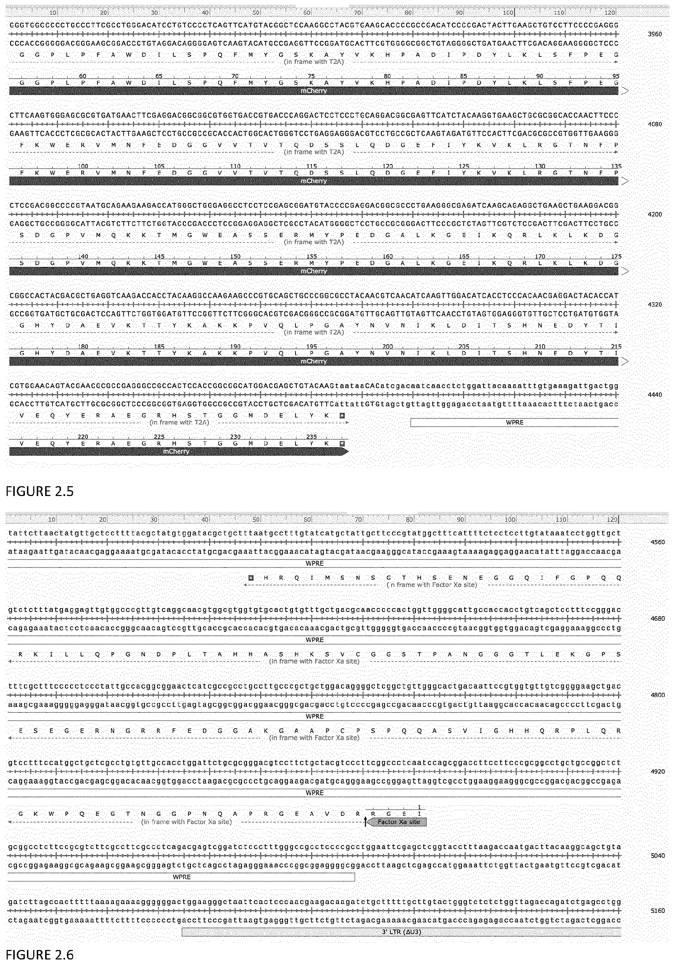

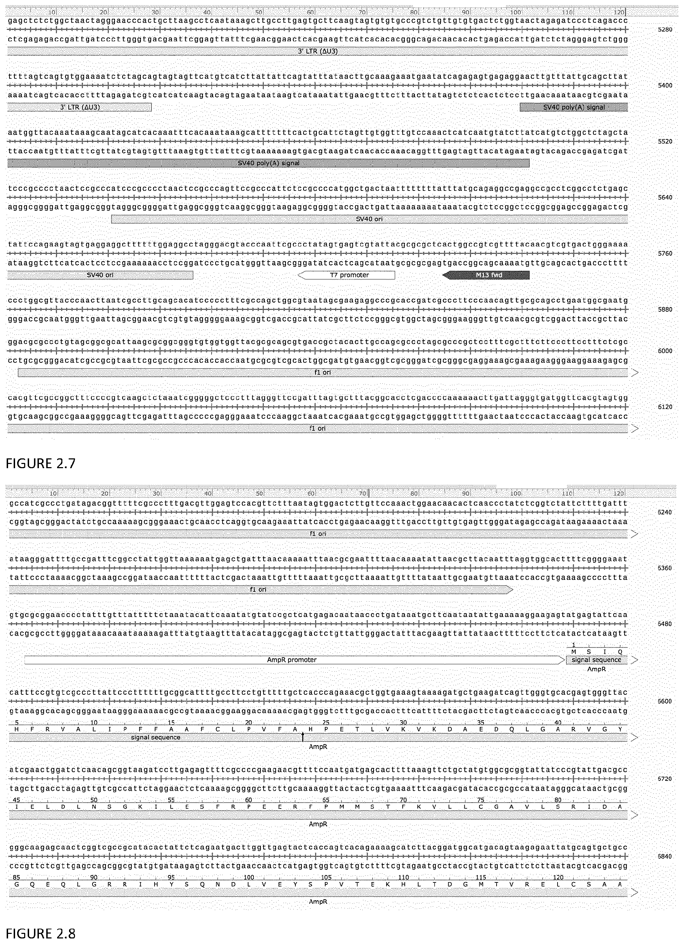

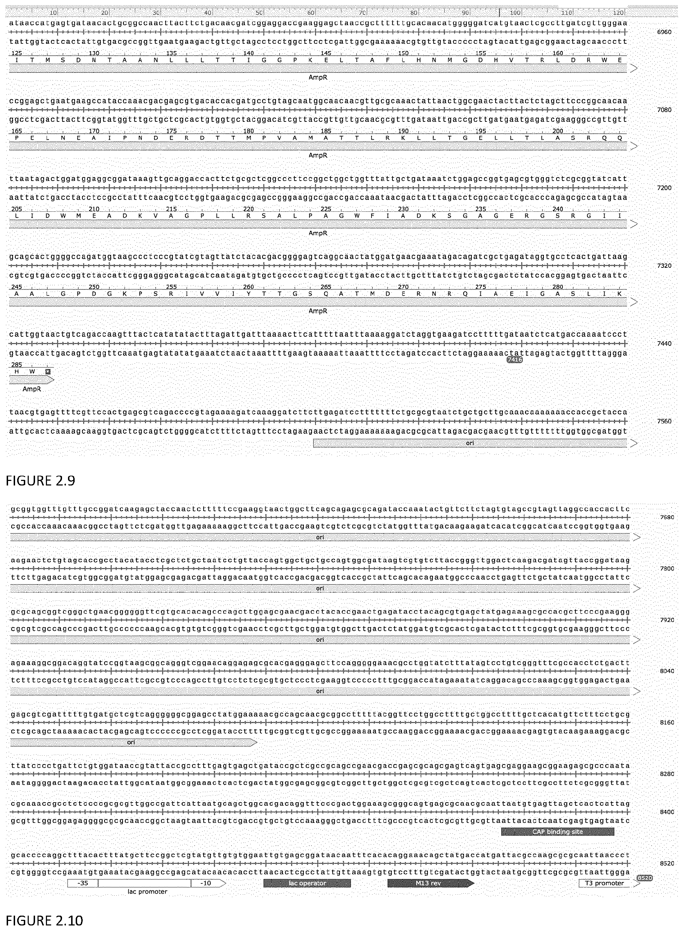

Figure 2 nucleotide and encodes amino acid sequence for pLentiPuro_flipGFP_3C

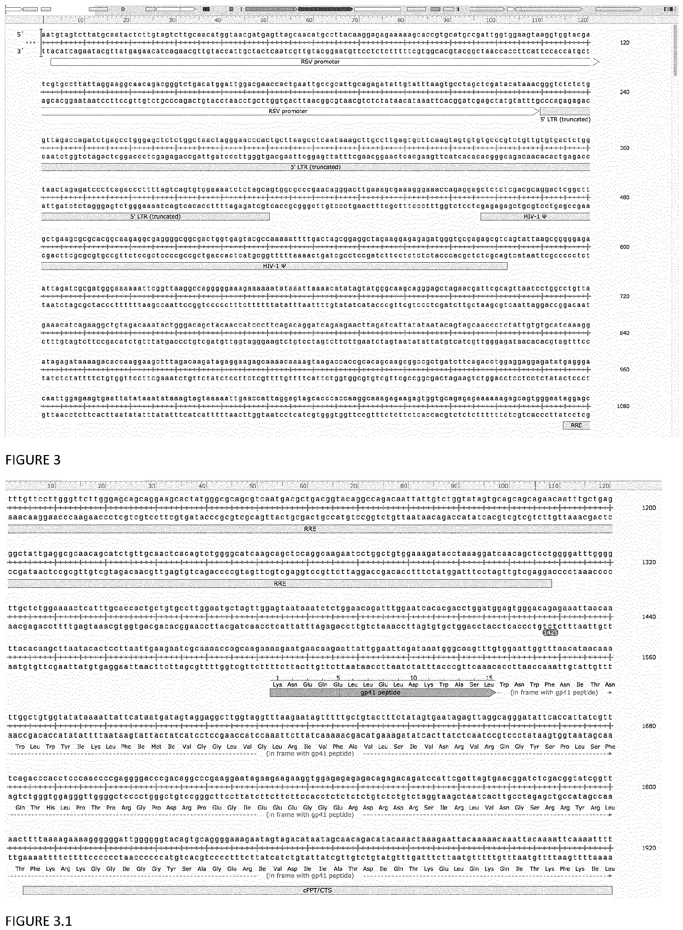

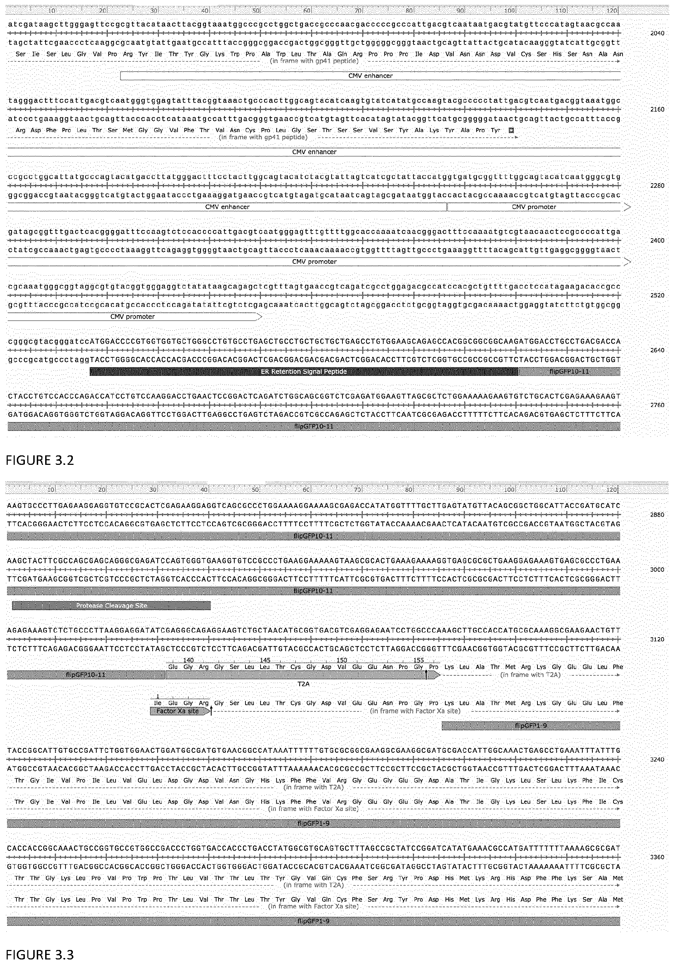

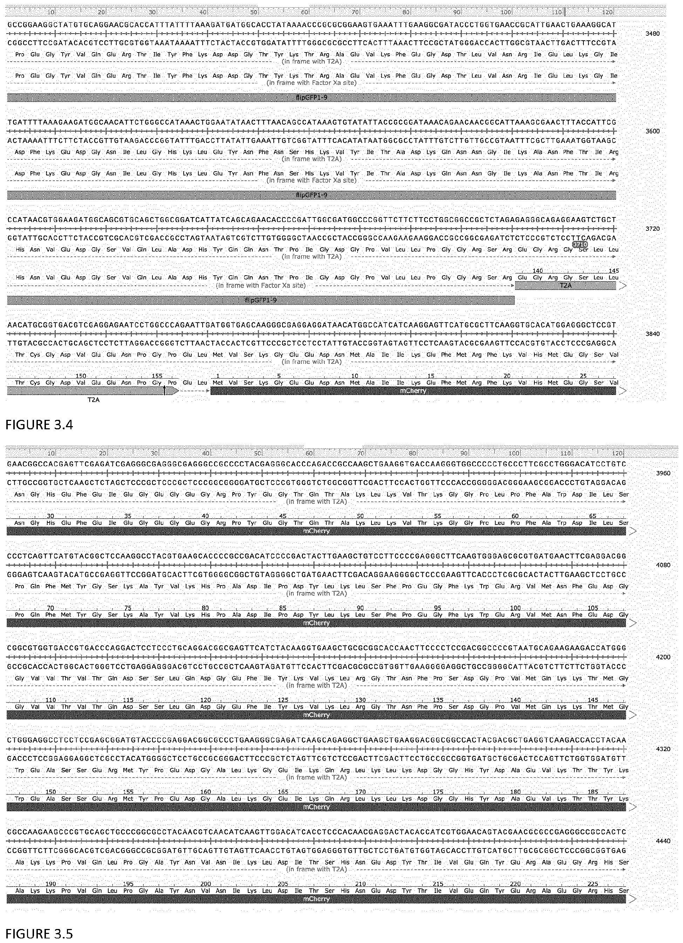

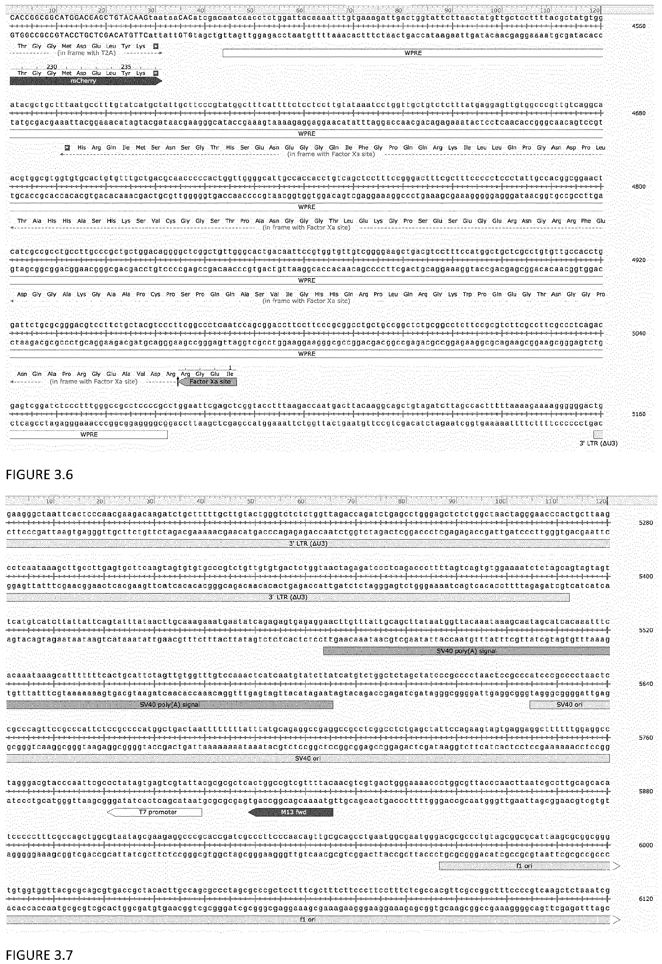

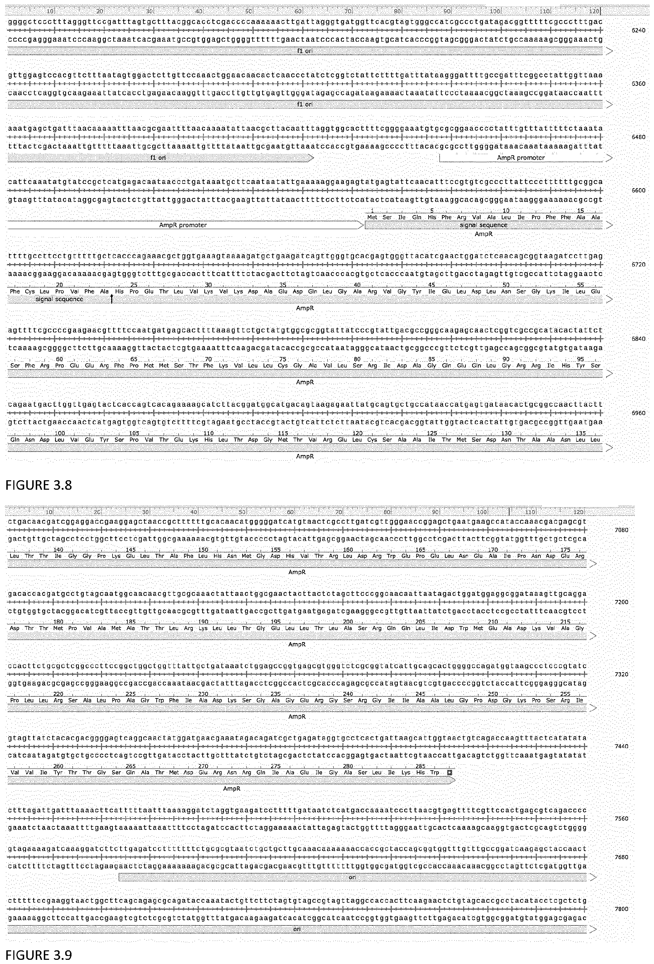

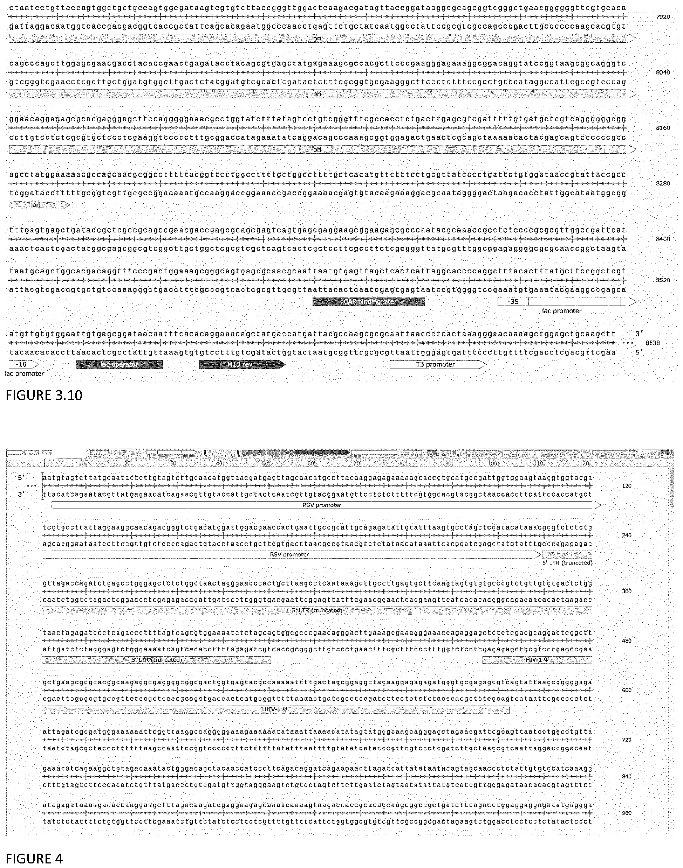

Figure 3: nucleotide and encodes amino acid sequence for pLentiPuro_flipGFP_ER_3C

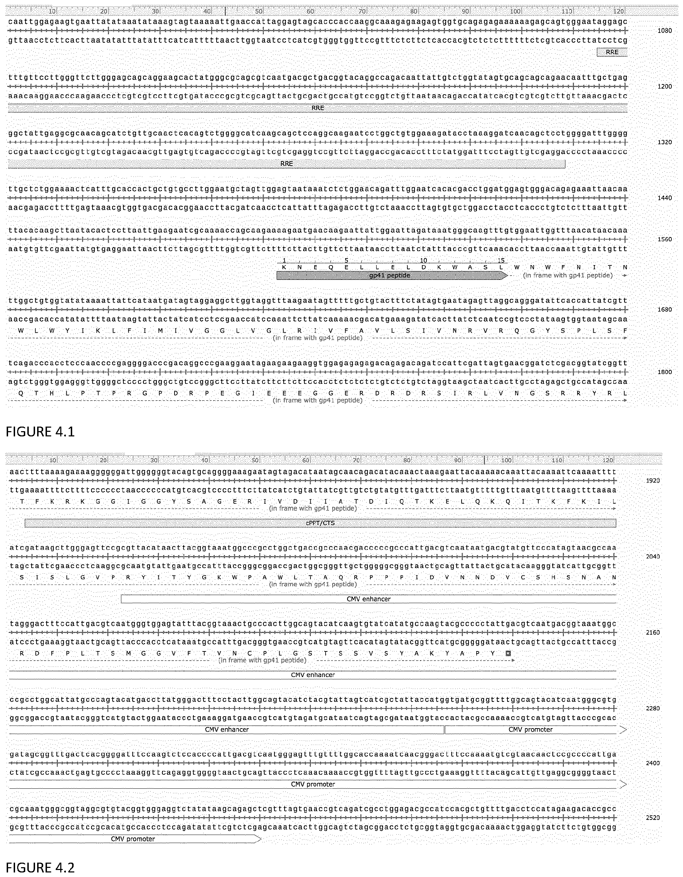

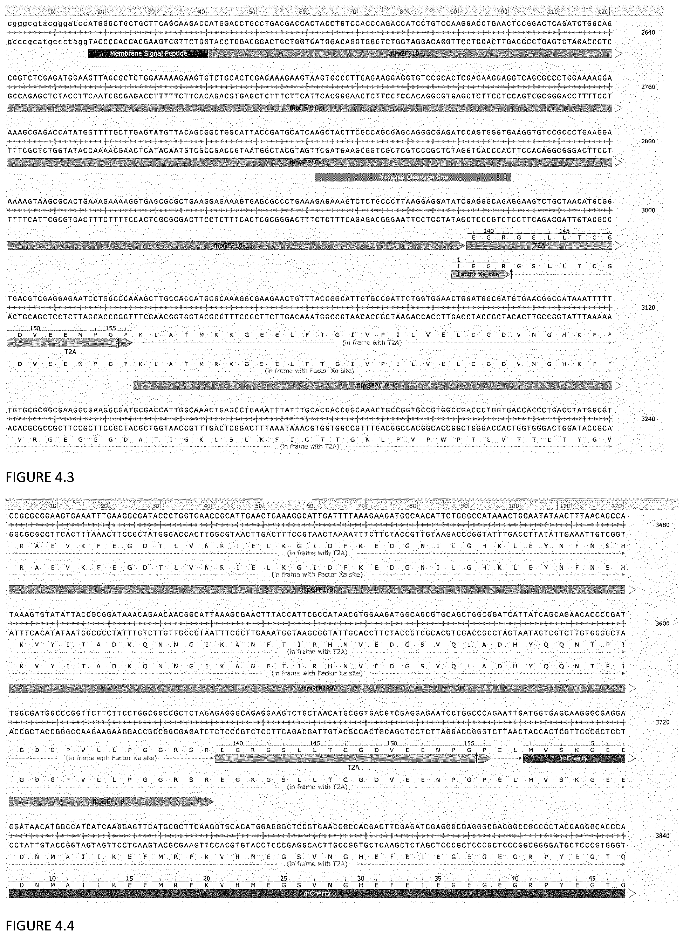

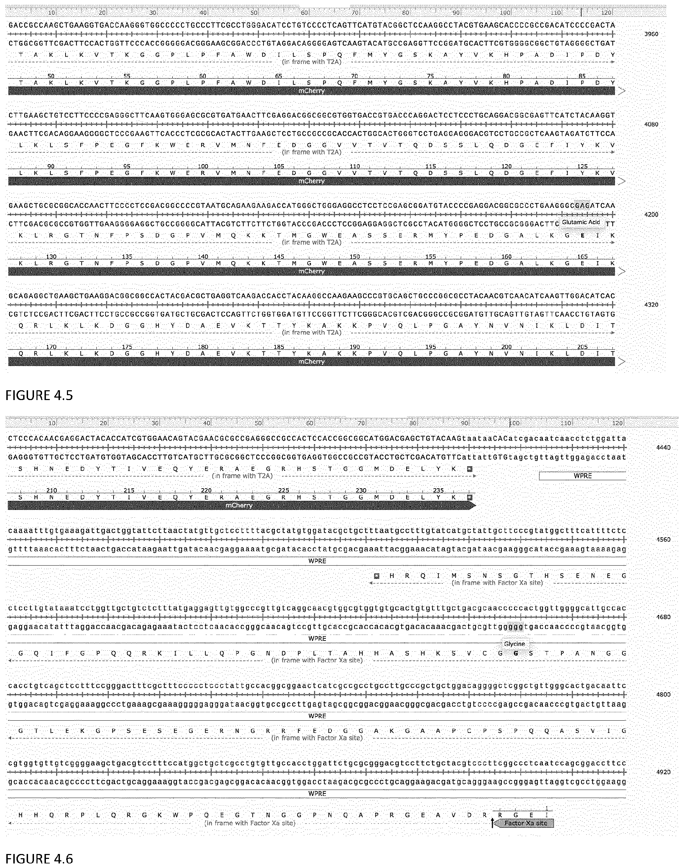

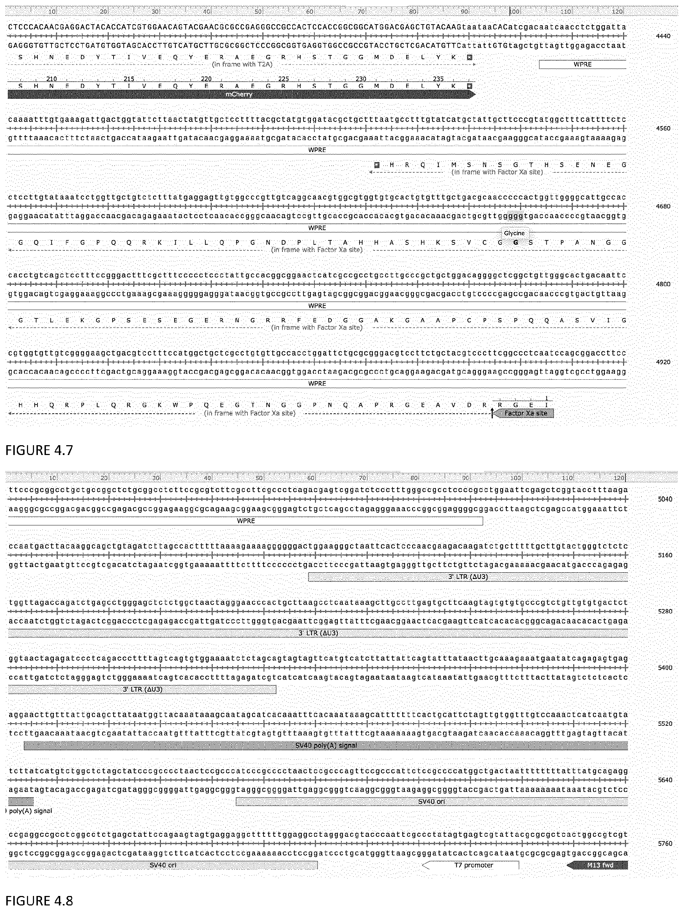

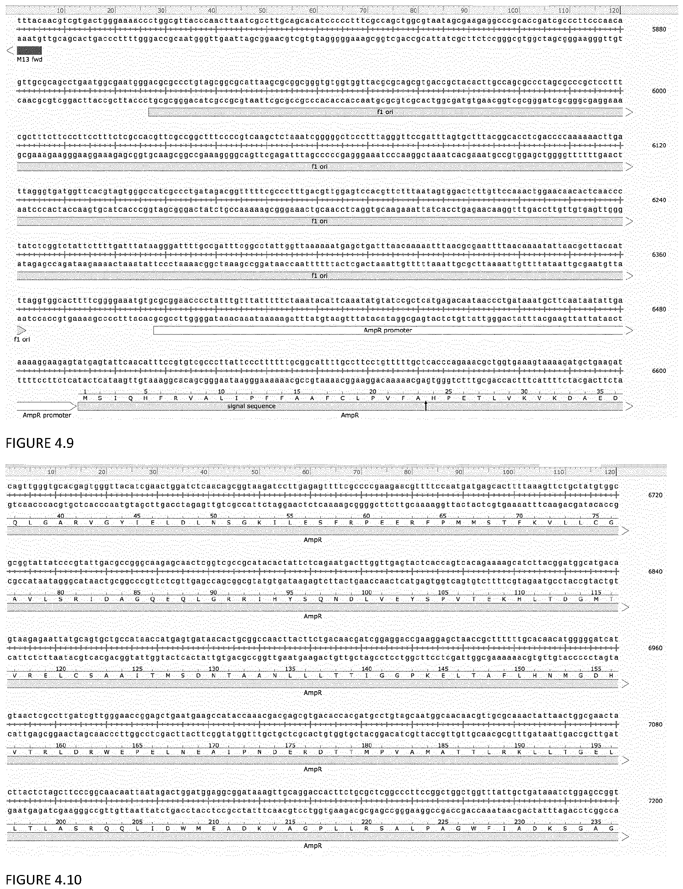

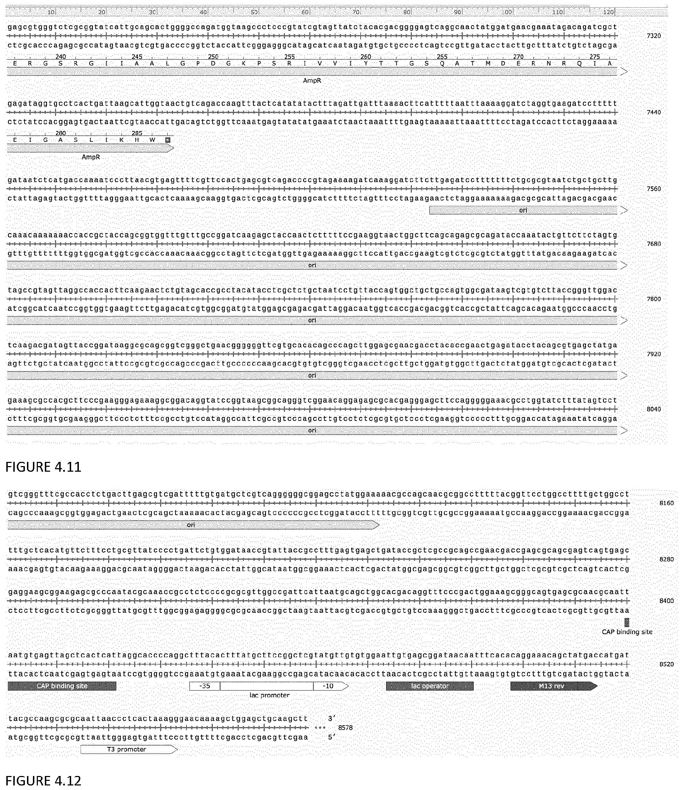

Figure 4: nucleotide and encodes amino acid sequence for pLentiPuro_flipGFP_Membrane_3C

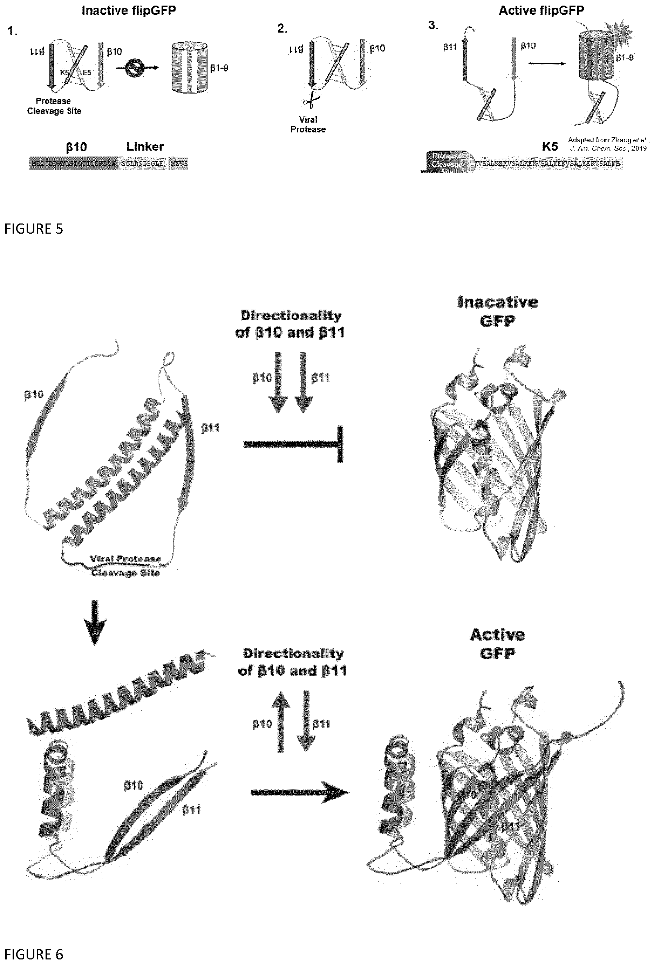

Figures 5 to 7: flipGFP_Schematics
Figure 8 : **flipGFP by Transfection and Transfected Protease** - flipGFP detection of SARS CoV-2 protease:
   HEK293T cells were seeded in 24 well plates. Cells were transfected with the SARS-CoV-2 cleavage-site 3 ER Retention signal peptide flipGFP construct alone (Mock) or together with a plasmid expressing the Nsp5 protease (+Nsp5). Cells were incubated for 24 hours, before fixing with 4% Formalin. Cells were imaged using an EVOS FL microscope.

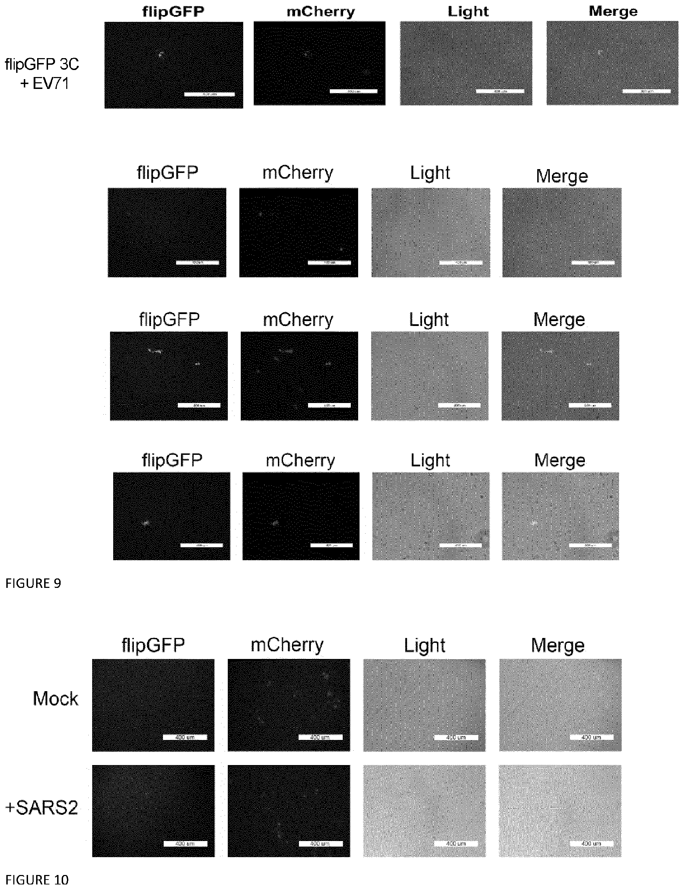

Figures 9,: **flipGFP by Transfection and Virus Infection** - flipGFP detection of chikungunya virus infection, enterovirus 71 infection, CHKV virus infection, SARS CoV-2 infection, ZIK virus infection:
   Vero E6 cells were seeded in 24 well plates. Cells were transfected with the individual flipGFP constructs. After 24 hours cells were infected with either enterovirus 71 (EV71), chikungunya virus (CHKV), SARS-CoV-2 (BetaCoV/France/IDF0372/2020, SARS-CoV-2) or Zika virus (ZIKV) at an MOI = 1 (SARS2). Cells were incubated for a further 48 hours, before fixing with 4% Formalin. Cells were imaged using an EVOS FL microscope.
Figure 10: **flipGFP expressed in Stable Cell Line and Infected-** Stable cell line FlipGFP detection of SARS Cov-2 infection
   A549 cells overexpressing Ace2 were transduced using a lentivirus encoding for the SARS-CoV-2 flipGFP Membrane 3 construct. Cells were selected using puromycin, followed by sorting flow-cytometry sorting, gating for high mCherry and GFP negative expressing cells. Cells were seeded in 24 well plates. Cells were infected with SARS-CoV-2 (BetaCoV/France/IDF0372/2020) at an MOI = 1 (SARS2) or left uninfected (Mock). Cells were incubated for 48 hours, before fixing with 4% Formalin. Cells were imaged using an EVOS FL microscope.

### Experimental data

### Methods

### Cells and Viruses

Vero E6 (Vero 76, clone E6, Vero E6, ATCC^{®} CRL-1586TM) were maintained in a humidified atmosphere at 37°C with 5% CO₂, in Dulbecco's modified Eagle's medium (DMEM, Life Technologies) containing 10% (v/v) fetal bovine serum (FBS, Life Technologies). ACE2-expressing A549 cells, a human lung epithelial cell line (Institut Pasteur; Bouhaddou M. et al - The Global Phosphorylation landscape of SARS-CoV-2 infection https://doi.org/10.1016/j.cell.2020.06.034). A549-ACE2 cells were cultured in DMEM supplemented with 10% (v/v) FBS and maintained at 37°C with 5% CO₂. HEK293T cells were DMEM supplemented with 10% (v/v) FBS and maintained at 37°C with 5% CO₂.

SARS-CoV-2 (BetaCoV/France/IDF0372/2020 isolate) was supplied through the European Virus Archive goes Global (EVAg) platform. The virus was propagated in Vero E6 cells in DMEM supplemented with 2% FBS.

Enterovirus 71 (EV71) Sep006 isolate (GenBank: KX197462.1) was generated using plasmid-based reverse genetics system. For this, the virus sequence was synthesized and cloned into a pCAGGS plasmid that flanked the virus genome sequence with a hammer-head ribozyme (HHRz) at the 5' end and a hepatitis delta ribozyme (HdRz) sequence at the 3' end.

Zika virus used for the identification of DVGs is the African strain MR766 of Zika virus (ZIKV). Accession number for this strain is Genbank # LC002520.1

Chikungunya virus was generated from CHIKV infectious clones derived from the Indian Ocean lineage, ECSA genotype. Accession number for such strain SZ 1050 of this lineage is Genbank # MG664850.1

All experiments with live viruses were performed in compliance with Institut Pasteur Paris's guidelines for Biosafety Level 3 (BSL-3) containment procedures in approved laboratories. All experiments were performed in at least three biologically independent samples.

### flipGFP Plasmids

The template for the flipGFP plasmids was kindly provided by Xiaokun Shu (University of California, San Francisco) (Zhang et al., 2019). The lentivial plasmid pLenti-puro (Guan et al., 2011) was acquired through Addgene (#39481). The plasmid and flipGFP insert were linearized by PCR (see Table 1 for primer sequence). The two fragments were joined using InFusion cloning (Clonetech) according to the manufacturer's instructions. The plasmid was confirmed by Sanger sequencing. The nucleotide sequences for the individual protease cleavage sites were changed by single PCR reactions (Q5, Thermo Scientific) (see Table 1 for primers). The correctness of the inserted sequences was confirmed by Sanger sequencing.

The membrane signal peptide was inserted by a single PCR reaction (Q5, Thermo Scientific) (see Table 1 for primers) before changing the nucleotide sequences for the individual protease cleavage sites by PCR.

The endoplasmatic reticulum (ER) retention signal peptide was inserted by linearizing the pLenti-puro-flipGFP plasmid (see Table 1 for primers) and inserting the nucleotide sequence of the ER retention signal peptide using annealed oligonucleotides by InFusion cloning (Clonetech) according to the manufacturer's instructions. The correct insertion of the peptide sequence was confirmed by Sanger sequencing. The individual protease cleavage sites were altered by PCR (Q5, Thermo Scientific).

### Transient flipGFP expression

HEK293T or Vero E6 cells were seeded on cover slips. The following day cells were transfected with individual flipGFP plasmids using TransIT-LT1 (Mirus) according to the manufacturer's instructions. For infections, transfected cells were incubated for 24 hours prior to infections. To assess the activity of individual SARS-CoV-2 flipGFP constructs, flipGFP plasmids were co-transfected with either pLVX-EF1alpha-nCoV2019-nsp5-2xStrep-IRES-Puro or pLVX-EF1alpha-nCoV2019-nsp5-C145A-2xStrep-IRES-Puro (kindly provided by Nevan Krogan, University of California, San Francisco, Gordon et al., 2020) for the expression of the viral protease.

### Stable flipGFP expression Cells

Individual flipGFP constructs in pLenti-puro were transfected together with pAX2 (Proc Natl Acad Sci U S A. 2011 Jun 21; 108(25): 10343-8. Epub 2011 Jun 6. This plasmid is available through Addgene) and pCMV-VSV-G (published in RNA 2003 Apr;9(4):493-501. This plasmid is available through Addgene) in HEK293T. Lentiviruses were harvested 48 hours post transfection and supernatants were cleared from cellular debris by centrifugation. A549-Ace2 cells were seeded and individual lentivirus containing the flipGFP constructs were used to transduce the target cells using 6 µg/ml Diethylaminoethyl (DEAE)-dextran (Sigma) together with centrifugation using 1,000x g for 1 hour. After two days of incubation, cells containing flipGFP were selected using 10 µg/ml puromycin (Sigma). To obtain a homogeneous population of flipGFP expressing cells, cells were FACS-sorted using mCherry expression as marker for the presence and expression of flipGFP.

### Detection of viral protease and virus infection in transfected cells and in stable cells

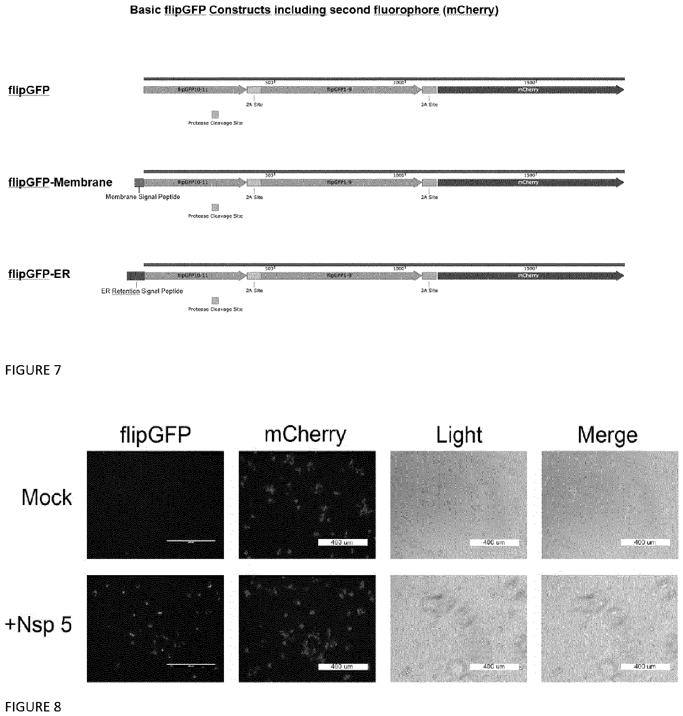

The protocol for transfection and infection of cells has been described in the legends of the figures end the results of the detection are provided on Figures 8, 9 and 10.

**Table 1**

| **SEQ ID No**. and **Name** | **5'-3' Sequence** | **Purpose** |
|---|---|---|
| 1.pLenti-Puro Linear For | GACGAGCTGTACAAGTAATAACACATCGACAATCAAC CTCTGG | Linearizing plasmid for InFusion Cloning to insert flipGFP |
| 2.pLenti-Puro Linear Rev | CGTCAGGCAGGTCCATGGATCCCGTACGCCCGGGCG GTGTC | Linearizing plasmid for InFusion Cloning to insert flipGFP |
| 3.flipGFP For | ATGGACCTGCCTGACGACCACTAC | Preparing flipGFP for Infusion Cloning |
| 4.flipGFP Rev | TTACTTGTACAGCTCGTCCATGCC | Preparing flipGFP for Infusion Cloning |
| 5.Membrane For | GCTGCTGCTTCAGCAAGACCATGGACCTGCCTGACGA CCACTAC | Inserting Membrane Signal Peptide into |
| | | pLenti-Puro plasmid |
| 6.Membrane Rev | CTTGCTGAAGCAGCAGCCCATGGATCCCGTACGCCC GGGCGG | Inserting Membrane Signal Peptide into pLenti-Puro plasmid |
| 7.pLenti-Puro ER For | CACGGCGGCGGCAAGATGGACCTGCCTGACGACCAC | Linearizing plasmid for InFusion Cloning to insert ER Retention Signal Peptide |
| 8.pLenti-Puro ER Rev | CCACCACGGGGTCCATGGATCCCGTACGCCCGGGCG G | Linearizing plasmid for InFusion Cloning to insert ER Retention Signal Peptide |
| 9.ER Signal For | ATGGACCCCGTGGTGGTGCTGGGCCTGTGCCTGAGC TGCCTGCTGCTGCTGAGCCTGTGGAAGCAGAGCCAC GGCGGCGGCAAG | Inserting ER Retention Signal Peptide into pLenti-Puro plasmid |
| 10.ER Signal Rev | CTTGCCGCCGCCGTGGCTCTGCTTCCACAGGCTCAG CAGCAGCAGGCAGCTCAGGCACAGGCCCAGCACCAC CACGGGGTCCAT | Inserting ER Retention Signal Peptide into pLenti-Puro plasmid |
| 11.SARS2-1 For | CGGCGGCGCCTACACCAGGTACGTGAAGGTGTCCGC CCTGAAGGAAAAAG | Generation of flipGFP recognized by SARS-CoV-2 Nsp3 protease |
| 12.SARS2-1 Rev | GGTGTAGGCGCCGCCGTTCAGCTCCCTTGATGCATC GGTAATGCCAGCCGC | Generation of flipGFP recognized by SARS-CoV-2 Nsp3 protease |
| 13.SARS2-2 For | GGGCGGCGCCCCCACCAAGGTGACCAAGGTGTCCGC CCTGAAGGAAAAAG | Generation of flipGFP recognized by SARS-CoV-2 Nsp3 protease |
| 14.SARS2-2 Rev | GGTGGGGGCGCCGCCCTTCAGGGTGAATGATGCATC GGTAATGCCAGCCGC | Generation of flipGFP recognized by SARS-CoV-2 Nsp3 protease |
| 15.SARS2-3 For | GAAGGGCGGCAAGATCGTGAACAACTGGAAGGTGTC CGCCCTGAAGGAAAAAG | Generation of flipGFP recognized by SARS-CoV-2 Nsp5 protease |
| 16.SARS2-3 Rev | CACGATCTTGCCGCCCTTCAGGGCGATTGATGCATCG GTAATGCCAGCCGC | Generation of flipGFP recognized by SARS-CoV-2 Nsp5 protease |
| 17.SARS2-4 For | GTGCTGCAGAGCGGCTTCAGGAAGATGAAGGTGTCC GCCCTGAAGGAAAAAG | Generation of flipGFP recognized by SARS-CoV-2 Nsp5 protease |
| 18.SARS2-4 Rev | CTGAAGCCGCTCTGCAGCACGGCGCTGGTTGATGCA TCGGTAATGCCAGCCGC | Generation of flipGFP recognized by SARS-CoV-2 Nsp5 protease |
| 19.SARS2-5 For | GACCTTCCAGAGCGCCGTGAAGAGGACCAAGGTGTC CGCCCTGAAGGAAAAAG | Generation of flipGFP recognized by SARS-CoV-2 Nsp5 protease |
| 20.SARS2-5 Rev | CACGGCGCTCTGGAAGGTCACGCCGCTTGATGCATC GGTAATGCCAGCCGC | Generation of flipGFP recognized by SARS-CoV-2 Nsp5 protease |
| 21.SARS2-6 For | CACCGTGCAGAGCAAGATGAGCGACGTGAAGGTGTC CGCCCTGAAGGAAAAAG | Generation of flipGFP recognized by SARS-CoV-2 Nsp5 protease |
| 22.SARS2-6 Rev | CATCTTGCTCTGCACGGTGGCCACCTTTGATGCATCG GTAATGCCAGCCGC | Generation of flipGFP recognized by SARS-CoV-2 Nsp5 protease |
| 23.SARS2-7 For | GAAGCTGCAGAACAACGAGCTGAGCCCCAAGGTGTC CGCCCTGAAGGAAAAAG | Generation of flipGFP recognized by SARS-CoV-2 Nsp5 protease |
| 24.SARS2-7 Rev | GCTCGTTGTTCTGCAGCTTCACGGCGCTTGATGCATC GGTAATGCCAGCCGC | Generation of flipGFP recognized by SARS-CoV-2 Nsp5 protease |
| 25.SARS2-8 For | GAGGCTGCAGGCCGGCAACGCCACCGAGAAGGTGTC CGCCCTGAAGGAAAAAG | Generation of flipGFP recognized by SARS-CoV-2 Nsp5 protease |
| 26.SARS2-8 Rev | GTTGCCGGCCTGCAGCCTCACGGTGGCTGATGCATC GGTAATGCCAGCCGC | Generation of flipGFP recognized by SARS-CoV-2 Nsp5 protease |
| 27.SARS2-9 For | CATGCTGCAGAGCGCCGACGCCCAGAGCAAGGTGTC CGCCCTGAAGGAAAAAG | Generation of flipGFP recognized by SARS-CoV-2 Nsp5 protease |
| 28.SARS2-9 Rev | GTCGGCGCTCTGCAGCATGGGCTCCCTTGATGCATC GGTAATGCCAGCCGC | Generation of flipGFP recognized by SARS-CoV-2 Nsp5 protease |
| 29.ZIKV-1 For | GGAAGAGGAGGGGCGCCGACACCAGCATCAAGGTGT CCGCCCTGAAGGAAAAAG | Generation of flipGFP recognized by ZIKV NS2B-NS3 protease |
| 30.ZIKV-1 Rev | GTCGGCGCCCCTCCTCTTCCTCTCCTTTGATGCATCG GTAATGCCAGCCGC | Generation of flipGFP recognized by ZIKV NS2B-NS3 protease |
| 31.ZIKV-2 For | GCGGCAAGAGGAGCTGGCCCCCCAGCGAGAAGGTGT CCGCCCTGAAGGAAAAAG | Generation of flipGFP recognized by ZIKV NS2B-NS3 protease |
| 32.ZIKV-2 Rev | GGGGCCAGCTCCTCTTGCCGCTCCTGGTTGATGCATC GGTAATGCCAGCCGC | Generation of flipGFP recognized by ZIKV NS2B-NS3 protease |
| 33.ZIKV-3 For | GAAGCAGAGGAGCCCCCAGGACAACCAGAAGGTGTC CGCCCTGAAGGAAAAAG | Generation of flipGFP recognized by ZIKV NS2B-NS3 protease |
| 34.ZIKV-3 Rev | CTGGGGGCTCCTCTGCTTCTCGGGCTCTGATGCATCG GTAATGCCAGCCGC | Generation of flipGFP recognized by ZIKV NS2B-NS3 protease |
| 35.ZIKV-4 For | GTGAAGAGGAGGGGCGGCGGCACCGGCGAGAAGGT GTCCGCCCTGAAGGAAAAAG | Generation of flipGFP recognized by ZIKV NS2B-NS3 protease |
| 36.ZIKV-4 Rev | GCCGCCGCCCCTCCTCTTCACCAGGCCTGATGCATC GGTAATGCCAGCCGC | Generation of flipGFP recognized by ZIKV NS2B-NS3 protease |
| 37.CHKV-1 For | GGGCCGGCGCCGGCATCATCGAGACCCCCAAGGTGT CCGCCCTGAAGGAAAAAG | Generation of flipGFP recognized by CHKV Nsp2 protease |
| 38.CHKV-1 Rev | GATGATGCCGGCGCCGGCCCTGTCCTCTGATGCATC GGTAATGCCAGCCGC | Generation of flipGFP recognized by CHKV Nsp2 protease |
| 39.CHKV-2 For | CACCCTGTAGCTGGGGGCGCAGCCGGCCCAAGGTGT CCGCCCTGAAGGAAAAAG | Generation of flipGFP recognized by CHKV Nsp2 protease |
| 40.CHKV-2 Rev | GCTGGGGGCGCAGCCGGCCCTGGTGGCTGATGCATC GGTAATGCCAGCCGC | Generation of flipGFP recognized by CHKV Nsp2 protease |
| 41.CHKV-3 For | GGGCCGGCGGCTACATCTTCAGCAGCGACAAGGTGT CCGCCCTGAAGGAAAAG | Generation of flipGFP recognized by CHKV Nsp2 protease |
| 42.CHKV-3 Rev | GAAGATGTAGCCGCCGGCCCTGTCCAGTGATGCATC GGTAATGCCAGCCGC | Generation of flipGFP recognized by CHKV Nsp2 protease |
| 43.EV71-2A For | CACTCTTGGGAAATTTGGACAAAAGGTGTCCGCCCTG AAGGAAAAAG | Generation of flipGFP recognized by EV71 2A protease |
| 44.EV71-2A | CAAATTTCCCAAGAGTGGTGATTGATGCATCGGTAAT | Generation of flipGFP |
| Rev | GCCAGCCGC | recognized by EV71 2A protease |
| 45.EV71-3C For | CGCCAGCGAGCAGGGCGAGATCCAGTGGGTG AAGGTGTCCGCCCTGAAGGAAAAAG | Generation of flipGFP recognized by EV71 3C protease |
| 46.EV71-3C Rev | GATCTCGCCCTGCTCGCTGGCGAAGTAGCTTGATGCA TCGGTAATGCCAGCCGC | Generation of flipGFP recognized by EV71 3C protease |

The sequence contained in the sequence listing are numbered as follows:

| **(SEQ ID)** | **Nom** |
|---|---|
| 1 | pLenti-Puro Linear For |
| 2 | pLenti-Puro Linear Rev |
| 3 | flipGFP For |
| 4 | flipGFP Rev |
| 5 | Membrane For (nt) |
| 6 | Membrane Rev (nt) |
| 7 | pLenti-Puro ER For (nt) |
| 8 | pLenti-Puro ER Rev (nt) |
| 9 | ER Signal For (nt) |
| 10 | ER Signal Rev (nt) |
| 11 | SARS2-1 For (nt) |
| 12 | SARS2-1 Rev (nt) |
| 13 | SARS2-2 For (nt) |
| 14 | SARS2-2 Rev (nt) |
| 15 | SARS2-3 For (nt) |
| 16 | SARS2-3 Rev (nt) |
| 17 | SARS2-4 For (nt) |
| 18 | SARS2-4 Rev (nt) |
| 19 | SARS2-5 For (nt) |
| 20 | SARS2-5 Rev (nt) |
| 21 | SARS2-6 For (nt) |
| | |
| 22 | SARS2-6 Rev (nt) |
| 23 | SARS2-7 For (nt) |
| 24 | SARS2-7 Rev (nt) |
| 25 | SARS2-8 For (nt) |
| 26 | SARS2-8 Rev (nt) |
| 27 | SARS2-9 For (nt) |
| 28 | SARS2-9 Rev (nt) |
| 29 | ZIKV-1 For (nt) |
| 30 | ZIKV-1 Rev (nt) |
| 31 | ZIKV-2 For (nt) |
| 32 | ZIKV-2 Rev (nt) |
| 33 | ZIKV-3 For (nt) |
| 34 | ZIKV-3 Rev (nt) |
| 35 | ZIKV-4 For (nt) |
| 36 | ZIKV-4 Rev (nt) |
| 37 | CHKV-1 For (nt) |
| 38 | CHKV-1 Rev (nt) |
| 39 | CHKV-2 For (nt) |
| 40 | CHKV-2 Rev (nt) |
| 41 | CHKV-3 For (nt) |
| 42 | CHKV-3 Rev (nt) |
| 43 | EV71-2A For (nt) |
| 44 | EV71-2A Rev (nt) |
| 45 | EV71-3C For (nt) |
| 46 | EV71-3C Rev (nt) |
| 47 | flipGFP_CHKV_1 (nt) |
| 48 | flipGFP_CHKV_2 (nt) |
| 49 | flipGFP_CHKV_3 (nt) |
| 50 | flipGFP_ER_CHKV_1 (nt) |
| 51 | flipGFP_ER_CHKV_2 (nt) |
| 52 | flipGFP_ER_CHKV_3 (nt) |
| 53 | flipGFP_ER_EV71_2A (nt) |
| 54 | flipGFP_ER_EV71_3C (nt) |
| 55 | flipGFP_ER_SARSCoV2_1 (nt) |
| 56 | flipGFP_ER_SARSCoV2_2 (nt) |
| 57 | flipGFP_ER_SARSCoV2_3 (nt) |
| 58 | flipGFP_ER_SARSCoV2_4 (nt) |
| 59 | flipGFP_ER_SARSCoV2_5 (nt) |
| 60 | flipGFP_ER_SARSCoV2_6 (nt) |
| 61 | flipGFP_ER_SARSCoV2_7 (nt) |
| 62 | flipGFP_ER_SARSCoV2_8 (nt) |
| 63 | flipGFP_ER_SARSCoV2_9 (nt) |
| 64 | flipGFP_ER_ZIKV_1 (nt) |
| 65 | flipGFP_ER_ZIKV_2 (nt) |
| 66 | flipGFP_ER_ZIKV_3 (nt) |
| 67 | flipGFP_ER_ZIKV_4 (nt) |
| 68 | flipGFP_EV71_2A (nt) |
| 69 | flipGFP_EV71_3C (nt) |
| 70 | flipGFP_Membrane_CHKV_1 (nt) |
| 71 | flipGFP_Membrane_CHKV_2 (nt) |
| 72 | flipGFP_Membrane_CHKV_3 (nt) |
| 73 | flipGFP_Membrane_EV71_2A (nt) |
| 74 | flipGFP_Membrane_EV71_3C (nt) |
| 75 | flipGFP_Membrane_SARSCoV2_1 (nt) |
| 76 | flipGFP_Membrane_SARSCoV2_2 (nt) |
| 77 | flipGFP_Membrane_SARSCoV2_3 (nt) |
| 78 | flipGFP_Membrane_SARSCoV2_4 (nt) |
| 79 | flipGFP_Membrane_SARSCoV2_5 (nt) |
| 80 | flipGFP_Membrane_SARSCoV2_6 (nt) |
| 81 | flipGFP_Membrane_SARSCoV2_7 (nt) |
| 82 | flipGFP_Membrane_SARSCoV2_8 (nt) |
| 83 | flipGFP_Membrane_SARSCoV2_9 (nt) |
| 84 | flipGFP_Membrane_ZIKV_1 (nt) (nt) |
| 85 | flipGFP_Membrane_ZIKV_2 (nt) |
| 86 | flipGFP_Membrane_ZIKV _3 (nt) |
| 87 | flipGFP_Membrane_ZIKV_4 (nt) |
| 88 | flipGFP_SARSCoV2_1 (nt) |
| 89 | flipGFP_SARSCoV2_2 (nt) |
| 90 | flipGFP_SARSCoV2_3 (nt) |
| 91 | flipGFP_SARSCoV2_4 (nt) |
| 92 | flipGFP_SARSCoV2_5 (nt) |
| 93 | flipGFP_SARSCoV2_6 (nt) |
| 94 | flipGFP_SARSCoV2_7 (nt) |
| 95 | flipGFP_SARSCoV2_8 (nt) |
| 96 | flipGFP_SARSCoV2_9 (nt) |
| 97 | flipGFP_ZIKV_1 (nt) |
| 98 | flipGFP_ZIKV_2 (nt) |
| 99 | flipGFP_ZIKV_3 (nt) |
| 100 | flipGFP_ZIKV_4 (nt) |
| 101 | pLentiPuro_flipGFP_ER_3C(nt) |
| 102 | pLentiPuro_flipGFP_Membrane_3C (nt) |
| 103 | pLentiPuro-flipGFP-3C (nt) |
| 104 | ER retention signal (nt) |
| 105 | ER retention signal/1 (aa) |
| 106 | Membrane targeting signal (nt) |
| 107 | Membrane targeting signal (aa) |
| 108 | cleavage site for for CHKV-1 (nt) |
| 109 | cleavage site for for CHKV-2 |
| 110 | cleavage site for for CHKV-3 |
| 111 | EV71_2A |
| 112 | EV71_3C |
| 113 | SARSCoV2_1 |
| 114 | SARSCoV2_2 |
| 115 | SARSCoV2_3 |
| 116 | SARSCoV2_4 |
| 117 | SARSCoV2_5 |
| 118 | SARSCoV2_6 |
| 119 | SARSCoV2_7 |
| 120 | SARSCoV2_8 |
| 121 | SARSCoV2_9 |
| 122 | ZIKV_1 |
| 123 | ZIKV_2 |
| 124 | ZIKV_3 |
| 125 | ZIKV_4 |
| 126 | Enterovirus 2A protease cleavage site (aa) |
| 127 | Enterovirus 3C protease cleavage site (aa) |
| 128 | Enterovirus 3C protease cleavage site (aa) |
| 129 | Alphaviruses protease cleavage site (aa) |
| 130 | SARS-CoV-2 protease cleavage site (aa) |
| 131 | SARS-CoV-2 protease cleavage site/1 (aa) |
| 132 | SARS-CoV-2 protease cleavage site/2 (aa) |
| 133 | SARS-CoV-2 protease cleavage site/3 (aa) |
| 134 | SARS-CoV-2 protease cleavage site/4 (aa) |
| 135 | SARS-CoV-2 protease cleavage |
| | site/5 (aa) |
| 136 | SARS-CoV-2 protease cleavage site/6 (aa) |
| 137 | SARS-CoV-2 protease cleavage site/7 (aa) |
| 138 | SARS-CoV-2 protease cleavage site/8 (aa) |
| 139 | SARS virus protease cleavage site (aa) |
| 140 | Flavivirus protease cleavage site (aa) |
| 141 | ZIK viruses protease cleavage site (aa) |
| 142 | ZIK viruses protease cleavage site/1 (aa) |
| 143 | ZIK viruses protease cleavage site/2 (aa) |
| 144 | ZIK viruses protease cleavage site/3 (aa) |
| 145 | Flip-GFP |
| 146 | mCherry (nt) |
| 147 | 2A peptide from Thosea asigna virus capsid |

| | |
|---|---|
| 47 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAGAGGACAGGCC GGCGCCGGCATCATCGAGACCCCCAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 48 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAGCCACCAGGGCC GGCTGCGCCCCCAGCTACAGGGTGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 49 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCACTGGACAGGGCC GGCGGCTACATCTTCAGCAGCGACAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 50 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAGAGGACAGGGCCGGCGCCGGCATCATCGAGACCC CCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 51 | ATGGACCCCGTGGTGGTGCTGGGCCTGTGCCTGAGCTGCCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAGCCACCAGGGCCGGCTGCGCCCCCAGCTACAGGG TGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG |
| | GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 52 | ATGGACCCCGTGGTGGTGCTGGGCCTGTGCCTGAGCTGCCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCACTGGACAGGGCCGGCGGCTACATCTTCAGCAGCG ACAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 53 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAATCACCACTCTTGGGAAATTTGGACAAAAGGTGTC CGCCCTGAAGGAAAAAGTAAGCGCACTGAAAGAAAAGGTGAGCGCGCTGAAGGA GAAAGTGAGCGCCCTGAAAGAGAAAGTCTCTGCCCTTAAGGAGGATATCGAGGG CAGAGGAAGTCTGCTAACATGCGGTGACGTCGAGGAGAATCCTGGCCCAAAGCT TGCCACCATGCGCAAAGGCGAAGAACTGTTTACCGGCATTGTGCCGATTCTGGTG GAACTGGATGGCGATGTGAACGGCCATAAATTTTTTGTGCGCGGCGAAGGCGAA GGCGATGCGACCATTGGCAAACTGAGCCTGAAATTTATTTGCACCACCGGCAAAC TGCCGGTGCCGTGGCCGACCCTGGTGACCACCCTGACCTATGGCGTGCAGTGCT TTAGCCGCTATCCGGATCATATGAAACGCCATGATTTTTTTAAAAGCGCGATGCCG GAAGGCTATGTGCAGGAACGCACCATTTATTTTAAAGATGATGGCACCTATAAAAC CCGCGCGGAAGTGAAATTTGAAGGCGATACCCTGGTGAACCGCATTGAACTGAAA GGCATTGATTTTAAAGAAGATGGCAACATTCTGGGCCATAAACTGGAATATAACTT TAACAGCCATAAAGTGTATATTACCGCGGATAAACAGAACAACGGCATTAAAGCG AACTTTACCATTCGCCATAACGTGGAAGATGGCAGCGTGCAGCTGGCGGATCATT ATCAGCAGAACACCCCGATTGGCGATGGCCCGGTTCTTCTTCCTGGCGGCCGCT CTAGA |
| 54 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAGCTACTTCGCCAGCGAGCAGGGCGAGATCCAGT GGGTGAAGGTGTCCGCCCTGAAGGAAAAAGTAAGCGCACTGAAAGAAAAGGTGA GCGCGCTGAAGGAGAAAGTGAGCGCCCTGAAAGAGAAAGTCTCTGCCCTTAAGG AGGATATCGAGGGCAGAGGAAGTCTGCTAACATGCGGTGACGTCGAGGAGAATC CTGGCCCAAAGCTTGCCACCATGCGCAAAGGCGAAGAACTGTTTACCGGCATTGT GCCGATTCTGGTGGAACTGGATGGCGATGTGAACGGCCATAAATTTTTGTGCGC GGCGAAGGCGAAGGCGATGCGACCATTGGCAAACTGAGCCTGAAATTTATTTGCA CCACCGGCAAACTGCCGGTGCCGTGGCCGACCCTGGTGACCACCCTGACCTATG GCGTGCAGTGCTTTAGCCGCTATCCGGATCATATGAAACGCCATGATTTTTTAAA AGCGCGATGCCGGAAGGCTATGTGCAGGAACGCACCATTTATTTTAAAGATGATG GCACCTATAAAACCCGCGCGGAAGTGAAATTTGAAGGCGATACCCTGGTGAACC GCATTGAACTGAAAGGCATTGATTTTAAAGAAGATGGCAACATTCTGGGCCATAAA CTGGAATATAACTTTAACAGCCATAAAGTGTATATTACCGCGGATAAACAGAACAA CGGCATTAAAGCGAACTTTACCATTCGCCATAACGTGGAAGATGGCAGCGTGCAG CTGGCGGATCATTATCAGCAGAACACCCCGATTGGCGATGGCCCGGTTCTTCTTC CTGGCGGCCGCTCTAGA |
| 55 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAGGGAGCTGAACGGCGGCGCCTACACCAGGTACG TGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 56 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCATTCACCCTGAAGGGCGGCGCCCCCACCAAGGTGA CCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC |
| | ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 57 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAATCGCCCTGAAGGGCGGCAAGATCGTGAACAACT GGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 58 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAACCAGCGCCGTGCTGCAGAGCGGCTTCAGGAAGA TGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 59 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAGCGGCGTGACCTTCCAGAGCGCCGTGAAGAGGA CCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 60 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAAGGTGGCCACCGTGCAGAGCAAGATGAGCGACG TGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 61 | ATGGACCCCGTGGTGGTGCTGGGCCTGTGCCTGAGCTGCCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAGCGCCGTGAAGCTGCAGAACAACGAGCTGAGCC CCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC |
| | ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 62 | ATGGACCCCGTGGTGGTGCTGGGCCTGTGCCTGAGCTGCCTGCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAGCCACCGTGAGGCTGCAGGCCGGCAACGCCACCG AGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 63 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAGGGAGCCCATGCTGCAGAGCGCCGACGCCCAGA GCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 64 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAAAGGAGAGGAAGAGGAGGGGCGCCGACACCAGCA TCAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 65 | ATGGACCCCGTGGTGGTGCTGGGCCTGTGCCTGAGCTGCCTGCTGCTGAGC CTGTGGAAGCAGAGCCACGGCGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAACCAGGAGCGGCAAGAGGAGCTGGCCCCCCAGCG AGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 66 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAGAGCCCGAGAAGCAGAGGAGCCCCCAGGACAACC AGAAGGTGTCCGCCCTGAAGGAAAAAGTAAGCGCACTGAAAGAAAAGGTGAGCG CGCTGAAGGAGAAAGTGAGCGCCCTGAAAGAGAAAGTCTCTGCCCTTAAGGAGG ATATCGAGGGCAGAGGAAGTCTGCTAACATGCGGTGACGTCGAGGAGAATCCTG GCCCAAAGCTTGCCACCATGCGCAAAGGCGAAGAACTGTTTACCGGCATTGTGC CGATTCTGGTGGAACTGGATGGCGATGTGAACGGCCATAAATTTTTTGTGCGCGG CGAAGGCGAAGGCGATGCGACCATTGGCAAACTGAGCCTGAAATTTATTTGCACC ACCGGCAAACTGCCGGTGCCGTGGCCGACCCTGGTGACCACCCTGACCTATGGC GTGCAGTGCTTTAGCCGCTATCCGGATCATATGAAACGCCATGATTTTTTTAAAAG CGCGATGCCGGAAGGCTATGTGCAGGAACGCACCATTTATTTTAAAGATGATGGC |
| | ACCTATAAAACCCGCGCGGAAGTGAAATTTGAAGGCGATACCCTGGTGAACCGCA TTGAACTGAAAGGCATTGATTTTAAAGAAGATGGCAACATTCTGGGCCATAAACTG GAATATAACTTTAACAGCCATAAAGTGTATATTACCGCGGATAAACAGAACAACGG CATTAAAGCGAACTTTACCATTCGCCATAACGTGGAAGATGGCAGCGTGCAGCTG GCGGATCATTATCAGCAGAACACCCCGATTGGCGATGGCCCGGTTCTTCTTCCTG GCGGCCGCTCTAGA |
| 67 | ATGGACCCCGTGGTGGTGCTGGGCCTGTGCCTGAGCTGCCTGCTGCTGCTGAGC CTGTGGAAGCAGAGCCACGGCGGCAAGATGGACCTGCCTGACGACCACTA CCTGTCCACCCAGACCATCCTGTCCAAGGACCTGAACTCCGGACTCAGATCTGGC AGCGGTCTCGAGATGGAAGTTAGCGCTCTGGAAAAAGAAGTGTCTGCACTCGAG AAAGAAGTAAGTGCCCTTGAGAAGGAGGTGTCCGCACTCGAGAAGGAGGTCAGC GCCCTGGAAAAGGAAAAGCGAGACCATATGGTTTTGCTTGAGTATGTTACAGCGG CTGGCATTACCGATGCATCAGGCCTGGTGAAGAGGAGGGGCGGCGGCACCGGC GAGAAGGTGTCCGCCCTGAAGGAAAAAGTAAGCGCACTGAAAGAAAAGGTGAGC GCGCTGAAGGAGAAAGTGAGCGCCCTGAAAGAGAAAGTCTCTGCCCTTAAGGAG GATATCGAGGGCAGAGGAAGTCTGCTAACATGCGGTGACGTCGAGGAGAATCCT GGCCCAAAGCTTGCCACCATGCGCAAAGGCGAAGAACTGTTTACCGGCATTGTG CCGATTCTGGTGGAACTGGATGGCGATGTGAACGGCCATAAATTTTTGTGCGCG GCGAAGGCGAAGGCGATGCGACCATTGGCAAACTGAGCCTGAAATTTATTTGCAC CACCGGCAAACTGCCGGTGCCGTGGCCGACCCTGGTGACCACCCTGACCTATGG CGTGCAGTGCTTTAGCCGCTATCCGGATCATATGAAACGCCATGATTTTTTTAAAA GCGCGATGCCGGAAGGCTATGTGCAGGAACGCACCATTTATTTTAAAGATGATGG CACCTATAAAACCCGCGCGGAAGTGAAATTTGAAGGCGATACCCTGGTGAACCGC ATTGAACTGAAAGGCATTGATTTTAAAGAAGATGGCAACATTCTGGGCCATAAACT GGAATATAACTTTAACAGCCATAAAGTGTATATTACCGCGGATAAACAGAACAACG GCATTAAAGCGAACTTTACCATTCGCCATAACGTGGAAGATGGCAGCGTGCAGCT GGCGGATCATTATCAGCAGAACACCCCGATTGGCGATGGCCCGGTTCTTCTTCCT GGCGGCCGCTCTAGA |
| 68 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAATCACCACTCTTG GGAAATTTGGACAAAAGGTGTCCGCCCTGAAGGAAAAAGTAAGCGCACTGAAAGA AAAGGTGAGCGCGCTGAAGGAGAAAGTGAGCGCCCTGAAAGAGAAAGTCTCTGC CCTTAAGGAGGATATCGAGGGCAGAGGAAGTCTGCTAACATGCGGTGACGTCGA GGAGAATCCTGGCCCAAAGCTTGCCACCATGCGCAAAGGCGAAGAACTGTTTAC CGGCATTGTGCCGATTCTGGTGGAACTGGATGGCGATGTGAACGGCCATAAATTT TTTGTGCGCGGCGAAGGCGAAGGCGATGCGACCATTGGCAAACTGAGCCTGAAA TTTATTTGCACCACCGGCAAACTGCCGGTGCCGTGGCCGACCCTGGTGACCACC CTGACCTATGGCGTGCAGTGCTTTAGCCGCTATCCGGATCATATGAAACGCCATG ATTTTTTTAAAAGCGCGATGCCGGAAGGCTATGTGCAGGAACGCACCATTTATTTT AAAGATGATGGCACCTATAAAACCCGCGCGGAAGTGAAATTTGAAGGCGATACCC TGGTGAACCGCATTGAACTGAAAGGCATTGATTTTAAAGAAGATGGCAACATTCTG GGCCATAAACTGGAATATAACTTTAACAGCCATAAAGTGTATATTACCGCGGATAA ACAGAACAACGGCATTAAAGCGAACTTTACCATTCGCCATAACGTGGAAGATGGC AGCGTGCAGCTGGCGGATCATTATCAGCAGAACACCCCGATTGGCGATGGCCCG GTTCTTCTTCCTGGCGGCCGCTCTAGA |
| 69 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAGCTACTTCGCC AGCGAGCAGGGCGAGATCCAGTGGGTGAAGGTGTCCGCCCTGAAGGAAAAAGTA AGCGCACTGAAAGAAAAGGTGAGCGCGCTGAAGGAGAAAGTGAGCGCCCTGAAA GAGAAAGTCTCTGCCCTTAAGGAGGATATCGAGGGCAGAGGAAGTCTGCTAACAT GCGGTGACGTCGAGGAGAATCCTGGCCCAAAGCTTGCCACCATGCGCAAAGGCG AAGAACTGTTTACCGGCATTGTGCCGATTCTGGTGGAACTGGATGGCGATGTGAA CGGCCATAAATTTTTTGTGCGCGGCGAAGGCGAAGGCGATGCGACCATTGGCAA ACTGAGCCTGAAATTTATTTGCACCACCGGCAAACTGCCGGTGCCGTGGCCGAC CCTGGTGACCACCCTGACCTATGGCGTGCAGTGCTTTAGCCGCTATCCGGATCAT ATGAAACGCCATGATTTTTTTAAAAGCGCGATGCCGGAAGGCTATGTGCAGGAAC GCACCATTTATTTTAAAGATGATGGCACCTATAAAACCCGCGCGGAAGTGAAATTT GAAGGCGATACCCTGGTGAACCGCATTGAACTGAAAGGCATTGATTTTAAAGAAG ATGGCAACATTCTGGGCCATAAACTGGAATATAACTTTAACAGCCATAAAGTGTAT ATTACCGCGGATAAACAGAACAACGGCATTAAAGCGAACTTTACCATTCGCCATAA CGTGGAAGATGGCAGCGTGCAGCTGGCGGATCATTATCAGCAGAACACCCCGAT TGGCGATGGCCCGGTTCTTCTTCCTGGCGGCCGCTCTAGA |
| 70 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAGAGGACAGGGCCGGCGCCGGCATCATCGAGACCCCCAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 71 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAGCCACCAGGGCCGGCTGCGCCCCCAGCTACAGGGTGAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT |
| | CATTATCAGCAGAACACCCCGATTGGCGATGGCCCGGTTCTTCTTCCTGGCGGCC GCTCTAGA |
| 72 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCACTGGACAGGGCCGGCGGCTACATCTTCAGCAGCGACAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 73 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAATCACCACTCTTGGGAAATTTGGACAAAAGGTGTCCGCCCTGA AGGAAAAAGTAAGCGCACTGAAAGAAAAGGTGAGCGCGCTGAAGGAGAAAGTGA GCGCCCTGAAAGAGAAAGTCTCTGCCCTTAAGGAGGATATCGAGGGCAGAGGAA GTCTGCTAACATGCGGTGACGTCGAGGAGAATCCTGGCCCAAAGCTTGCCACCA TGCGCAAAGGCGAAGAACTGTTTACCGGCATTGTGCCGATTCTGGTGGAACTGGA TGGCGATGTGAACGGCCATAAATTTTTTGTGCGCGGCGAAGGCGAAGGCGATGC GACCATTGGCAAACTGAGCCTGAAATTTATTTGCACCACCGGCAAACTGCCGGTG CCGTGGCCGACCCTGGTGACCACCCTGACCTATGGCGTGCAGTGCTTTAGCCGC TATCCGGATCATATGAAACGCCATGATTTTTTTAAAAGCGCGATGCCGGAAGGCTA TGTGCAGGAACGCACCATTTATTTTAAAGATGATGGCACCTATAAAACCCGCGCG GAAGTGAAATTTGAAGGCGATACCCTGGTGAACCGCATTGAACTGAAAGGCATTG ATTTTAAAGAAGATGGCAACATTCTGGGCCATAAACTGGAATATAACTTTAACAGC CATAAAGTGTATATTACCGCGGATAAACAGAACAACGGCATTAAAGCGAACTTTAC CATTCGCCATAACGTGGAAGATGGCAGCGTGCAGCTGGCGGATCATTATCAGCA GAACACCCCGATTGGCGATGGCCCGGTTCTTCTTCCTGGCGGCCGCTCTAGA |
| 74 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAGCTACTTCGCCAGCGAGCAGGGCGAGATCCAGTGGGTGAAG GTGTCCGCCCTGAAGGAAAAAGTAAGCGCACTGAAAGAAAAGGTGAGCGCGCTG AAGGAGAAAGTGAGCGCCCTGAAAGAGAAAGTCTCTGCCCTTAAGGAGGATATC GAGGGCAGAGGAAGTCTGCTAACATGCGGTGACGTCGAGGAGAATCCTGGCCCA AAGCTTGCCACCATGCGCAAAGGCGAAGAACTGTTTACCGGCATTGTGCCGATTC TGGTGGAACTGGATGGCGATGTGAACGGCCATAAATTTTTTGTGCGCGGCGAAG GCGAAGGCGATGCGACCATTGGCAAACTGAGCCTGAAATTTATTTGCACCACCGG CAAACTGCCGGTGCCGTGGCCGACCCTGGTGACCACCCTGACCTATGGCGTGCA GTGCTTTAGCCGCTATCCGGATCATATGAAACGCCATGATTTTTTTAAAAGCGCGA TGCCGGAAGGCTATGTGCAGGAACGCACCATTTATTTTAAAGATGATGGCACCTA TAAAACCCGCGCGGAAGTGAAATTTGAAGGCGATACCCTGGTGAACCGCATTGAA CTGAAAGGCATTGATTTTAAAGAAGATGGCAACATTCTGGGCCATAAACTGGAATA TAACTTTAACAGCCATAAAGTGTATATTACCGCGGATAAACAGAACAACGGCATTA AAGCGAACTTTACCATTCGCCATAACGTGGAAGATGGCAGCGTGCAGCTGGCGG ATCATTATCAGCAGAACACCCCGATTGGCGATGGCCCGGTTCTTCTTCCTGGCGG CCGCTCTAGA |
| 75 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAGGGAGCTGAACGGCGGCGCCTACACCAGGTACGTGAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 76 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCATTCACCCTGAAGGGCGGCGCCCCCACCAAGGTGACCAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG |
| | CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 77 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAATCGCCCTGAAGGGCGGCAAGATCGTGAACAACTGGAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 78 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAACCAGCGCCGTGCTGCAGAGCGGCTTCAGGAAGATGAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 79 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAGCGGCGTGACCTTCCAGAGCGCCGTGAAGAGGACCAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 80 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAAGGTGGCCACCGTGCAGAGCAAGATGAGCGACGTGAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 81 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAGCGCCGTGAAGCTGCAGAACAACGAGCTGAGCCCCAAGGTG TCCGCCCTGAAGGAAAAAGTAAGCGCACTGAAAGAAAAGGTGAGCGCGCTGAAG GAGAAAGTGAGCGCCCTGAAAGAGAAAGTCTCTGCCCTTAAGGAGGATATCGAG GGCAGAGGAAGTCTGCTAACATGCGGTGACGTCGAGGAGAATCCTGGCCCAAAG CTTGCCACCATGCGCAAAGGCGAAGAACTGTTTACCGGCATTGTGCCGATTCTGG TGGAACTGGATGGCGATGTGAACGGCCATAAATTTTTTGTGCGCGGCGAAGGCG AAGGCGATGCGACCATTGGCAAACTGAGCCTGAAATTTATTTGCACCACCGGCAA ACTGCCGGTGCCGTGGCCGACCCTGGTGACCACCCTGACCTATGGCGTGCAGTG CTTTAGCCGCTATCCGGATCATATGAAACGCCATGATTTTTTTAAAAGCGCGATGC CGGAAGGCTATGTGCAGGAACGCACCATTTATTTTAAAGATGATGGCACCTATAAA ACCCGCGCGGAAGTGAAATTTGAAGGCGATACCCTGGTGAACCGCATTGAACTG AAAGGCATTGATTTTAAAGAAGATGGCAACATTCTGGGCCATAAACTGGAATATAA CTTTAACAGCCATAAAGTGTATATTACCGCGGATAAACAGAACAACGGCATTAAAG |
| | CGAACTTTACCATTCGCCATAACGTGGAAGATGGCAGCGTGCAGCTGGCGGATC ATTATCAGCAGAACACCCCGATTGGCGATGGCCCGGTTCTTCTTCCTGGCGGCC GCTCTAGA |
| 82 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAGCCACCGTGAGGCTGCAGGCCGGCAACGCCACCGAGAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 83 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAGGGAGCCCATGCTGCAGAGCGCCGACGCCCAGAGCAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 84 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAAAGGAGAGGAAGAGGAGGGGCGCCGACACCAGCATCAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 85 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAACCAGGAGCGGCAAGAGGAGCTGGCCCCCAGCGAGAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 86 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAGAGCCCGAGAAGCAGAGGAGCCCCCAGGACAACCAGAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA |
| | GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 87 | ATGGGCTGCTGCTTCAGCAAGACCATGGACCTGCCTGACGACCACTACCTGTCCA CCCAGACCATCCTGTCCAAGGACCTGAACTCCGGACTCAGATCTGGCAGCGGTC TCGAGATGGAAGTTAGCGCTCTGGAAAAAGAAGTGTCTGCACTCGAGAAAGAAGT AAGTGCCCTTGAGAAGGAGGTGTCCGCACTCGAGAAGGAGGTCAGCGCCCTGGA AAAGGAAAAGCGAGACCATATGGTTTTGCTTGAGTATGTTACAGCGGCTGGCATT ACCGATGCATCAGGCCTGGTGAAGAGGAGGGGCGGCGGCACCGGCGAGAAGGT GTCCGCCCTGAAGGAAAAAGTAAGCGCACTGAAAGAAAAGGTGAGCGCGCTGAA GGAGAAAGTGAGCGCCCTGAAAGAGAAAGTCTCTGCCCTTAAGGAGGATATCGA GGGCAGAGGAAGTCTGCTAACATGCGGTGACGTCGAGGAGAATCCTGGCCCAAA GCTTGCCACCATGCGCAAAGGCGAAGAACTGTTTACCGGCATTGTGCCGATTCTG GTGGAACTGGATGGCGATGTGAACGGCCATAAATTTTTTGTGCGCGGCGAAGGC GAAGGCGATGCGACCATTGGCAAACTGAGCCTGAAATTTATTTGCACCACCGGCA AACTGCCGGTGCCGTGGCCGACCCTGGTGACCACCCTGACCTATGGCGTGCAGT GCTTTAGCCGCTATCCGGATCATATGAAACGCCATGATTTTTTTAAAAGCGCGATG CCGGAAGGCTATGTGCAGGAACGCACCATTTATTTTAAAGATGATGGCACCTATA AAACCCGCGCGGAAGTGAAATTTGAAGGCGATACCCTGGTGAACCGCATTGAACT GAAAGGCATTGATTTTAAAGAAGATGGCAACATTCTGGGCCATAAACTGGAATATA ACTTTAACAGCCATAAAGTGTATATTACCGCGGATAAACAGAACAACGGCATTAAA GCGAACTTTACCATTCGCCATAACGTGGAAGATGGCAGCGTGCAGCTGGCGGAT CATTATCAGCAGAACACCCCGATTGGCGATGGCCCGGTTCTTCTTCCTGGCGGCC GCTCTAGA |
| 88 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAGGGAGCTGAAC GGCGGCGCCTACACCAGGTACGTGAAGGTGTCCGCCCTGAAGGAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 89 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCATTCACCCTGAAG GGCGGCGCCCCCACCAAGGTGACCAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG |
| | AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 90 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAATCGCCCTGAAG GGCGGCAAGATCGTGAACAACTGGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 91 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAACCAGCGCCGTG CTGCAGAGCGGCTTCAGGAAGATGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 92 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAGCGGCGTGACC TTCCAGAGCGCCGTGAAGAGGACCAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG |
| | AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 93 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAAGGTGGCCACC GTGCAGAGCAAGATGAGCGACGTGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 94 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAGCGCCGTGAAG CTGCAGAACAACGAGCTGAGCCCCAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 95 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAGCCACCGTGAGG CTGCAGGCCGGCAACGCCACCGAGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG |
| | AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 96 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAGGGAGCCCATG CTGCAGAGCGCCGACGCCCAGAGCAAGGTGTCCGCCCTGAAGGAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 97 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAAGGAGAGGAAG AGGAGGGGCGCCGACACCAGCATCAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 98 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAACCAGGAGCGGC AAGAGGAGCTGGCCCCCCAGCGAGAAGGTGTCCGCCCTGAAGGAAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG |
| | AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 99 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAGAGCCCGAGAAG CAGAGGAGCCCCCAGGACAACCAGAAGGTGTCCGCCCTGAAGGAAAAGTAAGC GCACTGAAAGAAAAGGTGAGCGCGCTGAAGGAGAAAGTGAGCGCCCTGAAAGAG AAAGTCTCTGCCCTTAAGGAGGATATCGAGGGCAGAGGAAGTCTGCTAACATGCG GTGACGTCGAGGAGAATCCTGGCCCAAAGCTTGCCACCATGCGCAAAGGCGAAG AACTGTTTACCGGCATTGTGCCGATTCTGGTGGAACTGGATGGCGATGTGAACGG CCATAAATTTTTTGTGCGCGGCGAAGGCGAAGGCGATGCGACCATTGGCAAACTG AGCCTGAAATTTATTTGCACCACCGGCAAACTGCCGGTGCCGTGGCCGACCCTG GTGACCACCCTGACCTATGGCGTGCAGTGCTTTAGCCGCTATCCGGATCATATGA AACGCCATGATTTTTTTAAAAGCGCGATGCCGGAAGGCTATGTGCAGGAACGCAC CATTTATTTTAAAGATGATGGCACCTATAAAACCCGCGCGGAAGTGAAATTTGAAG GCGATACCCTGGTGAACCGCATTGAACTGAAAGGCATTGATTTTAAAGAAGATGG CAACATTCTGGGCCATAAACTGGAATATAACTTTAACAGCCATAAAGTGTATATTA CCGCGGATAAACAGAACAACGGCATTAAAGCGAACTTTACCATTCGCCATAACGT GGAAGATGGCAGCGTGCAGCTGGCGGATCATTATCAGCAGAACACCCCGATTGG CGATGGCCCGGTTCTTCTTCCTGGCGGCCGCTCTAGA |
| 100 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAGGCCTGGTGAAG AGGAGGGGCGGCGGCACCGGCGAGAAGGTGTCCGCCCTGAAGGAAAAAGTAAG CGCACTGAAAGAAAAGGTGAGCGCGCTGAAGGAGAAAGTGAGCGCCCTGAAAGA GAAAGTCTCTGCCCTTAAGGAGGATATCGAGGGCAGAGGAAGTCTGCTAACATGC GGTGACGTCGAGGAGAATCCTGGCCCAAAGCTTGCCACCATGCGCAAAGGCGAA GAACTGTTTACCGGCATTGTGCCGATTCTGGTGGAACTGGATGGCGATGTGAACG GCCATAAATTTTTTGTGCGCGGCGAAGGCGAAGGCGATGCGACCATTGGCAAACT GAGCCTGAAATTTATTTGCACCACCGGCAAACTGCCGGTGCCGTGGCCGACCCT GGTGACCACCCTGACCTATGGCGTGCAGTGCTTTAGCCGCTATCCGGATCATATG AAACGCCATGATTTTTTTAAAAGCGCGATGCCGGAAGGCTATGTGCAGGAACGCA CCATTTATTTTAAAGATGATGGCACCTATAAAACCCGCGCGGAAGTGAAATTTGAA GGCGATACCCTGGTGAACCGCATTGAACTGAAAGGCATTGATTTTAAAGAAGATG GCAACATTCTGGGCCATAAACTGGAATATAACTTTAACAGCCATAAAGTGTATATT ACCGCGGATAAACAGAACAACGGCATTAAAGCGAACTTTACCATTCGCCATAACG TGGAAGATGGCAGCGTGCAGCTGGCGGATCATTATCAGCAGAACACCCCGATTG GCGATGGCCCGGTTCTTCTTCCTGGCGGCCGCTCTAGA |
| 101 | aatgtagtcttatgcaatactcttgtagtcttgcaacatggtaacgatgagttagcaacatgccttacaaggagagaaaa gcaccgtgcatgccgattggtggaagtaaggtggtacgatcgtgccttattaggaaggcaacagacgggtctgacatgg attggacgaaccactgaattgccgcattgcagagatattgtatttaagtgcctagctcgatacataaacgggtctctctggtt agaccagatctgagcctgggagctctctggctaactagggaacccactgcttaagcctcaataaagcttgccttgagtgct tcaagtagtgtgtgcccgtctgttgtgtgactctggtaactagagatccctcagacccttttagtcagtgtggaaaatctctag cagtggcgcccgaacagggacttgaaagcgaaagggaaaccagaggagctctctcgacgcaggactcggcttgctg aagcgcgcacggcaagaggcgaggggcggcgactggtgagtacgccaaaaattttgactagcggaggctagaagg agagagatgggtgcgagagcgtcagtattaagcgggggagaattagatcgcgatgggaaaaaattcggttaaggcca gggggaaagaaaaaatataaattaaaacatatagtatgggcaagcagggagctagaacgattcgcagttaatcctgg cctgttagaaacatcagaaggctgtagacaaatactgggacagctacaaccatcccttcagacaggatcagaagaact tagatcattatataatacagtagcaaccctctattgtgtgcatcaaaggatagagataaaagacaccaaggaagctttag acaagatagaggaagagcaaaacaaaagtaagaccaccgcacagcaagcggccgctgatcttcagacctggagg aggagatatgagggacaattggagaagtgaattatataaatataaagtagtaaaaattgaaccattaggagtagcaccc accaaggcaaagagaagagtggtgcagagagaaaaaagagcagtgggaataggagctttgttccttgggttcttggg agcagcaggaagcactatgggcgcagcgtcaatgacgctgacggtacaggccagacaattattgtctggtatagtgca gcagcagaacaatttgctgagggctattgaggcgcaacagcatctgttgcaactcacagtctggggcatcaagcagctc caggcaagaatcctggctgtggaaagatacctaaaggatcaacagctcctggggatttggggttgctctggaaaactcat ttgcaccactgctgtgccttggaatgctagttggagtaataaatctctggaacagatttggaatcacacgacctggatggag tgggacagagaaattaacaattacacaagcttaatacactccttaattgaagaatcgcaaaaccagcaagaaaagaat gaacaagaattattggaattagataaatgggcaagtttgtggaattggtttaacataacaaattggctgtggtatataaaatt attcataatgatagtaggaggcttggtaggtttaagaatagtttttgctgtactttctatagtgaatagagttaggcagggatatt caccattatcgtttcagacccacctcccaaccccgaggggacccgacaggcccgaaggaatagaagaagaaggtgg agagagagacagagacagatccattcgattagtgaacggatctcgacggtatcggttaacttttaaaagaaaagggggg gattggggggtacagtgcaggggaaagaatagtagacataatagcaacagacatacaaactaaagaattacaaaaa caaattacaaaattcaaaattttatcgataagcttgggagttccgcgttacataacttacggtaaatggcccgcctggctga ccgcccaacgacccccgcccattgacgtcaataatgacgtatgttcccatagtaacgccaatagggactttccattgacgt caatgggtggagtatttacggtaaactgcccacttggcagtacatcaagtgtatcatatgccaagtacgccccctattgac gtcaatgacggtaaatggcccgcctggcattatgcccagtacatgaccttatgggactttcctacttggcagtacatctacgt attagtcatcgctattaccatggtgatgcggttttggcagtacatcaatgggcgtggatagcggtttgactcacggggatttcc aagtctccaccccattgacgtcaatgggagtttgttttggcaccaaaatcaacgggactttccaaaatgtcgtaacaactcc gccccattgacgcaaatgggcggtaggcgtgtacggtgggaggtctatataagcagagctcgtttagtgaaccgtcagat cgcctggagacgccatccacgctgttttgacctccatagaagacaccgcccgggcgtacgggatccATGGACCC CGTGGTGGTGCTGGGCCTGTGCCTGAGCTGCCTGCTGCTGCTGAGCCTGTGGAA GCAGAGCCACGGCGGCGGCAAGATGGACCTGCCTGACGACCACTACCTGTCCAC CCAGACCATCCTGTCCAAGGACCTGAACTCCGGACTCAGATCTGGCAGCGGTCT CGAGATGGAAGTTAGCGCTCTGGAAAAAGAAGTGTCTGCACTCGAGAAAGAAGTA AGTGCCCTTGAGAAGGAGGTGTCCGCACTCGAGAAGGAGGTCAGCGCCCTGGAA AAGGAAAAGCGAGACCATATGGTTTTGCTTGAGTATGTTACAGCGGCTGGCATTA CCGATGCATCAAGCTACTTCGCCAGCGAGCAGGGCGAGATCCAGTGGGTGAAGG TGTCCGCCCTGAAGGAAAAAGTAAGCGCACTGAAAGAAAAGGTGAGCGCGCTGA AGGAGAAAGTGAGCGCCCTGAAAGAGAAAGTCTCTGCCCTTAAGGAGGATATCG AGGGCAGAGGAAGTCTGCTAACATGCGGTGACGTCGAGGAGAATCCTGGCCCAA AGCTTGCCACCATGCGCAAAGGCGAAGAACTGTTTACCGGCATTGTGCCGATTCT GGTGGAACTGGATGGCGATGTGAACGGCCATAAATTTTTTGTGCGCGGCGAAGG CGAAGGCGATGCGACCATTGGCAAACTGAGCCTGAAATTTATTTGCACCACCGGC AAACTGCCGGTGCCGTGGCCGACCCTGGTGACCACCCTGACCTATGGCGTGCAG TGCTTTAGCCGCTATCCGGATCATATGAAACGCCATGATTTTTTTAAAAGCGCGAT GCCGGAAGGCTATGTGCAGGAACGCACCATTTATTTTAAAGATGATGGCACCTAT AAAACCCGCGCGGAAGTGAAATTTGAAGGCGATACCCTGGTGAACCGCATTGAAC TGAAAGGCATTGATTTTAAAGAAGATGGCAACATTCTGGGCCATAAACTGGAATAT AACTTTAACAGCCATAAAGTGTATATTACCGCGGATAAACAGAACAACGGCATTAA AGCGAACTTTACCATTCGCCATAACGTGGAAGATGGCAGCGTGCAGCTGGCGGA TCATTATCAGCAGAACACCCCGATTGGCGATGGCCCGGTTCTTCTTCCTGGCGGC CGCTCTAGAGAGGGCAGAGGAAGTCTGCTAACATGCGGTGACGTCGAGGAGAAT CCTGGCCCAGAATTGATGGTGAGCAAGGGCGAGGAGGATAACATGGCCATCATC AAGGAGTTCATGCGCTTCAAGGTGCACATGGAGGGCTCCGTGAACGGCCACGAG TTCGAGATCGAGGGCGAGGGCGAGGGCCGCCCCTACGAGGGCACCCAGACCGC CAAGCTGAAGGTGACCAAGGGTGGCCCCCTGCCCTTCGCCTGGGACATCCTGTC CCCTCAGTTCATGTACGGCTCCAAGGCCTACGTGAAGCACCCCGCCGACATCCC CGACTACTTGAAGCTGTCCTTCCCCGAGGGCTTCAAGTGGGAGCGCGTGATGAA |
| | CTTCGAGGACGGCGGCGTGGTGACCGTGACCCAGGACTCCTCCCTGCAGGACG GCGAGTTCATCTACAAGGTGAAGCTGCGCGGCACCAACTTCCCCTCCGACGGCC CCGTAATGCAGAAGAAGACCATGGGCTGGGAGGCCTCCTCCGAGCGGATGTACC CCGAGGACGGCGCCCTGAAGGGCGAGATCAAGCAGAGGCTGAAGCTGAAGGAC GGCGGCCACTACGACGCTGAGGTCAAGACCACCTACAAGGCCAAGAAGCCCGTG CAGCTGCCCGGCGCCTACAACGTCAACATCAAGTTGGACATCACCTCCCACAACG AGGACTACACCATCGTGGAACAGTACGAACGCGCCGAGGGCCGCCACTCCACCG GCGGCATGGACGAGCTGTACAAGtaataaCACatcgacaatcaacctctggattacaaaatttgtgaa agattgactggtattcttaactatgttgctccttttacgctatgtggatacgctgctttaatgcctttgtatcatgctattgcttcccgt atggctttcattttctcctccttgtataaatcctggttgctgtctctttatgaggagttgtggcccgttgtcaggcaacgtggcgtg gtgtgcactgtgtttgctgacgcaacccccactggttggggcattgccaccacctgtcagctcctttccgggactttcgctttc cccctccctattgccacggcggaactcatcgccgcctgccttgcccgctgctggacaggggctcggctgttgggcactga caattccgtggtgttgtcggggaagctgacgtcctttccatggctgctcgcctgtgttgccacctggattctgcgcgggacgt ccttctgctacgtcccttcggccctcaatccagcggaccttccttcccgcggcctgctgccggctctgcggcctcttccgcgt cttcgccttcgccctcagacgagtcggatctccctttgggccgcctccccgcctggaattcgagctcggtacctttaagacc aatgacttacaaggcagctgtagatcttagccactttttaaaagaaaaggggggactggaagggctaattcactcccaac gaagacaagatctgctttttgcttgtactgggtctctctggttagaccagatctgagcctgggagctctctggctaactaggg aacccactgcttaagcctcaataaagcttgccttgagtgcttcaagtagtgtgtgcccgtctgttgtgtgactctggtaactag agatccctcagacccttttagtcagtgtggaaaatctctagcagtagtagttcatgtcatcttattattcagtatttataacttgca aagaaatgaatatcagagagtgagaggaacttgtttattgcagcttataatggttacaaataaagcaatagcatcacaaa tttcacaaataaagcatttttttcactgcattctagttgtggtttgtccaaactcatcaatgtatcttatcatgtctggctctagctat cccgcccctaactccgcccatcccgcccctaactccgcccagttccgcccattctccgccccatggctgactaattttttttat ttatgcagaggccgaggccgcctcggcctctgagctattccagaagtagtgaggaggcttttttggaggcctagggacgt acccaattcgccctatagtgagtcgtattacgcgcgctcactggccgtcgttttacaacgtcgtgactgggaaaaccctgg cgttacccaacttaatcgccttgcagcacatccccctttcgccagctggcgtaatagcgaagaggcccgcaccgatcgcc cttcccaacagttgcgcagcctgaatggcgaatggacgcgccctgtagcggcgcattaagcgcggcgggtgtggtggt tacgcgcagcgtgaccgctacacttgccagcgccctagcgcccgctcctttcgctttcttcccttcctttctcgccacgttcgc cggctttccccgtcaagctctaaatcgggggctccctttagggttccgatttagtgctttacggcacctcgaccccaaaaaa cttgattagggtgatggttcacgtagtgggccatcgccctgatagacggtttttcgccctttgacgttggagtccacgttcttta atagtggactcttgttccaaactggaacaacactcaaccctatctcggtctattcttttgatttataagggattttgccgatttcg gcctattggttaaaaaatgagctgatttaacaaaaatttaacgcgaattttaacaaaatattaacgcttacaatttaggtggc acttttcggggaaatgtgcgcggaacccctatttgtttatttttctaaatacattcaaatatgtatccgctcatgagacaataac cctgataaatgcttcaataatattgaaaaaggaagagtatgagtattcaacatttccgtgtcgcccttattcccttttttgcggc attttgccttcctgtttttgctcacccagaaacgctggtgaaagtaaaagatgctgaagatcagttgggtgcacgagtgggtt acatcgaactggatctcaacagcggtaagatccttgagagttttcgccccgaagaacgttttccaatgatgagcacttttaa agttctgctatgtggcgcggtattatcccgtattgacgccgggcaagagcaactcggtcgccgcatacactattctcagaat gacttggttgagtactcaccagtcacagaaaagcatcttacggatggcatgacagtaagagaattatgcagtgctgccat aaccatgagtgataacactgcggccaacttacttctgacaacgatcggaggaccgaaggagctaaccgcttttttgcaca acatgggggatcatgtaactcgccttgatcgttgggaaccggagctgaatgaagccataccaaacgacgagcgtgaca ccacgatgcctgtagcaatggcaacaacgttgcgcaaactattaactggcgaactacttactctagcttcccggcaacaa ttaatagactggatggaggcggataaagttgcaggaccacttctgcgctcggcccttccggctggctggtttattgctgata aatctggagccggtgagcgtgggtctcgcggtatcattgcagcactggggccagatggtaagccctcccgtatcgtagtt atctacacgacggggagtcaggcaactatggatgaacgaaatagacagatcgctgagataggtgcctcactgattaag cattggtaactgtcagaccaagtttactcatatatactttagattgatttaaaacttcatttttaatttaaaaggatctaggtgaag atcctttttgataatctcatgaccaaaatcccttaacgtgagttttcgttccactgagcgtcagaccccgtagaaaagatcaa aggatcttcttgagatcctttttttctgcgcgtaatctgctgcttgcaaacaaaaaaaccaccgctaccagcggtggtttgtttg ccggatcaagagctaccaactctttttccgaaggtaactggcttcagcagagcgcagataccaaatactgttcttctagtgt agccgtagttaggccaccacttcaagaactctgtagcaccgcctacatacctcgctctgctaatcctgttaccagtggctgc tgccagtggcgataagtcgtgtcttaccgggttggactcaagacgatagttaccggataaggcgcagcggtcgggctga acggggggttcgtgcacacagcccagcttggagcgaacgacctacaccgaactgagatacctacagcgtgagctatg agaaagcgccacgcttcccgaagggagaaaggcggacaggtatccggtaagcggcagggtcggaacaggagagc gcacgagggagcttccagggggaaacgcctggtatctttatagtcctgtcgggtttcgccacctctgacttgagcgtcgattt ttgtgatgctcgtcaggggggcggagcctatggaaaaacgccagcaacgcggcctttttacggttcctggccttttgctggc cttttgctcacatgttctttcctgcgttatcccctgattctgtggataaccgtattaccgcctttgagtgagctgataccgctcgcc gcagccgaacgaccgagcgcagcgagtcagtgagcgaggaagcggaagagcgcccaatacgcaaaccgcctctc cccgcgcgttggccgattcattaatgcagctggcacgacaggtttcccgactggaaagcgggcagtgagcgcaacgca attaatgtgagttagctcactcattaggcaccccaggctttacactttatgcttccggctcgtatgttgtgtggaattgtgagcg gataacaatttcacacaggaaacagctatgaccatgattacgccaagcgcgcaattaaccctcactaaagggaacaa aagctggagctgcaagctt |
| 102 | aatgtagtcttatgcaatactcttgtagtcttgcaacatggtaacgatgagttagcaacatgccttacaaggagagaaaaa gcaccgtgcatgccgattggtggaagtaaggtggtacgatcgtgccttattaggaaggcaacagacgggtctgacatgg attggacgaaccactgaattgccgcattgcagagatattgtatttaagtgcctagctcgatacataaacgggtctctctggtt agaccagatctgagcctgggagctctctggctaactagggaacccactgcttaagcctcaataaagcttgccttgagtgct tcaagtagtgtgtgcccgtctgttgtgtgactctggtaactagagatccctcagacccttttagtcagtgtggaaaatctctag cagtggcgcccgaacagggacttgaaagcgaaagggaaaccagaggagctctctcgacgcaggactcggcttgctg aagcgcgcacggcaagaggcgaggggcggcgactggtgagtacgccaaaaattttgactagcggaggctagaagg agagagatgggtgcgagagcgtcagtattaagcgggggagaattagatcgcgatgggaaaaaattcggttaaggcca gggggaaagaaaaaatataaattaaaacatatagtatgggcaagcagggagctagaacgattcgcagttaatcctgg cctgttagaaacatcagaaggctgtagacaaatactgggacagctacaaccatcccttcagacaggatcagaagaact tagatcattatataatacagtagcaaccctctattgtgtgcatcaaaggatagagataaaagacaccaaggaagctttag acaagatagaggaagagcaaaacaaaagtaagaccaccgcacagcaagcggccgctgatcttcagacctggagg aggagatatgagggacaattggagaagtgaattatataaatataaagtagtaaaaattgaaccattaggagtagcaccc accaaggcaaagagaagagtggtgcagagagaaaaaagagcagtgggaataggagctttgttccttgggttcttggg agcagcaggaagcactatgggcgcagcgtcaatgacgctgacggtacaggccagacaattattgtctggtatagtgca gcagcagaacaatttgctgagggctattgaggcgcaacagcatctgttgcaactcacagtctggggcatcaagcagctc caggcaagaatcctggctgtggaaagatacctaaaggatcaacagctcctggggatttggggttgctctggaaaactcat ttgcaccactgctgtgccttggaatgctagttggagtaataaatctctggaacagatttggaatcacacgacctggatggag tgggacagagaaattaacaattacacaagcttaatacactccttaattgaagaatcgcaaaaccagcaagaaaagaat gaacaagaattattggaattagataaatgggcaagtttgtggaattggtttaacataacaaattggctgtggtatataaaatt attcataatgatagtaggaggcttggtaggtttaagaatagtttttgctgtactttctatagtgaatagagttaggcagggatatt caccattatcgtttcagacccacctcccaaccccgaggggacccgacaggcccgaaggaatagaagaagaaggtgg agagagagacagagacagatccattcgattagtgaacggatctcgacggtatcggttaacttttaaaagaaaagggggg gattggggggtacagtgcaggggaaagaatagtagacataatagcaacagacatacaaactaaagaattacaaaaa caaattacaaaattcaaaattttatcgataagcttgggagttccgcgttacataacttacggtaaatggcccgcctggctga ccgcccaacgacccccgcccattgacgtcaataatgacgtatgttcccatagtaacgccaatagggactttccattgacgt caatgggtggagtatttacggtaaactgcccacttggcagtacatcaagtgtatcatatgccaagtacgccccctattgac gtcaatgacggtaaatggcccgcctggcattatgcccagtacatgaccttatgggactttcctacttggcagtacatctacgt attagtcatcgctattaccatggtgatgcggttttggcagtacatcaatgggcgtggatagcggtttgactcacggggatttcc aagtctccaccccattgacgtcaatgggagtttgttttggcaccaaaatcaacgggactttccaaaatgtcgtaacaactcc gccccattgacgcaaatgggcggtaggcgtgtacggtgggaggtctatataagcagagctcgtttagtgaaccgtcagat cgcctggagacgccatccacgctgttttgacctccatagaagacaccgcccgggcgtacggatccATGGCTG CTGCTTCAGCAAGACCATGGACCTGCCTGACGACCACTACCTGTCCACCCAGACC ATCCTGTCCAAGGACCTGAACTCCGGACTCAGATCTGGCAGCGGTCTCGAGATG GAAGTTAGCGCTCTGGAAAAAGAAGTGTCTGCACTCGAGAAAGAAGTAAGTGCCC TTGAGAAGGAGGTGTCCGCACTCGAGAAGGAGGTCAGCGCCCTGGAAAAGGAAA AGCGAGACCATATGGTTTTGCTTGAGTATGTTACAGCGGCTGGCATTACCGATGC ATCAAGCTACTTCGCCAGCGAGCAGGGCGAGATCCAGTGGGTGAAGGTGTCCGC CCTGAAGGAAAAAGTAAGCGCACTGAAAGAAAAGGTGAGCGCGCTGAAGGAGAA AGTGAGCGCCCTGAAAGAGAAAGTCTCTGCCCTTAAGGAGGATATCGAGGGCAG AGGAAGTCTGCTAACATGCGGTGACGTCGAGGAGAATCCTGGCCCAAAGCTTGC CACCATGCGCAAAGGCGAAGAACTGTTTACCGGCATTGTGCCGATTCTGGTGGAA CTGGATGGCGATGTGAACGGCCATAAATTTTTTGTGCGCGGCGAAGGCGAAGGC GATGCGACCATTGGCAAACTGAGCCTGAAATTTATTTGCACCACCGGCAAACTGC CGGTGCCGTGGCCGACCCTGGTGACCACCCTGACCTATGGCGTGCAGTGCTTTA GCCGCTATCCGGATCATATGAAACGCCATGATTTTTTTAAAAGCGCGATGCCGGA AGGCTATGTGCAGGAACGCACCATTTATTTTAAAGATGATGGCACCTATAAAACCC GCGCGGAAGTGAAATTTGAAGGCGATACCCTGGTGAACCGCATTGAACTGAAAG GCATTGATTTTAAAGAAGATGGCAACATTCTGGGCCATAAACTGGAATATAACTTT AACAGCCATAAAGTGTATATTACCGCGGATAAACAGAACAACGGCATTAAAGCGA ACTTTACCATTCGCCATAACGTGGAAGATGGCAGCGTGCAGCTGGCGGATCATTA TCAGCAGAACACCCCGATTGGCGATGGCCCGGTTCTTCTTCCTGGCGGCCGCTC TAGAGAGGGCAGAGGAAGTCTGCTAACATGCGGTGACGTCGAGGAGAATCCTGG CCCAGAATTGATGGTGAGCAAGGGCGAGGAGGATAACATGGCCATCATCAAGGA GTTCATGCGCTTCAAGGTGCACATGGAGGGCTCCGTGAACGGCCACGAGTTCGA GATCGAGGGCGAGGGCGAGGGCCGCCCCTACGAGGGCACCCAGACCGCCAAG CTGAAGGTGACCAAGGGTGGCCCCCTGCCCTTCGCCTGGGACATCCTGTCCCCT CAGTTCATGTACGGCTCCAAGGCCTACGTGAAGCACCCCGCCGACATCCCCGAC TACTTGAAGCTGTCCTTCCCCGAGGGCTTCAAGTGGGAGCGCGTGATGAACTTCG AGGACGGCGGCGTGGTGACCGTGACCCAGGACTCCTCCCTGCAGGACGGCGAG |
| | TTCATCTACAAGGTGAAGCTGCGCGGCACCAACTTCCCCTCCGACGGCCCCGTA ATGCAGAAGAAGACCATGGGCTGGGAGGCCTCCTCCGAGCGGATGTACCCCGAG GACGGCGCCCTGAAGGGCGAGATCAAGCAGAGGCTGAAGCTGAAGGACGGCGG CCACTACGACGCTGAGGTCAAGACCACCTACAAGGCCAAGAAGCCCGTGCAGCT GCCCGGCGCCTACAACGTCAACATCAAGTTGGACATCACCTCCCACAACGAGGA CTACACCATCGTGGAACAGTACGAACGCGCCGAGGGCCGCCACTCCACCGGCG GCATGGACGAGCTGTACAAGtaataaCACatcgacaatcaacctctggattacaaaatttgtgaaagatt gactggtattcttaactatgttgctccttttacgctatgtggatacgctgctttaatgcctttgtatcatgctattgcttcccgtatgg ctttcattttctcctccttgtataaatcctggttgctgtctctttatgaggagttgtggcccgttgtcaggcaacgtggcgtggtgtg cactgtgtttgctgacgcaacccccactggttggggcattgccaccacctgtcagctcctttccgggactttcgctttccccct ccctattgccacggcggaactcatcgccgcctgccttgcccgctgctggacaggggctcggctgttgggcactgacaatt ccgtggtgttgtcggggaagctgacgtcctttccatggctgctcgcctgtgttgccacctggattctgcgcgggacgtccttct gctacgtcccttcggccctcaatccagcggaccttccttcccgcggcctgctgccggctctgcggcctcttccgcgtcttcgc cttcgccctcagacgagtcggatctccctttgggccgcctccccgcctggaattcgagctcggtacctttaagaccaatga cttacaaggcagctgtagatcttagccactttttaaaagaaaaggggggactggaagggctaattcactcccaacgaag acaagatctgctttttgcttgtactgggtctctctggttagaccagatctgagcctgggagctctctggctaactagggaaccc actgcttaagcctcaataaagcttgccttgagtgcttcaagtagtgtgtgcccgtctgttgtgtgactctggtaactagagatc cctcagacccttttagtcagtgtggaaaatctctagcagtagtagttcatgtcatcttattattcagtatttataacttgcaaaga aatgaatatcagagagtgagaggaacttgtttattgcagcttataatggttacaaataaagcaatagcatcacaaatttcac aaataaagcatttttttcactgcattctagttgtggtttgtccaaactcatcaatgtatcttatcatgtctggctctagctatcccgc ccctaactccgcccatcccgcccctaactccgcccagttccgcccattctccgccccatggctgactaattttttttatttatgc agaggccgaggccgcctcggcctctgagctattccagaagtagtgaggaggcttttttggaggcctagggacgtaccca attcgccctatagtgagtcgtattacgcgcgctcactggccgtcgttttacaacgtcgtgactgggaaaaccctggcgttac ccaacttaatcgccttgcagcacatccccctttcgccagctggcgtaatagcgaagaggcccgcaccgatcgcccttccc aacagttgcgcagcctgaatggcgaatggacgcgccctgtagcggcgcattaagcgcggcgggtgtggtggttacgc gcagcgtgaccgctacacttgccagcgccctagcgcccgctcctttcgctttcttcccttcctttctcgccacgttcgccggctt tccccgtcaagctctaaatcgggggctccctttagggttccgatttagtgctttacggcacctcgaccccaaaaaacttgatt agggtgatggttcacgtagtgggccatcgccctgatagacggtttttcgccctttgacgttggagtccacgttctttaatagtg gactcttgttccaaactggaacaacactcaaccctatctcggtctattcttttgatttataagggattttgccgatttcggcctatt ggttaaaaaatgagctgatttaacaaaaatttaacgcgaattttaacaaaatattaacgcttacaatttaggtggcacttttcg gggaaatgtgcgcggaacccctatttgtttatttttctaaatacattcaaatatgtatccgctcatgagacaataaccctgata aatgcttcaataatattgaaaaaggaagagtatgagtattcaacatttccgtgtcgcccttattcccttttttgcggcattttgcct tcctgtttttgctcacccagaaacgctggtgaaagtaaaagatgctgaagatcagttgggtgcacgagtgggttacatcga actggatctcaacagcggtaagatccttgagagttttcgccccgaagaacgttttccaatgatgagcacttttaaagttctgc tatgtggcgcggtattatcccgtattgacgccgggcaagagcaactcggtcgccgcatacactattctcagaatgacttggt tgagtactcaccagtcacagaaaagcatcttacggatggcatgacagtaagagaattatgcagtgctgccataaccatg agtgataacactgcggccaacttacttctgacaacgatcggaggaccgaaggagctaaccgcttttttgcacaacatgg gggatcatgtaactcgccttgatcgttgggaaccggagctgaatgaagccataccaaacgacgagcgtgacaccacg atgcctgtagcaatggcaacaacgttgcgcaaactattaactggcgaactacttactctagcttcccggcaacaattaata gactggatggaggcggataaagttgcaggaccacttctgcgctcggcccttccggctggctggtttattgctgataaatctg gagccggtgagcgtgggtctcgcggtatcattgcagcactggggccagatggtaagccctcccgtatcgtagttatctaca cgacggggagtcaggcaactatggatgaacgaaatagacagatcgctgagataggtgcctcactgattaagcattggt aactgtcagaccaagtttactcatatatactttagattgatttaaaacttcatttttaatttaaaaggatctaggtgaagatcctttt tgataatctcatgaccaaaatcccttaacgtgagttttcgttccactgagcgtcagaccccgtagaaaagatcaaaggatc ttcttgagatcctttttttctgcgcgtaatctgctgcttgcaaacaaaaaaaccaccgctaccagcggtggtttgtttgccggat caagagctaccaactctttttccgaaggtaactggcttcagcagagcgcagataccaaatactgttcttctagtgtagccgt agttaggccaccacttcaagaactctgtagcaccgcctacatacctcgctctgctaatcctgttaccagtggctgctgccag tggcgataagtcgtgtcttaccgggttggactcaagacgatagttaccggataaggcgcagcggtcgggctgaacgggg ggttcgtgcacacagcccagcttggagcgaacgacctacaccgaactgagatacctacagcgtgagctatgagaaag cgccacgcttcccgaagggagaaaggcggacaggtatccggtaagcggcagggtcggaacaggagagcgcacga gggagcttccagggggaaacgcctggtatctttatagtcctgtcgggtttcgccacctctgacttgagcgtcgatttttgtgat gctcgtcaggggggcggagcctatggaaaaacgccagcaacgcggcctttttacggttcctggccttttgctggccttttgc tcacatgttctttcctgcgttatcccctgattctgtggataaccgtattaccgcctttgagtgagctgataccgctcgccgcagc cgaacgaccgagcgcagcgagtcagtgagcgaggaagcggaagagcgcccaatacgcaaaccgcctctccccgc gcgttggccgattcattaatgcagctggcacgacaggtttcccgactggaaagcgggcagtgagcgcaacgcaattaat gtgagttagctcactcattaggcaccccaggctttacactttatgcttccggctcgtatgttgtgtggaattgtgagcggataa caatttcacacaggaaacagctatgaccatgattacgccaagcgcgcaattaaccctcactaaagggaacaaaagctg gagctgcaagctt |
| 103 | aatgtagtcttatgcaatactcttgtagtcttgcaacatggtaacgatgagttagcaacatgccttacaaggagagaaaaa gcaccgtgcatgccgattggtggaagtaaggtggtacgatcgtgccttattaggaaggcaacagacgggtctgacatgg attggacgaaccactgaattgccgcattgcagagatattgtatttaagtgcctagctcgatacataaacgggtctctctggtt agaccagatctgagcctgggagctctctggctaactagggaacccactgcttaagcctcaataaagcttgccttgagtgct tcaagtagtgtgtgcccgtctgttgtgtgactctggtaactagagatccctcagacccttttagtcagtgtggaaaatctctag cagtggcgcccgaacagggacttgaaagcgaaagggaaaccagaggagctctctcgacgcaggactcggcttgctg aagcgcgcacggcaagaggcgaggggcggcgactggtgagtacgccaaaaattttgactagcggaggctagaagg agagagatgggtgcgagagcgtcagtattaagcgggggagaattagatcgcgatgggaaaaaattcggttaaggcca gggggaaagaaaaaatataaattaaaacatatagtatgggcaagcagggagctagaacgattcgcagttaatcctgg cctgttagaaacatcagaaggctgtagacaaatactgggacagctacaaccatcccttcagacaggatcagaagaact tagatcattatataatacagtagcaaccctctattgtgtgcatcaaaggatagagataaaagacaccaaggaagctttag acaagatagaggaagagcaaaacaaaagtaagaccaccgcacagcaagcggccgctgatcttcagacctggagg aggagatatgagggacaattggagaagtgaattatataaatataaagtagtaaaaattgaaccattaggagtagcaccc accaaggcaaagagaagagtggtgcagagagaaaaaagagcagtgggaataggagctttgttccttgggttcttggg agcagcaggaagcactatgggcgcagcgtcaatgacgctgacggtacaggccagacaattattgtctggtatagtgca gcagcagaacaatttgctgagggctattgaggcgcaacagcatctgttgcaactcacagtctggggcatcaagcagctc caggcaagaatcctggctgtggaaagatacctaaaggatcaacagctcctggggatttggggttgctctggaaaactcat ttgcaccactgctgtgccttggaatgctagttggagtaataaatctctggaacagatttggaatcacacgacctggatggag tgggacagagaaattaacaattacacaagcttaatacactccttaattgaagaatcgcaaaaccagcaagaaaagaat gaacaagaattattggaattagataaatgggcaagtttgtggaattggtttaacataacaaattggctgtggtatataaaatt attcataatgatagtaggaggcttggtaggtttaagaatagtttttgctgtactttctatagtgaatagagttaggcagggatatt caccattatcgtttcagacccacctcccaaccccgaggggacccgacaggcccgaaggaatagaagaagaaggtgg agagagagacagagacagatccattcgattagtgaacggatctcgacggtatcggttaacttttaaaagaaaagggggg gattggggggtacagtgcaggggaaagaatagtagacataatagcaacagacatacaaactaaagaattacaaaaa caaattacaaaattcaaaattttatcgataagcttgggagttccgcgttacataacttacggtaaatggcccgcctggctga ccgcccaacgacccccgcccattgacgtcaataatgacgtatgttcccatagtaacgccaatagggactttccattgacgt caatgggtggagtatttacggtaaactgcccacttggcagtacatcaagtgtatcatatgccaagtacgccccctattgac gtcaatgacggtaaatggcccgcctggcattatgcccagtacatgaccttatgggactttcctacttggcagtacatctacgt attagtcatcgctattaccatggtgatgcggttttggcagtacatcaatgggcgtggatagcggtttgactcacggggatttcc aagtctccaccccattgacgtcaatgggagtttgttttggcaccaaaatcaacgggactttccaaaatgtcgtaacaactcc gccccattgacgcaaatgggcggtaggcgtgtacggtgggaggtctatataagcagagctcgtttagtgaaccgtcagat cgcctggagacgccatccacgctgttttgacctccatagaagacaccgcccgggcgtacggatccATGGACCT GCCTGACGACCACTACCTGTCCACCCAGACCATCCTGTCCAAGGACCTGAACTCC GGACTCAGATCTGGCAGCGGTCTCGAGATGGAAGTTAGCGCTCTGGAAAAAGAA GTGTCTGCACTCGAGAAAGAAGTAAGTGCCCTTGAGAAGGAGGTGTCCGCACTC GAGAAGGAGGTCAGCGCCCTGGAAAAGGAAAAGCGAGACCATATGGTTTTGCTT GAGTATGTTACAGCGGCTGGCATTACCGATGCATCAAGCTACTTCGCCAGCGAGC AGGGCGAGATCCAGTGGGTGAAGGTGTCCGCCCTGAAGGAAAAAGTAAGCGCAC TGAAAGAAAAGGTGAGCGCGCTGAAGGAGAAAGTGAGCGCCCTGAAAGAGAAAG TCTCTGCCCTTAAGGAGGATATCGAGGGCAGAGGAAGTCTGCTAACATGCGGTG ACGTCGAGGAGAATCCTGGCCCAAAGCTTGCCACCATGCGCAAAGGCGAAGAAC TGTTTACCGGCATTGTGCCGATTCTGGTGGAACTGGATGGCGATGTGAACGGCCA TAAATTTTTTGTGCGCGGCGAAGGCGAAGGCGATGCGACCATTGGCAAACTGAG CCTGAAATTTATTTGCACCACCGGCAAACTGCCGGTGCCGTGGCCGACCCTGGT GACCACCCTGACCTATGGCGTGCAGTGCTTTAGCCGCTATCCGGATCATATGAAA CGCCATGATTTTTTTAAAAGCGCGATGCCGGAAGGCTATGTGCAGGAACGCACCA TTTATTTTAAAGATGATGGCACCTATAAAACCCGCGCGGAAGTGAAATTTGAAGGC GATACCCTGGTGAACCGCATTGAACTGAAAGGCATTGATTTTAAAGAAGATGGCA ACATTCTGGGCCATAAACTGGAATATAACTTTAACAGCCATAAAGTGTATATTACC GCGGATAAACAGAACAACGGCATTAAAGCGAACTTTACCATTCGCCATAACGTGG AAGATGGCAGCGTGCAGCTGGCGGATCATTATCAGCAGAACACCCCGATTGGCG ATGGCCCGGTTCTTCTTCCTGGCGGCCGCTCTAGAGAGGGCAGAGGAAGTCTGC TAACATGCGGTGACGTCGAGGAGAATCCTGGCCCAGAATTGATGGTGAGCAAGG GCGAGGAGGATAACATGGCCATCATCAAGGAGTTCATGCGCTTCAAGGTGCACAT GGAGGGCTCCGTGAACGGCCACGAGTTCGAGATCGAGGGCGAGGGCGAGGGCC GCCCCTACGAGGGCACCCAGACCGCCAAGCTGAAGGTGACCAAGGGTGGCCCC CTGCCCTTCGCCTGGGACATCCTGTCCCCTCAGTTCATGTACGGCTCCAAGGCCT ACGTGAAGCACCCCGCCGACATCCCCGACTACTTGAAGCTGTCCTTCCCCGAGG GCTTCAAGTGGGAGCGCGTGATGAACTTCGAGGACGGCGGCGTGGTGACCGTG ACCCAGGACTCCTCCCTGCAGGACGGCGAGTTCATCTACAAGGTGAAGCTGCGC |
| | GGCACCAACTTCCCCTCCGACGGCCCCGTAATGCAGAAGAAGACCATGGGCTGG GAGGCCTCCTCCGAGCGGATGTACCCCGAGGACGGCGCCCTGAAGGGCGAGAT CAAGCAGAGGCTGAAGCTGAAGGACGGCGGCCACTACGACGCTGAGGTCAAGA CCACCTACAAGGCCAAGAAGCCCGTGCAGCTGCCCGGCGCCTACAACGTCAACA TCAAGTTGGACATCACCTCCCACAACGAGGACTACACCATCGTGGAACAGTACGA ACGCGCCGAGGGCCGCCACTCCACCGGCGGCATGGACGAGCTGTACAAGtaataa CACatcgacaatcaacctctggattacaaaatttgtgaaagattgactggtattcttaactatgttgctccttttacgctatgtg gatacgctgctttaatgcctttgtatcatgctattgcttcccgtatggctttcattttctcctccttgtataaatcctggttgctgtctctt tatgaggagttgtggcccgttgtcaggcaacgtggcgtggtgtgcactgtgtttgctgacgcaacccccactggttggggc attgccaccacctgtcagctcctttccgggactttcgctttccccctccctattgccacggcggaactcatcgccgcctgcctt gcccgctgctggacaggggctcggctgttgggcactgacaattccgtggtgttgtcggggaagctgacgtcctttccatgg ctgctcgcctgtgttgccacctggattctgcgcgggacgtccttctgctacgtcccttcggccctcaatccagcggaccttcct tcccgcggcctgctgccggctctgcggcctcttccgcgtcttcgccttcgccctcagacgagtcggatctccctttgggccgc ctccccgcctggaattcgagctcggtacctttaagaccaatgacttacaaggcagctgtagatcttagccactttttaaaag aaaaggggggactggaagggctaattcactcccaacgaagacaagatctgctttttgcttgtactgggtctctctggttaga ccagatctgagcctgggagctctctggctaactagggaacccactgcttaagcctcaataaagcttgccttgagtgcttca agtagtgtgtgcccgtctgttgtgtgactctggtaactagagatccctcagacccttttagtcagtgtggaaaatctctagcag tagtagttcatgtcatcttattattcagtatttataacttgcaaagaaatgaatatcagagagtgagaggaacttgtttattgca gcttataatggttacaaataaagcaatagcatcacaaatttcacaaataaagcatttttttcactgcattctagttgtggtttgtc caaactcatcaatgtatcttatcatgtctggctctagctatcccgcccctaactccgcccatcccgcccctaactccgcccag ttccgcccattctccgccccatggctgactaattttttttatttatgcagaggccgaggccgcctcggcctctgagctattccag aagtagtgaggaggcttttttggaggcctagggacgtacccaattcgccctatagtgagtcgtattacgcgcgctcactgg ccgtcgttttacaacgtcgtgactgggaaaaccctggcgttacccaacttaatcgccttgcagcacatccccctttcgccag ctggcgtaatagcgaagaggcccgcaccgatcgcccttcccaacagttgcgcagcctgaatggcgaatggacgcgc cctgtagcggcgcattaagcgcggcgggtgtggtggttacgcgcagcgtgaccgctacacttgccagcgccctagcgcc cgctcctttcgctttcttcccttcctttctcgccacgttcgccggctttccccgtcaagctctaaatcgggggctccctttagggtt ccgatttagtgctttacggcacctcgaccccaaaaaacttgattagggtgatggttcacgtagtgggccatcgccctgatag acggtttttcgccctttgacgttggagtccacgttctttaatagtggactcttgttccaaactggaacaacactcaaccctatct cggtctattcttttgatttataagggattttgccgatttcggcctattggttaaaaaatgagctgatttaacaaaaatttaacgcg aattttaacaaaatattaacgcttacaatttaggtggcacttttcggggaaatgtgcgcggaacccctatttgtttatttttctaa atacattcaaatatgtatccgctcatgagacaataaccctgataaatgcttcaataatattgaaaaaggaagagtatgagt attcaacatttccgtgtcgcccttattcccttttttgcggcattttgccttcctgtttttgctcacccagaaacgctggtgaaagtaa aagatgctgaagatcagttgggtgcacgagtgggttacatcgaactggatctcaacagcggtaagatccttgagagttttc gccccgaagaacgttttccaatgatgagcacttttaaagttctgctatgtggcgcggtattatcccgtattgacgccgggca agagcaactcggtcgccgcatacactattctcagaatgacttggttgagtactcaccagtcacagaaaagcatcttacgg atggcatgacagtaagagaattatgcagtgctgccataaccatgagtgataacactgcggccaacttacttctgacaacg atcggaggaccgaaggagctaaccgcttttttgcacaacatgggggatcatgtaactcgccttgatcgttgggaaccgga gctgaatgaagccataccaaacgacgagcgtgacaccacgatgcctgtagcaatggcaacaacgttgcgcaaactatt aactggcgaactacttactctagcttcccggcaacaattaatagactggatggaggcggataaagttgcaggaccacttc tgcgctcggcccttccggctggctggtttattgctgataaatctggagccggtgagcgtgggtctcgcggtatcattgcagca ctggggccagatggtaagccctcccgtatcgtagttatctacacgacggggagtcaggcaactatggatgaacgaaata gacagatcgctgagataggtgcctcactgattaagcattggtaactgtcagaccaagtttactcatatatactttagattgatt taaaacttcatttttaatttaaaaggatctaggtgaagatcctttttgataatctcatgaccaaaatcccttaacgtgagttttcgt tccactgagcgtcagaccccgtagaaaagatcaaaggatcttcttgagatcctttttttctgcgcgtaatctgctgcttgcaaa caaaaaaaccaccgctaccagcggtggtttgtttgccggatcaagagctaccaactctttttccgaaggtaactggcttca gcagagcgcagataccaaatactgttcttctagtgtagccgtagttaggccaccacttcaagaactctgtagcaccgccta catacctcgctctgctaatcctgttaccagtggctgctgccagtggcgataagtcgtgtcttaccgggttggactcaagacg atagttaccggataaggcgcagcggtcgggctgaacggggggttcgtgcacacagcccagcttggagcgaacgacct acaccgaactgagatacctacagcgtgagctatgagaaagcgccacgcttcccgaagggagaaaggcggacaggt atccggtaagcggcagggtcggaacaggagagcgcacgagggagcttccagggggaaacgcctggtatctttatagt cctgtcgggtttcgccacctctgacttgagcgtcgatttttgtgatgctcgtcaggggggcggagcctatggaaaaacgcca gcaacgcggcctttttacggttcctggccttttgctggccttttgctcacatgttctttcctgcgttatcccctgattctgtggataac cgtattaccgcctttgagtgagctgataccgctcgccgcagccgaacgaccgagcgcagcgagtcagtgagcgagga agcggaagagcgcccaatacgcaaaccgcctctccccgcgcgttggccgattcattaatgcagctggcacgacaggttt cccgactggaaagcgggcagtgagcgcaacgcaattaatgtgagttagctcactcattaggcaccccaggctttacactt tatgcttccggctcgtatgttgtgtggaattgtgagcggataacaatttcacacaggaaacagctatgaccatgattacgcc aagcgcgcaattaaccctcactaaagggaacaaaagctggagctgcaagctt |
| 104 | ATGGACCCCGTGGTGGTGCTGGGCCTGTGCCTGAGCTGCCTGCTGCTGCTGAGC CTGTGGAAGCAGAGCCACGGCGGCGGCAAG |
| 105 | MDPVWLGLCLSCLLLLSLWQSHGGGK |
| 106 | ATGGGCTGCTGCTTCAGCAAGACC |
| 107 | MGCCFSKT |
| 108 | GAGGACAGGGCCGGCGCCGGCATCATCGAGACCCCC |
| 109 | GCCACCAGGGCCGGCTGCGCCCCCAGCTACAGGGTG |
| 110 | CTGGACAGGGCCGGCGGCTACATCTTCAGCAGCGAC |
| 111 | A TCACCACTCTTGGGAAA TTTGGACAA |
| 112 | AGCTACTTCGCCAGCGAGCAGGGCGAGATCCAGTGGGTG |
| 113 | AGGGAGCTGAACGGCGGCGCCTACACCAGGTACGTG |
| 114 | TTCACCCTGAAGGGCGGCGCCCCCACCAAGGTGACC |
| 115 | ATCGCCCTGAAGGGCGGCAAGATCGTGAACAACTGG |
| 116 | ACCAGCGCCGTGCTGCAGAGCGGCTTCAGGAAGATG |
| 117 | AGCGGCGTGACCTTCCAGAGCGCCGTGAAGAGGACC |
| 118 | AAGGTGGCCACCGTGCAGAGCAAGATGAGCGACGTG |
| 119 | AGCGCCGTGAAGCTGCAGAACAACGAGCTGAGCCCC |
| 120 | GCCACCGTGAGGCTGCAGGCCGGCAACGCCACCGAG |
| 121 | AGGGAGCCCATGCTGCAGAGCGCCGACGCCCAGAGC |
| 122 | AAGGAGAGGAAGAGGAGGGGCGCCGACACCAGCATC |
| 123 | ACCAGGAGCGGCAAGAGGAGCTGGCCCCCCAGCGAG |
| 124 | GAGCCCGAGAAGCAGAGGAGCCCCCAGGACAACCAG |
| 125 | GGCCTGGTGAAGAGGAGGGGCGGCGGCACCGGCGAG |
| 126 | ITTLGKFGQ |
| 127 | EALFQGPK |
| 128 | SYFASEQGEIQWV |
| 129 | RAGAYIFS |
| 130 | RELNGGAYTRYV |
| 131 | FTLKGGAPTKVT |
| 132 | IALKGGKIVNNW |
| 133 | TSAVLQSGFRKM |
| 134 | KVATVQSKMSDV |
| 135 | SAVKLQNNELSP |
| 136 | ATVRLQAGNATE |
| 137 | REPMLQSADAQS |
| 138 | SGVTFQSAVKRT |
| 139 | SGVTFQGKFKK |
| 140 | YAKRGGVF |
| 141 | KERKRRGADTSI |
| 142 | TRSGKRSWPPSE |
| 143 | EPEKQRSPQDNQ |
| 144 | GLVKRRGGGTGE |
| 145 | ATGGACCTGCCTGACGACCACTACCTGTCCACCCAGACCATCCTGTCCAAGGACC TGAACTCCGGACTCAGATCTGGCAGCGGTCTCGAGATGGAAGTTAGCGCTCTGG AAAAAGAAGTGTCTGCACTCGAGAAAGAAGTAAGTGCCCTTGAGAAGGAGGTGTC CGCACTCGAGAAGGAGGTCAGCGCCCTGGAAAAGGAAAAGCGAGACCATATGGT TTTGCTTGAGTATGTTACAGCGGCTGGCATTACCGATGCATCAAAGGTGTCCGCC CTGAAGGAAAAAGTAAGCGCACTGAAAGAAAAGGTGAGCGCGCTGAAGGAGAAA GTGAGCGCCCTGAAAGAGAAAGTCTCTGCCCTTAAGGAGGATATCGAGGGCAGA GGAAGTCTGCTAACATGCGGTGACGTCGAGGAGAATCCTGGCCCAAAGCTTGCC ACCATGCGCAAAGGCGAAGAACTGTTTACCGGCATTGTGCCGATTCTGGTGGAAC |
| | TGGATGGCGATGTGAACGGCCATAAATTTTTGTGCGCGGCGAAGGCGAAGGCG ATGCGACCATTGGCAAACTGAGCCTGAAATTTATTTGCACCACCGGCAAACTGCC GGTGCCGTGGCCGACCCTGGTGACCACCCTGACCTATGGCGTGCAGTGCTTTAG CCGCTATCCGGATCATATGAAACGCCATGATTTTTTTAAAAGCGCGATGCCGGAA GGCTATGTGCAGGAACGCACCATTTATTTTAAAGATGATGGCACCTATAAAACCCG CGCGGAAGTGAAATTTGAAGGCGATACCCTGGTGAACCGCATTGAACTGAAAGG CATTGATTTTAAAGAAGATGGCAACATTCTGGGCCATAAACTGGAATATAACTTTA ACAGCCATAAAGTGTATATTACCGCGGATAAACAGAACAACGGCATTAAAGCGAA CTTTACCATTCGCCATAACGTGGAAGATGGCAGCGTGCAGCTGGCGGATCATTAT CAGCAGAACACCCCGATTGGCGATGGCCCGGTTCTTCTTCCTGGCGGCCGCTCT AGA |
| 146 | TGAGCAAGGGCGAGGAGGATAACATGGCCATCATCAAGGAGTTCATGCGCTTCA AGGTGCACATGGAGGGCTCCGTGAACGGCCACGAGTTCGAGATCGAGGGCGAG GGCGAGGGCCGCCCCTACGAGGGCACCCAGACCGCCAAGCTGAAGGTGACCAA GGGTGGCCCCCTGCCCTTCGCCTGGGACATCCTGTCCCCTCAGTTCATGTACGG CTCCAAGGCCTACGTGAAGCACCCCGCCGACATCCCCGACTACTTGAAGCTGTC CTTCCCCGAGGGCTTCAAGTGGGAGCGCGTGATGAACTTCGAGGACGGCGGCGT GGTGACCGTGACCCAGGACTCCTCCCTGCAGGACGGCGAGTTCATCTACAAGGT GAAGCTGCGCGGCACCAACTTCCCCTCCGACGGCCCCGTAATGCAGAAGAAGAC CATGGGCTGGGAGGCCTCCTCCGAGCGGATGTACCCCGAGGACGGCGCCCTGA AGGGCGAGATCAAGCAGAGGCTGAAGCTGAAGGACGGCGGCCACTACGACGCT GAGGTCAAGACCACCTACAAGGCCAAGAAGCCCGTGCAGCTGCCCGGCGCCTAC AACGTCAACATCAAGTTGGACATCACCTCCCACAACGAGGACTACACCATCGTGG AACAGTACGAACGCGCCGAGGGCCGCCACTCCACCGGCGGCATGGACGAGCTG TACAAGtaa |
| 147 | GAGGGCAGAGGAAGTCTGCTAACATGCGGTGACGTCGAGGAGAATCCTGGCCCA |

## Claims

1. A nucleic acid construct which comprises an operon wherein the operon comprises (a) a recombinant transgene that encodes a recombinant inactive form of a fluorescent reporter protein, and a cleavage site for a viral protease, and (b) a nucleic acid coding for a detectable expression control protein, wherein the nucleic acid sequences of (a) and (b) are operably assembled in said operon under the control of a single promoter and optionally of additional control sequence(s) for transcription and/or translation and wherein the nucleic acid sequences of (a) and (b) are optionally separated by the sequence of a polyprotein separating site such as the sequence of the separating 2A-peptide originating from Thosea asigna virus capsid such as the sequence of SEQ ID No.147.

2. A nucleic acid construct according to claim 1 wherein the recombinant transgene encoding the inactive form of the fluorescent reporter protein comprises a nucleotide sequence of an altered form of the Open Reading Frame that encodes the active form of the fluorescent reporter protein and wherein said alteration in the ORF comprises switching position in the ORF of at least one nucleotide sequence encoding specific structure domains of the active form of the fluorescent protein to prevent assembly of the expressed structure domains as a functional protein enabling maturation of the chromophore and wherein the nucleotide sequence encoding the inactive form of the fluorescent reporter protein additionally comprises a nucleotide sequence encoding a cleavage site for a determined protease.

3. A nucleic acid according to any one of claims 1 or 2, wherein the active fluorescent reporter protein is an active flip-GFP and the inactive fluorescent reporter protein is an inactive flip-GFP.

4. A nucleic acid construct according to claim 3, wherein the recombinant transgene comprises from 5'-end to 3'-end polynucleotides encoding the beta10 strand of GFP, a linker having from 3 to 15, in particular about 10, amino acid residues, the E5 domain of GFP, the beta11 strand of GFP, the cleavage site of the viral protease and the K5 domain of the GFP, the sequence of a polyprotein separating site such as the sequence of the separating 2A-peptide originating from Thosea asigna virus capsid, a polynucleotide encoding the beta1-9 strand of GFP wherein these polynucleotides together encode the inactive form of the recombinant GFP with the viral protease cleavage site.

5. A nucleic acid construct according to any one of claims 1 to 4, wherein the transgene further comprises upstream from the sequence encoding the inactive fluorescent reporter protein in particular the inactive flip-GFP, a signal peptide, in particular a signal peptide for retention of the expressed polypeptides in the endoplasmic reticulum (ER retention signal) or a signal peptide for targeting the expressed polypeptides to the cell membrane (membrane targeting signal), especially a Cytochrome P450 ER retention signal of sequence MDPVVVLGLCLSCLLLLSLWQSHGGGK (SEQ ID No.105) or a membrane targeting signal of sequence MGCCFSKT (SEQ ID No.107)..

6. A nucleic acid construct according to claim 5 wherein the polynucleotide encoding the signal peptide contains or consists of the sequence of ATGGACCCCGTGGTGGTGCTGGGCCTGTGCCTGAGCTGCCTGCTGCTGCTGAG CCTGTGGAAGCAGAGCCACGGCGGCGGCAAG (SEQ ID No.104) encoding the Cytochrome P450 ER retention signal peptide or contains or consists of the sequence of ATGGGCTGCTGCTTCAGCAAGACC (SEQ ID No. 106) encoding the membrane targeting signal peptide.

7. A nucleic acid construct according to any one of claims 1 to 6 wherein the expression control protein is a fluorescent protein with a fluorophore of a color that is different from the color of the reporter fluorescent protein, in partic104ular is mCherry protein or mTurquoise protein or cyan fluorescent protein (ECFP), yellow fluorescent protein such as mVenus, in particular the nucleic acid construct contains the polynucleotide of SEQ ID No.146 coding for mCherry.

8. A nucleic acid construct according to any one of claims 1 to 7, wherein the cleavage site is recognized by a protease of a determined virus family selected in the group of Alphaviruses, Coronaviruses, Enteroviruses, Retroviruses and Flaviviruses.

9. A nucleic acid construct according to any one of claims 1 to 5, wherein the nucleic acid sequence for the protease cleavage site encodes an amino acid sequence selected from the group of ITTLGKFGQ (SEQ ID No.126) for the Enterovirus 2A protease, EALFQGPK (SEQ ID No.127) or SYFASEQGEIQWV (SEQ ID No.128) for the Enterovirus 3C protease, RAGAYIFS (SEQ ID No.129) for Alphaviruses, RELNGGAYTRYV (SEQ ID No.130), FTLKGGAPTKVT (SEQ ID No.131), IALKGGKIVNNW (SEQ ID No.132), TSAVLQSGFRKM (SEQ ID No.133), KVATVQSKMSDV (SEQ ID No.134), SAVKLQNNELSP (SEQ ID No.135), ATVRLQAGNATE (SEQ ID No.136), REPMLQSADAQS (SEQ ID No.137) an SGVTFQSAVKRT (SEQ ID No.138) for SARS-CoV-2 coronavirus, SGVTFQGKFKK (SEQ ID No.139) for SARS virus coronavirus, YAKRGGVF (SEQ ID No140) for Flaviviruses, and more particularly KERKR**RG**ADTSI (SEQ ID No.141), TRSGK**RS**WPPSE (SEQ ID No. 142), EPEKQ**RS**PQDNQ (SEQ ID No.143), GLVKR**RG**GGTGE (SEQ ID No.144) for ZIKA viruses,.

10. A nucleic acid construct according to any one of claims 1 to 9 wherein the polynucleotide encoding the viral protease cleavage site consists of a polynucleotide selected from the group of:
GAGGACAGGGCCGGCGCCGGCATCATCGAGACCCCC for CHKV-1 (SEQ ID No.108) or GCCACCAGGGCCGGCTGCGCCCCCAGCTACAGGGTG for CHKV-2 (SEQ ID No.109) or CTGGACAGGGCCGGCGGCTACATCTTCAGCAGCGAC for CHKV-3 (SEQ ID No.110) or,
ATCACCACTCTTGGGAAATTTGGACAA for EV71_2A (SEQ ID No.111) or AGCTACTTCGCCAGCGAGCAGGGCGAGATCCAGTGGGTG for EV71_3C (SEQ ID No.112) or,
AGGGAGCTGAACGGCGGCGCCTACACCAGGTACGTG for SARSCoV2_1 (SEQ ID No.113) or TTCACCCTGAAGGGCGGCGCCCCCACCAAGGTGACC for SARSCoV2_2 (SEQ ID No.114) or
ATCGCCCTGAAGGGCGGCAAGATCGTGAACAACTGG for SARSCoV2_3 (SEQ ID No.115) or ACCAGCGCCGTGCTGCAGAGCGGCTTCAGGAAGATG for SARSCoV2_4 (SEQ ID No.116) or
AGCGGCGTGACCTTCCAGAGCGCCGTGAAGAGGACC for SARSCoV2_5 (SEQ ID No.117) or AAGGTGGCCACCGTGCAGAGCAAGATGAGCGACGTG for SARSCoV2_6 (SEQ ID No.118) or
AGCGCCGTGAAGCTGCAGAACAACGAGCTGAGCCCC for SARSCoV2_7 (SEQ ID No.119) or GCCACCGTGAGGCTGCAGGCCGGCAACGCCACCGAG for SARSCoV2_8 (SEQ ID No.120) or
AGGGAGCCCATGCTGCAGAGCGCCGACGCCCAGAGC for SARSCoV2_9 (SEQ ID No.121) or,
AAGGAGAGGAAGAGGAGGGGCGCCGACACCAGCATC for ZIKV_1 (SEQ ID No.122) or ACCAGGAGCGGCAAGAGGAGCTGGCCCCCCAGCGAG for ZIKV_2 (SEQ ID No.123) or GAGCCCGAGAAGCAGAGGAGCCCCCAGGACAACCAG for ZIKV_3 (SEQ ID No.124) or
GGCCTGGTGAAGAGGAGGGGCGGCGGCACCGGCGAG for ZIKV_4 (SEQ ID No.125).

11. A nucleic acid construct according to any one of claims 4 to 10, wherein the flip-GFP is encoded by the sequence of SEQ ID No. 145 and optionally wherein the sequence of the cleavage site for the protease is inserted at position 262 in said sequence.

12. A nucleic acid construct according to any one of claims 1 to 11, wherein the polynucleotide for insertion in the recombinant transgene to encode the inactive recombinant flip-GFP is selected from the group of SEQ ID No 47 to SEQ ID No.100 respectively for flipGFP_CHKV_1, flipGFP_CHKV_2, flipGFP_CHKV_3, flipGFP_ER_CHKV_1, flipGFP_ER_CHKV_2, flipGFP_ER_CHKV_3, flipGFP_ER_EV71_2A, flipGFP_ER_EV71_3C, flipGFP_ER_SARSCoV2_1, flipGFP_ER_SARSCoV2_2, flipGFP_ER_SARSCoV2_3, flipGFP_ER_SARSCoV2_4, flipGFP_ER_SARSCoV2_5, flipGFP_ER_SARSCoV2_6, flipGFP_ER_SARSCoV2_7, flipGFP_ER_SARSCoV2_8, flipGFP_ER_SARSCoV2_9, flipGFP_ER_ZIKV_1, flipGFP_ER_ZIKV_2, flipGFP_ER_ZIKV_3, flipGFP_ER_ZIKV_4, flipGFP_EV71_2A, flipGFP_EV71_3C, flipGFP_Membrane_CHKV_1, flipGFP_Membrane_CHKV_2, flipGFP_Membrane_CHKV_3, flipGFP_Membrane_EV71_2A, flipGFP_Membrane_EV71_3C, flipGFP_Membrane_SARSCoV2_1, flipGFP_Membrane_SARSCoV2_2, flipGFP_Membrane_SARSCoV2_3, flipGFP_Membrane_SARSCoV2_4, flipGFP_Membrane_SARSCoV2_5, flipGFP_Membrane_SARSCoV2_6, flipGFP_Membrane_SARSCoV2_7, flipGFP_Membrane_SARSCoV2_8, flipGFP_Membrane_SARSCoV2_9, flipGFP_Membrane_ZIKV_1, flipGFP_Membrane_ZIKV_2, flipGFP_Membrane_ZIKV_3, flipGFP_Membrane_ZIKV_4, flipGFP_SARSCoV2_1, flipGFP_SARSCoV2_2, flipGFP_SARSCoV2_3, flipGFP_SARSCoV2_4, flipGFP_SARSCoV2_5, flipGFP_SARSCoV2_6, flipGFP_SARSCoV2_7, flipGFP_SARSCoV2_8, flipGFP_SARSCoV2_9, flipGFP_ZIKV_1, flipGFP_ZIKV_2, flipGFP_ZIKV_3, flipGFP_ZIKV_4..

13. A recombinant nucleic acid according to any one of claims 1 to 13, which is selected from the group of pLentiPuro_flipGFP_ER_3C (SEQ ID No.101), pLentiPuro_flipGFP_Membrane_3C (SEQ ID No.102), and pLentiPuro-flipGFP-3C (SEQ ID No.103).

14. A set of at least two nucleic acid constructs according to any one of claims 1 to 13.

15. A nucleic acid construct according to any one of claims 1 to 14 wherein the promoter of the transgene is active in cells selected from the group of prokaryotic cells, in particular in bacterial cells or in archaeal cells, and/or is active in eukaryotic cells, in particular in mammalian cells or in insect cells, in particular the promoter is the CMV promoter.

16. A transformation vector which comprises a nucleic acid construct according to any one of claims 1 to 15, in particular a vector which is a plasmid for transfection or for transduction, especially a lentiviral vector plasmid.

17. A cell which is a prokaryotic or a eukaryotic cell or cell line transformed with the nucleic acid construct according to any one of claims 1 to 15 or with a transformation vector according to claim 16, in particular a stable cell line, especially a stable cell line transduced with lentiviral vector particles expressing a nucleic acid construct according to any one of claims 1 to 15.

18. A cell according to claim 17 which is a cell selected for its sensibility to infection by a determined human virus targeting the cleavage site of the inactive fluorescent protein expressed in the cell, wherein the cell is optionally a stable cell line.

19. A polynucleotide encoding a viral protease cleavage site which is selected from the group of GAGGACAGGGCCGGCGCCGGCATCATCGAGACCCCC for CHKV-1 (SEQ ID No.108) or GCCACCAGGGCCGGCTGCGCCCCCAGCTACAGGGTG for CHKV-2 (SEQ ID No.109) or CTGGACAGGGCCGGCGGCTACATCTTCAGCAGCGAC for CHKV-3 (SEQ ID No.110) or, ATCACCACTCTTGGGAAATTTGGACAA for EV71_2A (SEQ ID No.111) or AGCTACTTCGCCAGCGAGCAGGGCGAGATCCAGTGGGTG for EV71_3C (SEQ ID No.112) or, AGGGAGCTGAACGGCGGCGCCTACACCAGGTACGTG for SARSCoV2_1 (SEQ ID No.113) or TTCACCCTGAAGGGCGGCGCCCCCACCAAGGTGACC for SARSCoV2_2 (SEQ ID No.114) or ATCGCCCTGAAGGGCGGCAAGATCGTGAACAACTGG for SARSCoV2_3 (SEQ ID No.115) or ACCAGCGCCGTGCTGCAGAGCGGCTTCAGGAAGATG for SARSCoV2_4 (SEQ ID No.116) or AGCGGCGTGACCTTCCAGAGCGCCGTGAAGAGGACC for SARSCoV2_5 (SEQ ID No.117) or AAGGTGGCCACCGTGCAGAGCAAGATGAGCGACGTG for SARSCoV2_6 (SEQ ID No.118) or AGCGCCGTGAAGCTGCAGAACAACGAGCTGAGCCCC for SARSCoV2_7 (SEQ ID No.119) or GCCACCGTGAGGCTGCAGGCCGGCAACGCCACCGAG for SARSCoV2_8 (SEQ ID No.120) or AGGGAGCCCATGCTGCAGAGCGCCGACGCCCAGAGC for SARSCoV2_9 (SEQ ID No.121) or, AAGGAGAGGAAGAGGAGGGGCGCCGACACCAGCATC for ZIKV_1 (SEQ ID No.122) or ACCAGGAGCGGCAAGAGGAGCTGGCCCCCCAGCGAG for ZIKV_2 (SEQ ID No.123) or GAGCCCGAGAAGCAGAGGAGCCCCCAGGACAACCAG for ZIKV_3 (SEQ ID No.124) or GGCCTGGTGAAGAGGAGGGGCGGCGGCACCGGCGAG for ZIKV_4 (SEQ ID No.125).

20. The use of a nucleic acid construct according to any one of claims 1 to 15 or of a vector according to claim 16, for *in vitro* detection and optionally quantification of a viral infection in a biological sample of a human or animal subject, wherein the detection targets a virus recognizing the cleavage site inserted in the inactive fluorescent reporter protein.

21. An *in vitro* method of detecting or monitoring a pathogen infection, in particular a virus infection, in a biological sample previously obtained from a human or an animal subject, which comprises the steps of:
a. Providing cells according to claim 17 or 18 that express either transiently or stably an inactive fluorescent reporter protein comprising a cleavage site for a protease of the virus to be detected,
b. Contacting said cells with the assayed biological sample in condition that enable the virus when present in the sample, to infect the cells and its protease to cleave the protease cleavage site,
c. Allowing fluorescence to increase in the biological sample following activation of the inactive fluorescent reporter protein by cleavage of the viral protease cleavage site in step b. and measuring said fluorescence of the reporter protein,
d. Optionally comparing the fluorescence level of the active fluorescent reporter protein to a standard or fluorescent control protein expressed in the cells and optionally concluding on virus infectious activity in the subject and/or quantitating the virus.

22. An *in vitro* method of detecting or monitoring a virus infection, in a biological sample previously obtained from a human or an animal subject, which comprises the steps of:
a. Providing an inactive fluorescent protein as a reporter protein expressed from the nucleic acid construct of any one of claims 1 to 15, together with a control protein expressed from the same nucleic acid construct wherein the inactive fluorescent reporter protein comprises a cleavage site for a protease of the virus to be detected,
b. Contacting said inactive fluorescent reporter protein with the assayed biological sample in condition that enable the virus protease to target and to cleave the protease cleavage site in the inactive fluorescent reporter protein,
c. Allowing fluorescence to increase in the biological sample following activation of the inactive fluorescent reporter protein by cleavage in step b. and measuring said fluorescence,
d. Optionally comparing the fluorescence level to a standard or to the fluorescence of the control protein and optionally concluding on the virus infectious activity in the subject and/or quantitating the virus.

23. A method of any one of claims 21 or 22 of detecting or monitoring a virus infection, in a biological sample previously obtained from a human or an animal subject, wherein the virus is a determined virus selected in the group of Alphaviruses, Coronaviruses, Enteroviruses, Retroviruses and Flaviviruses, in particular is a coronavirus, especially is SARS-CoV-2 (or SARS-2) responsible for Covid-19.

24. A laboratory animal for experimental or clinical observation of the response to a virus infection, wherein the animal has been transformed to enable its genome to express a nucleic acid construct according to any one of claims 1 to 15either transiently or stably, the animal being in particular a rodent, an insect or a non-human mammal.
